# EUROPÄISCHE PATENTSCHRIFT

(11) **EP 3 140 268 B1**
(45) Veröffentlichungstag und Bekanntmachung des Hinweises auf die Patenterteilung: **18.07.2018**
(21) Anmeldenummer: 15722163.1
(22) Anmeldetag: 08.05.2015
(51) Int. Cl.: C07C 15/20, C09K 11/06, H05B 33/20

(54) **CYANIERTE PERYLEN-VERBINDUNGEN**
CYANATED PERYLENE COMPOUNDS
COMPOSÉS DE PÉRYLÈNE CYANURÉS

(30) Priorität: 09.05.2014 EP 14167746
(43) Veröffentlichungstag der Anmeldung: 15.03.2017
(73) Patentinhaber: BASF SE, 67056 Ludwigshafen am Rhein (DE)
(72) Erfinder: KÖNEMANN, Martin, 68199 Mannheim (DE); WAGENBLAST, Gerhard, 67157 Wachenheim (DE); IVANOVICI, Sorin, 69126 Heidelberg (DE); SEND, Robert, 76137 Karlsruhe (DE); MATTERN, Gabriele, 67105 Schifferstadt (DE); WEBER, Gerd, 67098 Bad Dürkheim (DE)
(86) Internationale Anmeldenummer: PCT/EP2015/060137
(87) Internationale Veröffentlichungsnummer: WO 2015/169935

(56) Entgegenhaltungen:
- WO-A1-2012/152812
- WO-A2-2012/094409
- JP-A- H11 273 864

## Beschreibung

Die vorliegende Erfindung betrifft neue cyanierte Perylen-Verbindungen und Mischungen davon, Zusammensetzungen, enthaltend mindestens eine cyanierte Perylen-Verbindung oder Mischungen davon sowie Verfahren zu deren Herstellung. Des Weiteren betrifft die vorliegende Erfindung Farbkonverter, enthaltend als Matrixmaterial mindestens ein Polymer und als Fluoreszenzfarbstoff mindestens eine cyanierte Perylen-Verbindung oder Mischungen davon oder eine Zusammensetzung, enthaltend mindestens eine cyanierte Perylen-Verbindung oder Mischungen davon, die Verwendung dieser Farbkonverter sowie Beleuchtungsvorrichtungen enthaltend mindestens eine LED und mindestens einen Farbkonverter.

Aufgrund ihres geringen Energieverbrauchs werden LEDs (engl., Light emitting diodes, LEDs) zunehmend als Lichtquelle für allgemeine Beleuchtung, z. B. in Büros und Wohnungen, oder für architektonische Beleuchtung, in Hinweisschildern, Kleingeräten, in der Automobil- oder Flugzeugindustrie eingesetzt. Die Lichtemission beruht auf der Rekombination von Elektron-Loch-Paaren (Excitonen) im Übergangsgebiet eines in Durchlassrichtung gepolten pn-Übergangs in einem Halbleiter. Die Größe der Bandlücke dieses Halbleiters bestimmt in etwa die Wellenlänge des ausgesendeten Lichts. Um eine beliebige Farbe zu erzeugen, können LEDs mit unterschiedlicher Bandlücke zu einer Multi-LED kombiniert werden.

Alternativ kann auch ein Strahlungskonversionsleuchtstoff (auch als Phosphor oder Fluoreszenzfarbmittel beziehungsweise Fluoreszenzfarbstoff bezeichnet) mit einer LED kombiniert werden. Hierbei wird die von der LED emittierte Strahlung teilweise von dem Strahlungskonversionsleuchtstoff absorbiert, der so zur Photolumineszenz angeregt wird. Die resultierende Lichtfarbe der LED ergibt sich aus dem transmittierenden Anteil des LED-Lichts und dem Emissionsspektrum des Strahlungskonversionsleuchtstoffs. Nach einer Methode wird dazu direkt auf die LED Lichtquelle (LED-Chip) ein polymeres Material aufgetragen, das einen Strahlungskonversionsleuchtstoff enthält. Häufig ist das polymere Material in etwa in Tropfenform oder halbkugelförmig auf den LED-Chip aufgebracht, wodurch bestimmte optische Effekte zur Aussendung des Lichts beitragen. Derartige Aufbauten, bei denen Strahlungskonversionsleuchtstoff in einer polymeren Matrix direkt und ohne Zwischenraum auf einen LED-Chip aufgebracht werden, werden auch als "Phosphor on a Chip" bezeichnet. Bei Phosphor on a Chip LEDs werden als Strahlungskonversionsleuchtstoffe in der Regel anorganische Materialien eingesetzt. Bei Phosphor on a Chip LEDs unterliegen das polymere Material und der Strahlungskonversionsleuchtstoff einer relativ hohen thermischen Belastung und Strahlungsbelastung. Aus diesem Grund eignen sich organische Strahlungskonversionsleuchtstoffe bisher nicht zur Anwendung in Phosphor on a Chip LED.

Nach einer anderen Methode befindet sich der Farbkonverter (auch als "Konverter" oder "Lichtkonverter" bezeichnet), der in der Regel eine Polymerschicht und einen Strahlungskonversionsleuchtstoff umfasst, in einem gewissen Abstand von dem LED Chip. Ein solcher Aufbau wird als "remote phosphor" bezeichnet.

Durch den räumlichen Abstand zwischen der primären Lichtquelle, der LED, und dem Farbkonverter wird die Belastung durch Wärme und Strahlung so weit verringert, dass auch organische Fluoreszenzfarbstoffe als Strahlungskonversionsleuchtstoffe eingesetzt werden können. Darüber hinaus sind LEDs nach dem "remote phosphor" Konzept energieeffizienter als solche nach dem "phosphor on a chip" Konzept. Der Einsatz organischer Fluoreszenzfarbstoffe in diesen Konvertern bietet verschiedene Vorteile. Zum einen lässt sich der Farbton des Lichtes mit Fluoreszenzfarbstoffen gut einstellen. Zum anderen werden keine Materialien benötigt, die Seltene Erden enthalten, welche aufwändig bergmännisch gewonnen und bereitgestellt werden müssen und nur in beschränktem Umfang zur Verfügung stehen.

Weißes Licht emittierende LEDs werden in vielen Anwendungsbereichen als Beleuchtungsquelle oder als Backlight in vollfarbigen Displays verwendet. Weißes Licht lässt sich mit LEDs auf verschiedene Arten erzeugen. Dabei ist die Grundlage für die Emission von weißem Licht immer die Überlagerung (Mischung) verschiedener Farben. In sogenannten Multi-LEDs werden beispielsweise drei verschiedenfarbiges Licht emittierende Dioden, in der Regel eine blaue, eine grüne und eine rote, oder zwei komplementärfarbiges Licht emittierende Dioden, eine blaue und eine gelbe, in einem Gehäuse vereinigt. Die Multi-LED ist aufgrund der unterschiedlichen Helligkeiten und Betriebsbedingungen der verschiedenen Leuchtdioden technisch aufwändig und daher teuer. Außerdem ist eine Bauteilminiaturisierung der Multi-LED stark begrenzt.

Weißes Licht lässt sich auch erzeugen, indem man mindestens einen Strahlungskonversionsstoff auf eine LED, die vorzugsweise blaues Licht mit einer Wellenlänge von 400 bis 500 nm emittiert, aufbringt. Als Strahlungskonversionsleuchtstoff wird häufig mit Cer dotierter Yttriumaluminiumgranat (im Folgenden auch als Ce:YAG bezeichnet), verwendet. Ce ist ein Leuchtstoff, der eine breite Emissionsbande mit einem Maximum bei ca. 560 nm zeigt. Je nach Konzentration des Strahlungskonversionsstoffs werden Teile des von der LED emittierten blauen Lichts absorbiert und in größtenteils gelbes Lumineszenzlicht umwandelt, so dass aus der Mischung des durchgelassenen blauen Lichts und des emittierten gelben Lichts weißes Licht entsteht. Der Weißton bzw. die Farbtemperatur der LED hängt daher von der Schichtdicke und der genauen Zusammensetzung des Ce:YAG Strahlungskonversionsstoffes ab. LEDs basierend auf einer blau emittierenden LED und Ce:YAG sind einfach herzustellen. Für einfache Anwendungen, bei denen die Farbwiedergabe und die Farbtönung von untergeordneter Bedeutung sind, ist die LED basierend auf der blau emittierenden LED Ce:YAG gut geeignet. Da der Rotanteil im Spektrum fehlt, dominiert der Blauteil des emittierten Licht. Daher ist eine LED basierend auf einer blau emittierenden LED und YAG als alleinigen Strahlungskonversionsleuchtstoff für viele Anwendungszwecke nicht geeignet. Für Anwendungen, bei denen eine hochwertige Farbwiedergabe erwünscht ist, ist die Lichtstrahlung der LED in dem Wellenlängenbereich von 460 bis 580 nm unzureichend. Nachteilig ist ferner die Verwendung von Materialien, die Seltene Erden wie Ce:YAG enthalten, wie zuvor erläutert.

Unter dem Farbwiedergabeindex (CRI, color rendering index) versteht man eine photometrische Größe, bei der eine Lichtquelle im Vergleich zu einer idealen Lichtquelle (Plankscher Lichtstrahler) hinsichtlich der Qualität bei der Farbwiedergabe von bis zu 14 gelisteten Referenzfärbungen bewertet wird (CIE 1974). Die Größe des CRI-Wertes kann zwischen 0 und 100 liegen und beschreibt, in wie weit eine Lichtquelle in der Lage ist, die unterschiedlichen Farben von Referenzfärbungen wiederzugeben. Die ersten kommerziell erhältlichen Weißlicht-LEDs wiesen eine Farbwiedergabe von 70 bis 80 auf. Sonnenlicht hat einen CRI von bis zu 100.

Die WO 2012/168395 beschreibt Farbkonverter, die mindestens ein Polymer und mindestens einen organischen Fluoreszenzfarbstoff enthalten, wobei der organische Fluoreszenzfarbstoff mindestens eine Struktureinheit der Formel (A) umfasst, 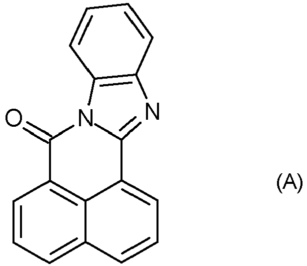 wobei die Struktureinheit einfach oder mehrfach mit gleichen oder verschiedenen Substituenten substituiert sein kann und wobei eine oder mehrere CH-Gruppen des Sechsrings der dargestellten Benzimidazolstruktur durch Stickstoff ersetzt sein kann. Cyanierte Fluoreszenzfarbstoffe sind in diesem Dokument nicht beschrieben.

Die unveröffentlichte EP 13179303.6 beschreibt cyanierte Naphthalinbenzimidazol-Verbindungen und Mischungen davon, deren Verwendung in Farbkonvertern, die Verwendung der Farbkonverter sowie Beleuchtungsvorrichtungen, enthaltend mindestens eine LED und mindestens einen Farbkonverter.

Die WO2012/094409 beschreibt cyanierte Perylen-Verbindungen als Fluoreszenzfarbstoffe für verschiedene Anwendungen. Die aus dem Stand der Technik bekannten organischen Fluoreszenzfarbstoffe sind teilweise hinsichtlich ihrer Photostabilität gegenüber blauem Licht im Wellenlängenbereich von 400 bis 500 nm und/oder der Fluoreszenzquantenausbeute in polymeren Matrices nicht zufriedenstellend.

Der vorliegenden Erfindung liegt die Aufgabe zugrunde, neue organische Fluoreszenzfarbstoffe bereitzustellen. Die Fluoreszenzfarbstoffe sollen mindestens eine der folgenden Eigenschaften aufweisen:
- hohe Photostabilität,
- hohe Fluoreszenzquantenausbeute in polymeren Matrices,
- hohe Kompatibilität mit dem LED-Herstellungsprozess,
- Verwendung als Strahlungskonversionsleuchtstoff anstelle von mit Seltenen Erden dotiertem YAG, insbesondere Ce:YAG und
- Verbesserung des Farbwiedergabeindex der Lichtquelle in Kombination mit weiteren rot emittierenden Fluoreszenzfarbstoffen.

Die Aufgabe wird überraschend gelöst durch die nachfolgend beschriebenen cyanierten Perylen-Verbindungen der Formel I 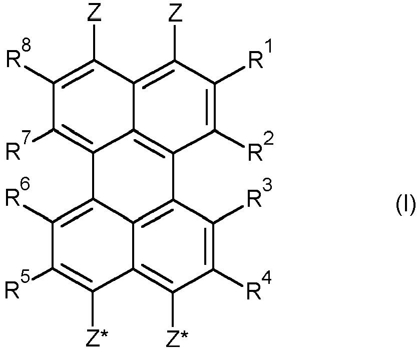 worin
einer der Substituenten Z für Cyano steht und der andere Substituent Z für CO₂R⁹, CONR¹⁰R¹¹, C₁-C₁₈-Alkyl, C₂-C₁₈-Alkenyl, C₂-C₁₈-Alkinyl, C₃-C₁₂-Cycloalkyl oder C₆-C₁₄-Aryl steht, wobei
   C₁-C₁₈-Alkyl, C₂-C₁₈-Alkenyl, C₂-C₁₈-Alkinyl unsubstituiert sind oder eine oder mehrere, gleiche oder verschiedene Substituenten Z^{a} tragen, C₃-C₁₂-Cycloalkyl unsubstituiert ist oder eine oder mehrere gleiche oder verschiedene Substituenten Z^{b} trägt, und
   C₆-C₁₄-Aryl unsubstituiert ist oder ein- oder mehrere gleiche oder verschiedene Substituenten Z^{Ar} trägt;
einer der Substituenten Z* für Cyano steht und der andere Substituent Z* für CO₂R⁹, CONR¹⁰R¹¹, C₁-C₁₈-Alkyl, C₂-C₁₈-Alkenyl, C₂-C₁₈-Alkinyl, C₃-C₁₂-Cycloalkyl oder C₆-C₁₄-Aryl steht, wobei
   C₁-C₁₈-Alkyl, C₂-C₁₈-Alkenyl, C₂-C₁₈-Alkinyl unsubstituiert sind oder eine oder mehrere, gleiche oder verschiedene Substituenten Z^{a} tragen, C₃-C₁₂-Cycloalkyl unsubstituiert ist oder eine oder mehrere gleiche oder verschiedene Substituenten Z^{b} trägt, und
   C₆-C₁₄-Aryl unsubstituiert ist oder ein- oder mehrere gleiche oder verschiedene Substituenten Z^{Ar} trägt;
R¹, R², R³, R⁴, R⁵, R⁶, R⁷ und R⁸ unabhängig voneinander ausgewählt sind unter Wasserstoff, Cyano, Brom und Chlor,
   unter der Maßgabe, dass 1, 2, 3, 4, 5, 6, 7 oder 8 der Substituenten R¹, R², R³, R⁴, R⁵, R⁶, R⁷ oder R⁸ für Cyano steht;
   wobei
   R⁹ für Wasserstoff, C₁-C₁₀-Alkyl, C₂-C₁₀-Alkenyl, C₂-C₁₀-Alkinyl, C₃-C₁₂-Cycloalkyl oder C₆-C₁₄-Aryl steht, wobei
      C₁-C₁₀-Alkyl, C₂-C₁₀-Alkenyl, C₂-C₁₀-Alkinyl unsubstituiert sind oder eine oder mehrere gleiche oder verschiedene Substituenten R^{a} tragen, C₃-C₁₂-Cycloalkyl unsubstituiert ist oder eine oder mehrere gleiche oder verschiedene Substituenten R^{b} trägt und C₆-C₁₄-Aryl unsubstituiert ist oder ein- oder mehrere gleiche oder verschiedene Substituenten R^{Ar} trägt;
   R¹⁰ und R¹¹, unabhängig voneinander, für Wasserstoff, C₁-C₁₀-Alkyl, C₂-C₁₀-Alkenyl, C₂-C₁₀-Alkinyl, C₃-C₁₂-Cycloalkyl oder C₆-C₁₄-Aryl stehen, wobei C₁-C₁₀-Alkyl, C₂-C₁₀-Alkenyl, C₂-C₁₀-Alkinyl, unsubstituiert sind oder eine oder mehrere gleiche oder verschiedene Substituenten R^{a} tragen, C₃-C₁₂-Cycloalkyl unsubstituiert ist oder eine oder mehrere gleiche oder verschiedene Substituenten R^{b} trägt und C₆-C₁₄-Aryl unsubstituiert ist oder ein- oder mehrere gleiche oder verschiedene Substituenten R^{Ar} trägt;
   jedes Z^{a} unabhängig voneinander für Halogen, Hydroxy, NR^{10a}R^{11a}, C₁-C₁₀-Alkoxy, C₁-C₁₀-Haloalkoxy, C₁-C₁₀-Alkylthio, C₃-C₁₂-Cycloalkyl, C₆-C₁₄-Aryl, C(=O)R^{9a}, C(=O)OR^{9a} oder C(O)NR^{10a}R^{11a} steht, wobei C₃-C₁₂-Cycloalkyl unsubstituiert ist oder eine oder mehrere gleiche oder verschiedene Substituenten R^{b} trägt und C₆-C₁₄-Aryl unsubstituiert ist oder ein- oder mehrere gleiche oder verschiedene Substituenten R^{Ar} trägt;
   jedes Z^{b} und jedes Z^{Ar} unabhängig voneinander für Halogen, Hydroxy, NR^{10a}R^{11a}, C₁-C₁₀-Alkyl, C₁-C₁₀-Alkoxy, C₁-C₁₀-Haloalkoxy, C₁-C₁₀-Alkylthio, C(=O)R^{9a}, C(=O)OR^{9a} oder C(O)NR^{10a}R^{11a} steht;
   jedes R^{a} unabhängig voneinander für Halogen, Hydroxy, C₁-C₁₀-Alkoxy, C₃-C₁₂-Cycloalkyl oder C₆-C₁₄-Aryl steht;
   jedes R^{b} unabhängig voneinander für Halogen, Hydroxy, C₁-C₁₀-Alkyl, C₁-C₁₀-Alkoxy, C₁-C₁₀-Haloalkoxy, C₁-C₁₀-Alkylthio, C₂-C₁₀-Alkenyl, C₂-C₁₀-Alkinyl, C₃-C₁₂-Cycloalkyl oder C₆-C₁₄-Aryl steht;
   jedes R^{Ar} unabhängig voneinander für Halogen, Hydroxy, C₁-C₁₀-Alkyl, C₁-C₁₀-Alkoxy, C₁-C₁₀-Haloalkoxy, C₁-C₁₀-Alkylthio, C₂-C₁₀-Alkenyl, C₂-C₁₀-Alkinyl, C₃-C₁₂-Cycloalkyl oder C₆-C₁₄-Aryl steht;
   R^{9a} für Wasserstoff, C₁-C₁₀-Alkyl, C₂-C₁₀-Alkenyl, C₂-C₁₀-Alkinyl, C₃-C₁₂-Cycloalkyl oder C₆-C₁₄-Aryl steht; und
   R^{10a}, R^{11a} unabhängig voneinander für Wasserstoff, C₁-C₁₀-Alkyl, C₂-C₁₀-Alkenyl, C₂-C₁₀-Alkinyl, C₃-C₁₂-Cycloalkyl oder C₆-C₁₄-Aryl stehen;
   und Mischungen davon.

Gegenstand der vorliegenden Erfindung ist ebenfalls eine Zusammensetzung, enthaltend mindestens eine cyanierte Perylen-Verbindung der Formel I wie zuvor definiert oder Mischungen davon und Verfahren zu ihrer Herstellung.

Gegenstand der vorliegenden Erfindung ist ebenfalls die Verwendung der cyanierten Perylen-Verbindung der Formel I wie zuvor definiert und Mischungen davon oder einer Zusammensetzung, enthaltend mindestens eine cyanierte Perylen-Verbindung der Formel I wie zuvor definiert und Mischungen davon in Farbkonvertern, für optische Label, zur unsichtbaren Markierung von Produkten, als Fluoreszenzfarbstoffe, vorzugsweise als Fluoreszenzlabel für Biomoleküle, als Pigmente, als Fluoreszenzfarbstoff in einem auf Fluoreszenzkonversion beruhenden Display; in einem lichtsammelnden Kunststoffteil, welches gegebenenfalls mit einer Solarzelle kombiniert ist; als Pigmentfarbstoff in elektrophoretischen Displays; als Fluoreszenzfarbstoff in einer auf Chemolumineszenz basierenden Anwendung.

Ein weiterer Gegenstand der vorliegenden Erfindung sind Farbkonverter, enthaltend mindestens ein Polymer als Matrix und mindestens eine cyanierte Perylen-Verbindung der Formel I wie zuvor definiert oder Mischungen davon oder einer Zusammensetzung enthaltend mindestens eine cyanierte Perylen-Verbindung der Formel I wie zuvor definiert und Mischungen davon als Fluoreszenzfarbstoff.

Ein weiterer Gegenstand der vorliegenden Erfindung ist die Verwendung der Farbkonverter zur Konversion von durch LEDs erzeugtem Licht.

Ein weiterer Gegenstand der vorliegenden Erfindung ist eine Beleuchtungsvorrichtung, umfassend mindestens eine LED und mindestens einen Farbkonverter wie zuvor definiert.

Die erfindungsgemäßen cyanierten Perylen-Verbindungen der Formel I und Mischungen davon sind überraschend photostabil und können vorteilhaft in einem Farbkonverter für blaue LEDs eingesetzt werden. Des Weiteren zeichnen sich die erfindungsgemäßen cyanierten Perylen-Verbindungen der Formel I und Mischungen davon durch eine hohe Fluoreszenzquantenausbeute in polymeren Matrices aus. Sie weisen eine hohe Kompatibilität mit dem LED-Herstellungsverfahren auf. Die erfindungsgemäßen cyanierten Perylen-Verbindungen der Formel I und Mischungen davon eignen sich in Kombination mit rot emittierenden Fluoreszenzfarbstoffen insbesondere für Farbkonverter in blau emittierenden LEDs zur Herstellung von Lichtquellen mit einem CRI-Wert oberhalb 90. Überraschenderweise eignen sich die neuen Fluoreszenzfarbstoffe auch als alternative Strahlungskonversionsleuchtstoffe für Ce:YAG, so dass weiße LEDs erhältlich sind, die als Leuchtstoff keine Seltenen Erden enthalten.

Bei den in den vorstehenden Formeln angegebenen Definitionen der Variablen werden Sammelbegriffe verwendet, die allgemein repräsentativ für die jeweiligen Substituenten stehen. Die Bedeutung Cₙ-Cₘ gibt die jeweils mögliche Anzahl von Kohlenstoffatomen in dem jeweiligen Substituenten oder Substituententeil an:
Halogen: Fluor, Chlor, Brom oder Iod.

Alkyl sowie Alkylteile in Alkoxy und Alkylthio: gesättigte, geradkettige oder verzweigte Kohlenwasserstoffreste mit 1 bis 30 (C₁-C₃₀-Alkyl), häufig 1 bis 20 (C₁-C₂₀-Alkyl), insbesondere 1 bis 10 (C₁-C₁₀-Alkyl) Kohlenstoffatomen wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, Heptyl, 1-Methylhexyl, Octyl, 1-Methylheptyl, 2-Ethylhexyl, n-Nonyl, n-Decyl.

Halo(gen)alkyl sowie alle Halogenalkylteile in Haloalkoxy: geradkettige oder verzweigte Alkylgruppen mit 1 bis 30, häufig 1 bis 20 und insbesondere 1 bis 10 Kohlenstoffatomen (wie vorstehend genannt), wobei in diesen Gruppen teilweise oder vollständig die Wasserstoffatome durch Halogenatome wie vorstehend genannt, ersetzt sein können.

Der Begriff "Alkylen" umfasst grundsätzlich geradkettige oder verzweigte Reste mit 1 bis 10 Kohlenstoffatomen wie Methylen, 1,1 -Ethylen, 1,2-Ethylen, Prop-1,2-ylen, Prop-1,3-ylen, But-1,2-ylen, But-1,3-ylen, But-1,4-ylen, 2-Methylprop-1,3-ylen, Pent-1,2-ylen, Pent-1,3-ylen, Pent-1,4-ylen, Pent-1,5-ylen, Pent-2,3-ylen, Pent-2,4-ylen, 1-Methylbut-1,4-ylen, 2-Methylbut-1,4-ylen, Hex-1,3-ylen, Hex-2,4-ylen, Hex-1,4-ylen, Hex-1,5-ylen, Hex-1,6-ylen und dergleichen.

Alkenyl: einfach ungesättigte, geradkettige oder verzweigte Kohlenwasserstoffreste mit 2 bis 10 (C₂-C₁₀-Alkenyl), beispielsweise 2 bis 10 oder 3 bis 10 Kohlenstoffatomen und einer Doppelbindung in einer beliebigen Position, z. B. Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl.

Alkinyl: geradkettige oder verzweigte Kohlenwasserstoffgruppen mit 2 bis 10 (C₂-C₁₀-Alkinyl), beispielsweise 2 bis 10 oder 3 bis 10 Kohlenstoffatomen und einer Dreifachbindung in einer beliebigen Position, z. B. Ethinyl, 1-Propinyl, 2-Propinyl, 1-Butinyl, 2-Butinyl, 3-Butinyl, 1-Methyl-2-propinyl.

Cycloalkyl: monocyclische, bicyclische oder tricyclische gesättigte Kohlenwasserstoffgruppe mit 3 bis 12 Kohlenstoffringgliedern, z. B. monocyclisches C₃-C₈-Cycloalkyl wie Cyclopropyl, Cyclobutyl, Cyclopentyl, Cyclohexyl, Cycloheptyl, Cyclooctyl, bicyclisches C₇-C₁₂-Cycloalkyl wie Bicyclo[2.2.1]hept-1-yl, Bicyclo[2.2.1]hept-2-yl, Bicyclo[2.2.1]hept-7-yl, Bicyclo[2.2.2]oct-1-yl, Bicyclo[2.2.2]oct-2-yl und Bicyclo[3.3.0]octyl und tricyclisches C₁₀-C₁₂-Cycloalkyl wie Tricyclo[3.3.1.1^{3,7}]decanyl.

Aryl: ein-, zwei- oder dreikernige (monocyclische, bicyclische oder tricyclische) aromatische Kohlenwasserstoffreste mit 6 bis 14, besonders bevorzugt 6 bis 10 Kohlenstoffatomen, die keine Ringheteroatome enthalten. Beispiele für Aryl sind insbesondere Phenyl, Naphthyl, Indenyl, Fluorenyl, Anthracenyl, Phenanthrenyl, und speziell Phenyl oder Naphthyl.

C₆-C₁₄-Aryl-C₁-C₁₀-alkylen: C₆-C₁₄-Aryl, wie zuvor definiert, welches über C₁-C₁₀-alkylen, wie zuvor definiert an das Gerüst gebunden ist. Beispiele hierfür sind Phenyl-C₁-C₁₀-alkylen (Phenyl-C₁-C₁₀-alkyl) und Naphthyl-C₁-C₁₀-alkylen (Naphthyl-C₁-C₁₀-alkyl) und speziell Phenyl-C₁-C₄-alkyl wie Benzyl oder 2-Phenylethyl.

C₆-C₁₄-Aryloxy: C₆-C₁₄-Aryl, wie zuvor definiert, welches über ein Sauerstoffatom (-O-) an das Gerüst gebunden ist. Bevorzugt sind Phenoxy und Naphthyloxy.

Heteroaryl (Hetaryl): ein-, zwei- oder dreikerniges (monocyclisches, bicyclisches oder tricyclisches) aromatisches Ringsystem mit 5 bis 14 Ringgliedern, enthaltend neben Kohlenstoffatomen als Ringglieder in der Regel 1, 2, 3 oder 4 Heteroatome als Ringglieder, die ausgewählt sind unter Sauerstoff, Schwefel und Stickstoff wie:
- fünf- oder sechsgliedriger aromatischer Heterocyclus, enthaltend ein, zwei, drei oder vier Heteroatome aus der Gruppe Sauerstoff, Stickstoff oder Schwefel: z. B. C-gebundenes 5-gliedriges Heteroaryl, enthaltend ein bis drei Stickstoffatome oder ein oder zwei Stickstoffatome und/oder ein Schwefel- oder Sauerstoffatom als Ringglieder wie 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-lsothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-lmidazolyl, 4-lmidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl; über Stickstoff gebundenes 5-gliedriges Heteroaryl, enthaltend ein bis drei Stickstoffatome als Ringglieder, wie Pyrrol-1-yl, Pyrazol-1-yl, Imidazol-1-yl, 1,2,3-Triazol-1-yl und 1,2,4-Triazol-1-yl; 6-gliedriges Heteroaryl, enthaltend ein bis drei Stickstoffatome als Ringglieder wie Pyridin-2-yl, Pyridin-3-yl, Pyridin-4-yl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl;
- benzokondensierter fünf- oder sechsgliedriger aromatischer Heterocyclus, enthaltend ein, zwei, drei oder vier, vorzugsweise ein, zwei oder drei Heteroatome aus der Gruppe Sauerstoff, Stickstoff oder Schwefel: z. B. fünf- oder sechsgliedrige aromatische Heterocyclen, wie zuvor definiert, welche neben Kohlenstoffatomen ein bis vier Stickstoffatome oder ein bis drei Stickstoffatome und ein Schwefel- oder Sauerstoffatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können wie Indolyl, Indazolyl, Benzofuryl, Dibenzofuryl, Isobenzofuranyl, Benzothiophenyl, Dibenzothiophenyl, Benzothiazolyl, Benzoxazolyl, Benzimidazolyl, Carbazolyl, Chinolinyl, Isochinolinyl, Chinazolinyl, Chinoxalinyl, Cinnolinyl, Phthalazinyl, Purinyl Acridinyl, Phenanthridinyl, Phenazinyl und 1,7-Phenanthrolinyl.

Im Rahmen der vorliegenden Erfindung versteht man unter einer "blauen LED" eine LED, die Licht im Wellenlängenbereich von 400 bis 500 nm, vorzugsweise 420 bis 480 nm, und insbesondere 440 bis 460 nm, emittiert. Geeignete Halbleitermaterialien sind Siliciumcarbid, Zinkselenid und Nitride wie Aluminiumnitrid (AIN), Galliumnitrid (GaN), Indiumnitrid (InN) und Indiumgalliumnitrid (InGaN). Im Rahmen der vorliegenden Erfindung versteht man unter einer "weißen LED" eine LED, die weißes Licht erzeugt. Beispiele für eine weiße LED sind Multi-LEDs oder eine blaue LED in Kombination mit mindestens einem Strahlungskonversionsleuchtstoff.

Im Rahmen der vorliegenden Erfindung versteht man unter Farbkonverter alle gegenständlichen Vorrichtungen, die in der Lage sind, Licht bestimmter Wellenlängen zu absorbieren und in Licht anderer Wellenlängen umzuwandeln. Farbkonverter sind zum Beispiel Bestandteil von Beleuchtungsvorrichtungen, insbesondere solchen Beleuchtungsvorrichtungen, die LEDs oder OLEDs als Lichtquelle nutzen oder von Fluoreszenzkonversionssolarzellen.

Der Begriff "im Wesentlichen" umfasst im Rahmen der vorliegenden Erfindung, die Begriffe vollständig, ganz und alle. Der Begriff umfasst einen Anteil von 90 % oder mehr, wie 95 % oder mehr, speziell 99 % oder 100 %.

Die nachfolgenden Ausführungen bezüglich bevorzugter Ausführungsformen der Substituenten (Reste) Z, Z*, R¹, R², R³, R⁴, R⁵, R⁶, R⁷ und R⁸ der Perylen-Verbindungen der Formel I, gelten für jeden Substituenten unabhängig voneinander und ebenso in Kombination der Substituenten miteinander.

Die nachfolgenden Ausführungen bezüglich bevorzugter Ausführungsformen der Substituenten Z, Z*, R¹, R², R³, R⁴, R⁵, R⁶, R⁷ und R⁸ gelten des Weiteren für die Perylen-Verbindungen der Formel I als auch für deren Verwendung in Farbkonvertern und Beleuchtungsvorrichtungen.

In den erfindungsgemäßen Perylen-Verbindungen der Formel I und Mischungen davon steht einer der Substituenten Z und einer der Substituenten Z* für Cyano und der andere Substituent Z und der andere Substituent Z* ist jeweils von Cyano verschieden. Die vorliegende Erfindung umfasst sowohl die Verbindung der Formel I einzeln als auch Mischungen davon.

Speziell umfasst die vorliegende Erfindung die nachfolgenden Verbindungen der Formeln I-a und I-b, sowie I-c und I-d 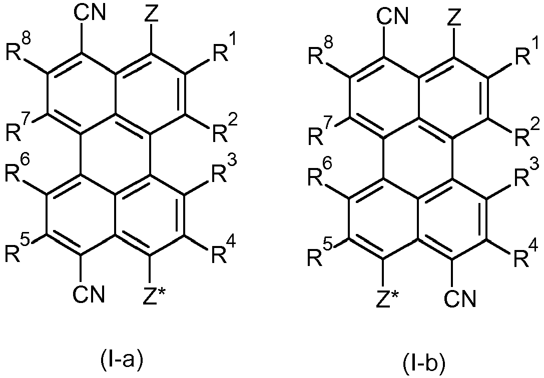 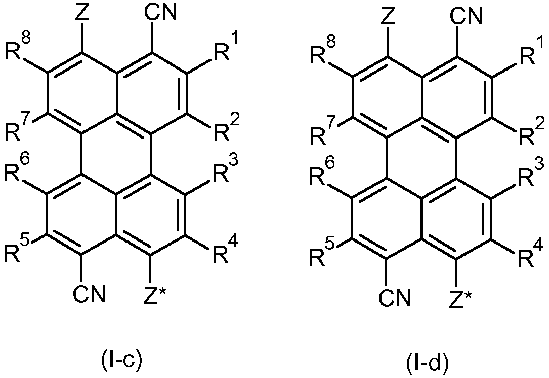 worin
R¹, R², R³, R⁴, R⁵, R⁶, R⁷, R⁸, Z und Z* die zuvor genannten Bedeutungen aufweisen,
einzeln als auch Mischungen davon. Insbesondere stehen in Verbindungen der Formeln I-a, I-b, I-c und I-d, die Substituenten R¹, R², R³, R⁴, R⁵, R⁶, R⁷, R⁸, die nicht für Cyano oder Wasserstoff stehen, alle für Chlor oder alle für Brom.

In den erfindungsgemäßen Perylen-Verbindungen der Formel I und Mischungen davon stehen 1, 2, 3, 4, 5, 6, 7 oder 8 der Substituenten R¹, R², R³, R⁴, R⁵, R⁶, R⁷ oder R⁸ für Cyano. Die anderen Substituenten R¹, R², R³, R⁴, R⁵, R⁶, R⁷ oder R⁸ stehen unabhängig voneinander für Wasserstoff, Brom oder Chlor. Insbesondere stehen die anderen Substituenten R¹, R², R³, R⁴, R⁵, R⁶, R⁷ oder R⁸ unabhängig voneinander für Wasserstoff oder Brom. Gemäß einer weiteren Ausführungsform stehen die anderen Substituenten R¹, R², R³, R⁴, R⁵, R⁶, R⁷ oder R⁸ unabhängig voneinander für Wasserstoff oder Chlor.

Gemäß einer ersten bevorzugten Ausführungsform stehen 1, 2, 3 oder 4 der Substituenten R¹, R², R³, R⁴, R⁵, R⁶, R⁷ oder R⁸ für Cyano. Die anderen Substituenten R¹, R², R³, R⁴, R⁵, R⁶, R⁷ oder R⁸ stehen unabhängig voneinander für Wasserstoff, Brom oder Chlor. Speziell stehen 1, 2, 3 oder 4 der Substituenten R², R³, R⁶ oder R⁷ für Cyano. Gemäß einer speziellen Ausführungsform steht keiner der Substituenten R¹, R², R³, R⁴, R⁵, R⁶, R⁷ oder R⁸ für Brom oder Chlor.

Gemäß einer zweiten bevorzugten Ausführungsform steht einer der Substituenten R¹, R², R³, R⁴, R⁵, R⁶, R⁷ oder R⁸ für Cyano, und die anderen Substituenten R¹, R², R³, R⁴, R⁵, R⁶, R⁷ oder R⁸ stehen für Wasserstoff. Insbesondere steht einer der Substituenten R², R³, R⁶ oder R⁷ für Cyano und die anderen Substituenten R¹, R², R³, R⁴, R⁵, R⁶, R⁷ oder R⁸ stehen für Wasserstoff.

Gemäß einer dritten bevorzugten Ausführungsform stehen zwei der Substituenten R¹, R², R³, R⁴, R⁵, R⁶, R⁷ oder R⁸ für Cyano, und die anderen Substituenten R¹, R², R³, R⁴, R⁵, R⁶, R⁷ oder R⁸ stehen für Wasserstoff. Insbesondere stehen zwei der Substituenten R², R³, R⁶ oder R⁷ für Cyano und die anderen Substituenten R¹, R², R³, R⁴, R⁵, R⁶, R⁷ oder R⁸ stehen für Wasserstoff.

In den erfindungsgemäßen Perylen-Verbindungen der Formel I und Mischungen davon stehen einer der Substituenten Z für Cyano und einer der Substituenten Z* für Cyano. Der andere Substituent Z und der andere Substituent Z* weisen die zuvor genannten Bedeutungen auf und sind vorzugsweise unabhängig voneinander ausgewählt unter C₁-C₁₀-Alkyl, CO₂R⁹, Phenyl-C₁-C₁₀-alkyl und Phenyl, wobei Phenyl und der Phenylteil von Phenyl-C₁-C₁₀-alkyl unsubstituiert sind oder einen oder mehrere, z. B. 1, 2 oder 3, unter C₁-C₆-Alkyl ausgewählte Substituenten tragen, und wobei R⁹ wie zuvor definiert ist. Vorzugsweise steht R⁹ für lineares oder verzweigtes C₁-C₆-Alkyl, speziell für Methyl, Ethyl, n-Propyl, Isopropyl, n-Butyl oder Isobutyl.

Ganz besonders bevorzugt steht einer der Substituenten Z für C₁-C₆-Alkyl, C₁-C₆-Alkoxycarbonyl oder Phenyl, das unsubstituiert ist oder 1, 2 oder 3 C₁-C₄-Alkylgruppen trägt. Speziell steht einer der Substituenten Z für C₁-C₆-Alkyl, wie Methyl, Ethyl, n-Propyl, Isopropyl, n-Butyl, Isobutyl, für C₁-C₆-Alkoxycarbonyl wie Methoxycarbonyl, Ethoxycarbonyl, n-Propoxycarbonyl, Isopropoxycarbonyl, n-Butoxycarbonyl, Isobutoxycarbonyl, Phenyl oder Phenyl, das 1, 2 oder 3 C₁-C₄-Alkylgruppen trägt, wie 2-Methylphenyl oder 2,6-Dimethylphenyl.

Ganz besonders bevorzugt steht einer der Substituenten Z* für C₁-C₆-Alkyl, C₁-C₆-Alkoxycarbonyl oder Phenyl, das unsubstituiert ist oder 1, 2 oder 3 C₁-C₄-Alkylgruppen trägt. Speziell steht einer der Substituenten Z* für C₁-C₆-Alkyl wie Methyl, Ethyl, n-Propyl, Isopropyl, n-Butyl, Isobutyl, für C₁-C₆-Alkoxycarbonyl wie Methoxycarbonyl, Ethoxycarbonyl, n-Propoxycarbonyl, Isopropoxycarbonyl, n-Butoxycarbonyl, Isobutoxycarbonyl, Phenyl, 2-Methylphenyl oder 2,6-Dimethylphenyl.

Gemäß einer besonders bevorzugten Ausführungsform ist die Perylen-Verbindung der Formel I ausgewählt unter Verbindungen der Formeln (1), (2), (3), (4), (5), (6), (7), (8), (9), (10), (11), (12), (13), (14), (15), (16), (17), (18), (19), (20) 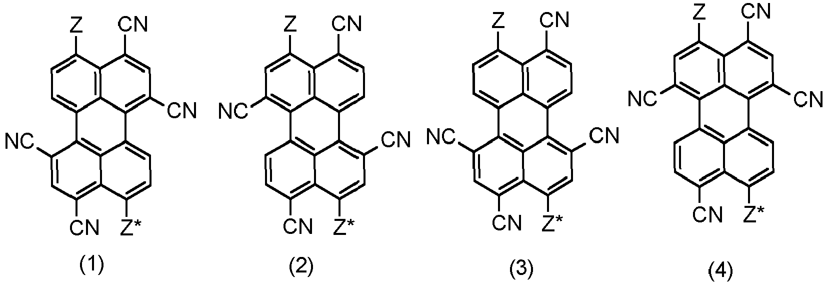 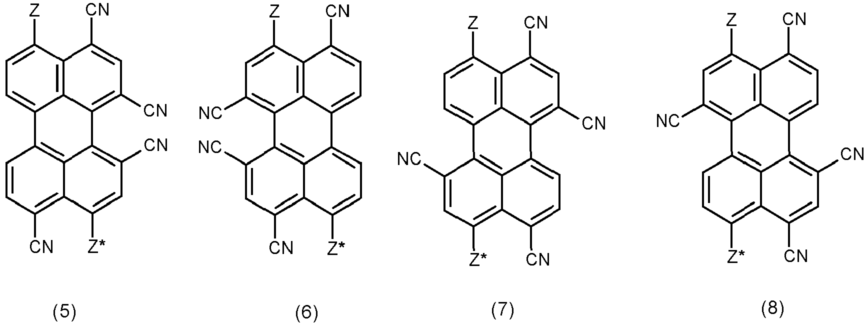 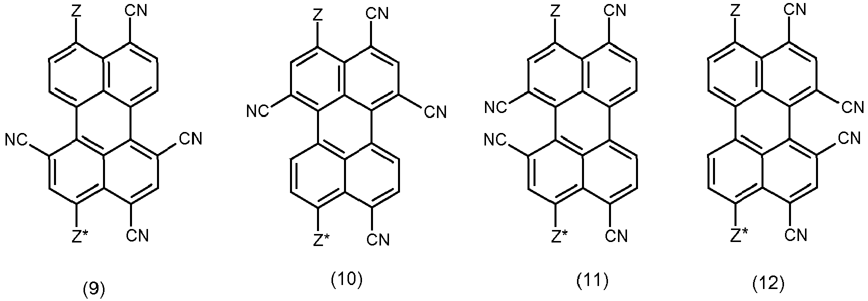 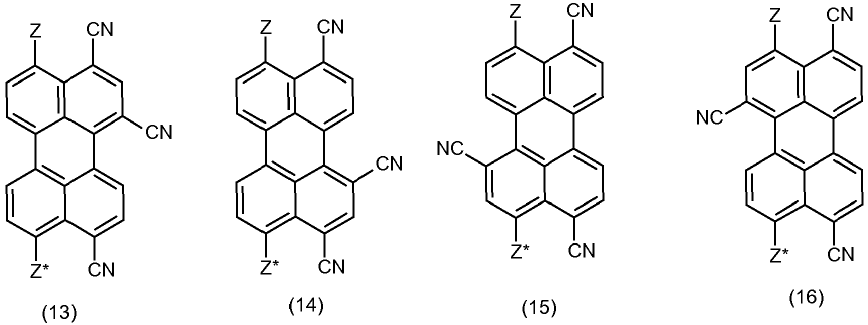 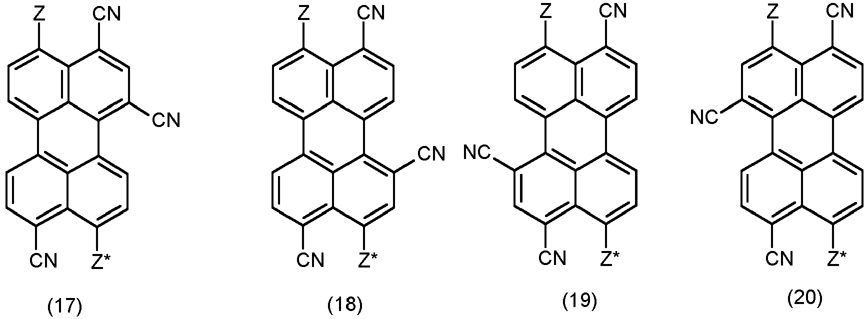 worin
- Z: ausgewählt ist unter C₁-C₆-Alkyl, C₁-C₆-Alkoxycarbonyl, Phenyl und Phenyl, das 1, 2 oder 3 C₁-C₄-Alkylgruppen trägt; und
- Z*: ausgewählt ist unter C₁-C₆-Alkyl, C₁-C₆-Alkoxycarbonyl, Phenyl und Phenyl, das 1, 2 oder 3 C₁-C₄-Alkylgruppen trägt;
und Mischungen davon.

Hierunter speziell bevorzugt sind Perylen-Verbindungen der Formeln (1), (2), (3), (4), (5), (6), (7), (8), (9), (10), (11), (12), (13), (14), (15), (16), (17), (18), (19), (20), worin Z und Z* die gleiche Bedeutung aufweisen.

Ein weiterer Gegenstand ist eine Zusammensetzung, enthaltend mindestens eine cyanierte Perylen-Verbindung der Formel I wie zuvor definiert oder Mischungen davon.

Die Herstellung der Perylen-Verbindungen der Formel I und Mischungen davon kann nach dem Fachmann bekannten Verfahren erfolgen oder wie nachfolgend beschrieben.

Ein weiterer Gegenstand ist eine Zusammensetzung, enthaltend mindestens eine cyanierte Perylen-Verbindung der Formel I-A, 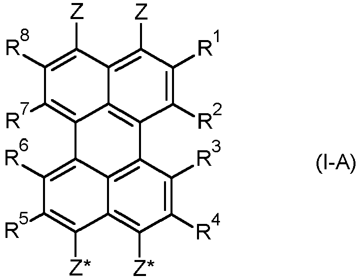 worin
einer der Substituenten Z für Cyano steht und der andere Substituent Z für C₁-C₁₈-Alkyl, C₂-C₁₈-Alkenyl, C₂-C₁₈-Alkinyl, C₃-C₁₂-Cycloalkyl oder C₆-C₁₄-Aryl steht, wobei
   C₁-C₁₈-Alkyl, C₂-C₁₈-Alkenyl, C₂-C₁₈-Alkinyl unsubstituiert sind oder eine oder mehrere, gleiche oder verschiedene Substituenten Z^{a} tragen, wobei Z^{a} die zuvor genannten Bedeutungen aufweist,
   C₃-C₁₂-Cycloalkyl unsubstituiert ist oder eine oder mehrere gleiche oder verschiedene Substituenten Z^{b} trägt, wobei Z^{b} die zuvor genannten Bedeutungen aufweist; und
   C₆-C₁₄-Aryl unsubstituiert ist oder ein- oder mehrere gleiche oder verschiedene Substituenten Z^{Ar} trägt, wobei Z^{Ar} die zuvor genannten Bedeutungen aufweist;
einer der Substituenten Z* für Cyano steht und der andere Substituent Z* für C₁-C₁₈-Alkyl, C₂-C₁₈-Alkenyl, C₂-C₁₈-Alkinyl, C₃-C₁₂-Cycloalkyl oder C₆-C₁₄-Aryl steht, wobei
   C₁-C₁₈-Alkyl, C₂-C₁₈-Alkenyl, C₂-C₁₈-Alkinyl unsubstituiert sind oder eine oder mehrere, gleiche oder verschiedene Substituenten Z^{a} tragen, wobei Z^{a} die zuvor genannten Bedeutungen aufweist,
   C₃-C₁₂-Cycloalkyl unsubstituiert ist oder eine oder mehrere gleiche oder verschiedene Substituenten Z^{b} trägt, wobei Z^{b} die zuvor genannten Bedeutungen aufweist; und
   C₆-C₁₄-Aryl unsubstituiert ist oder ein- oder mehrere gleiche oder verschiedene Substituenten Z^{Ar} trägt, wobei Z^{Ar} die zuvor genannten Bedeutungen aufweist;
R¹, R⁴, R⁵ und R⁸ jeweils für Wasserstoff stehen;
zwei der Substituenten R², R³, R⁶ oder R⁷ für Wasserstoff stehen; und die anderen Substituenten R², R³, R⁶ oder R⁷ für Cyano stehen;
oder Mischungen davon,
erhältlich nach einem Verfahren, bei dem man
   a) Perylen der Formel II 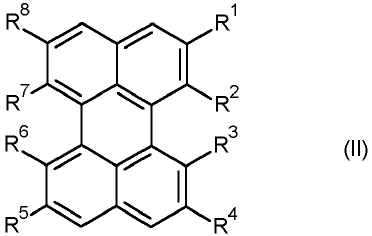 worin
      R¹, R², R³, R⁴, R⁵, R⁶, R⁷ und R⁸ jeweils für Wasserstoff stehen
      halogeniert unter Erhalt einer Mischung aus 3,9-Dihalogenperylen der Formel IIIa und 3,10-Dihalogenperylen der Formel IIIb 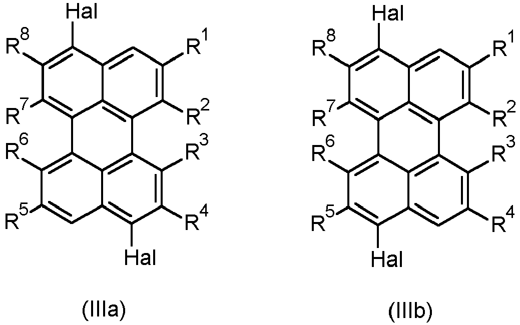 worin
         R¹, R², R³, R⁴, R⁵, R⁶, R⁷ und R⁸ jeweils für Wasserstoff stehen; und
         Hal jeweils alle für Chlor oder Brom stehen;
   b) die in Schritt a) erhaltene Mischung aus Verbindungen der Formeln lila und IIIb mit einer metallorganischen Verbindung der Formel IV

      Z-Met (IV)

      und gegebenenfalls mit einer metallorganischen Verbindung der Formel V

      Z*-Met (V)

      worin
      - Z: ausgewählt ist unter C₁-C₁₈-Alkyl, C₂-C₁₈-Alkenyl, C₂-C₁₈-Alkinyl, C₃-C₁₂-Cycloalkyl und C₆-C₁₄-Aryl, wobei C₁-C₁₈-Alkyl, C₂-C₁₈-Alkenyl, C₂-C₁₈-Alkinyl unsubstituiert sind oder eine oder mehrere, gleiche oder verschiedene Substituenten Z^{a} tragen,
      C₃-C₁₂-Cycloalkyl unsubstituiert ist oder eine oder mehrere gleiche oder verschiedene Substituenten Z^{b} trägt, und
      C₆-C₁₄-Aryl unsubstituiert ist oder ein- oder mehrere gleiche oder verschiedene Substituenten Z^{Ar} trägt;
      - Z*: ausgewählt ist unter C₁-C₁₈-Alkyl, C₂-C₁₈-Alkenyl, C₂-C₁₈-Alkinyl, C₃-C₁₂-Cycloalkyl und C₆-C₁₄-Aryl, wobei C₁-C₁₈-Alkyl, C₂-C₁₈-Alkenyl, C₂-C₁₈-Alkinyl unsubstituiert sind oder eine oder mehrere, gleiche oder verschiedene Substituenten Z^{a} tragen,
      C₃-C₁₂-Cycloalkyl unsubstituiert ist oder eine oder mehrere gleiche oder verschiedene Substituenten Z^{b} trägt, und
      C₆-C₁₄-Aryl unsubstituiert ist oder ein- oder mehrere gleiche oder verschiedene Substituenten Z^{Ar} trägt;
      wobei Z* auch die gleiche Bedeutung wie Z besitzen kann;
      - Met: für B(OH)₂, B(OR')(OR"), Zn-Hal oder Sn(R*)₃ steht, worin
      R' und R" unabhängig voneinander für Wasserstoff, C₁-C₃₀-Alkyl, C₅-C₈-Cycloalkyl, C₆-C₁₄-Aryl oder Heteroaryl stehen oder R' und R" zusammen für C₂-C₄-Alkylen stehen, das gegebenenfalls 1, 2, 3, 4, 5, 6, 7 oder 8 Substituenten trägt, die ausgewählt sind unter C₁-C₄-Alkyl, C₅-C₈-Cycloalkyl, C₆-C₁₄-Aryl und Heteroaryl;
      Hal für Chlor oder Brom steht; und
      R* für C₁-C₈-Alkyl oder Phenyl steht,
      umsetzt unter Erhalt einer Mischung aus Verbindungen der Formeln VIa und Vlb, 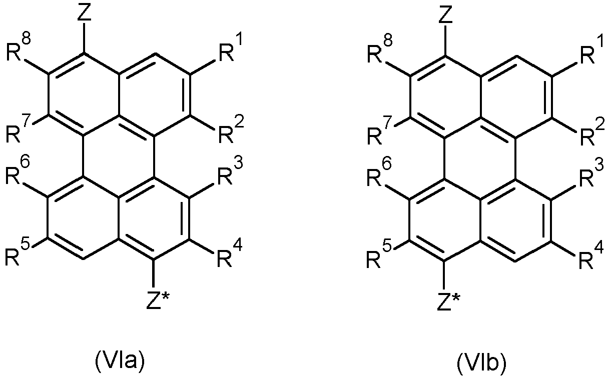 worin
      R¹, R², R³, R⁴, R⁵, R⁶, R⁷ und R⁸ jeweils für Wasserstoff stehen; und
      Z und Z* wie zuvor definiert sind;
   c) die in Schritt b) erhaltene Mischung aus Verbindungen der Formeln VIa und Vlb halogeniert unter Erhalt eines Reaktionsgemisches, das Verbindungen der Formeln Vlla und Vllb enthält, 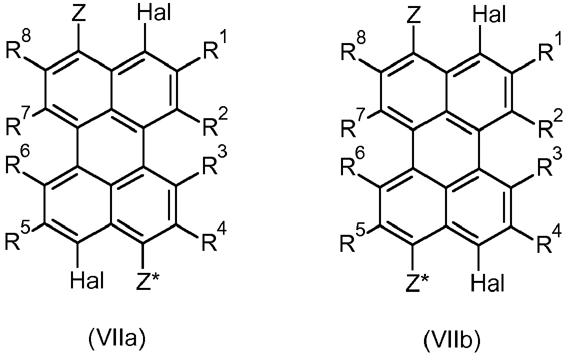 worin
      Z und Z* wie zuvor definiert sind;
      Hal für unter Chlor und Brom ausgewähltes Halogen steht, wobei die Substituenten Hal entweder alle für Chlor oder alle für Brom stehen,
      R¹, R², R³, R⁴, R⁵, R⁶, R⁷ und R⁸ für Wasserstoff oder unter Chlor und Brom ausgewähltes Halogen stehen, wobei die Substituenten R¹, R², R³, R⁴, R⁵, R⁶, R⁷ und R⁸, die nicht für Wasserstoff stehen, entweder alle für Chlor oder alle für Brom stehen;
   d) die in dem in Schritt c) erhaltenen Reaktionsgemisch enthaltenen Verbindungen der Formel Vlla und Vllb einer Substitution von Halogen durch Cyano, sowie gegebenenfalls teilweise durch Wasserstoff, unterzieht unter Erhalt wenigstens einer Verbindung der Formel I-A oder Mischungen davon; und
   e) gegebenenfalls die in dem in Schritt d) erhaltenen Reaktionsgemisch enthaltene wenigstens eine Verbindung der Formel I-A oder Mischungen davon mindestens einem Auftrennungs- und/oder Reinigungsschritt unterzieht.

### Schritt a)

Die Halogenierung von Perylen der Formel II erfolgt üblicherweise mit einem Bromierungsmittel oder einem Chlorierungsmittel, d. h. in den Verbindungen der Formeln IIIa oder IIIb stehen entweder alle Substituenten Hal für Brom oder alle Substituenten Hal stehen für Chlor.

Üblicherweise wird elementares Brom in einem Lösungsmittel als Bromierungsmittel verwendet. Weitere geeignete Bromierungsmittel sind N-Bromsuccinimid und Dibromisocyanursäure. Geeignete Lösungsmittel sind Wasser oder aliphatische Monocarbonsäuren sowie chlorierte Kohlenwasserstoffe wie Chlorbenzol und Chloroform. Geeignete aliphatische Monocarbonsäure sind solche mit 2 bis 6 C-Atomen wie Essigsäure, Propionsäure, Buttersäure, Pentancarbonsäure und Hexancarbonsäure und Mischungen davon. Bei Verwendung einer aliphatischen Monocarbonsäure als Lösungsmittel, kann es vorteilhaft sein, lod als Katalysator einzusetzen.

Geeignete Chlorierungsmittel sind Chlor in einem Lösungsmittel, z. B. Tetrachlormethan. Ebenfalls geeignet sind N-Chlorsuccinimid und Dichlorisocyanursäure. Die Chlorierung mit Dichlorisocyanursäure erfolgt vorzugsweise in konzentrierter Schwefelsäure.

Das Molmengenverhältnis von Bromierungsmittel zu Perylen der Formel II beträgt üblicherweise etwa 10 : 1 bis 2,5 : 1, besonders bevorzugt 9 : 1 bis 3,0 : 1. Das Molmengenverhältnis beträgt insbesondere 8,5 : 1 bis 3,5 : 1.

Das Molmengenverhältnis von Chlorierungsmittel zu Perylen der Formel II beträgt üblicherweise etwa 10 : 1 bis 2,5 : 1, besonders bevorzugt 9 : 1 bis 3,0 : 1. Das Molmengenverhältnis beträgt insbesondere 8,5 : 1 bis 3,5 : 1.

Die in dem Reaktionsschritt a) erhaltenen dihalogenierten Verbindungen der Formeln lila und IIIb werden in der Regel ohne weitere Reinigung in Schritt b) eingesetzt.

### Schritt b)

Bei der Umsetzung in Schritt b) werden die in Schritt a) erhaltenen Verbindungen der Formeln lila und IIIb einer Kreuz-Kupplung mit einer metallorganischen Verbindung der Formel IV und gegebenenfalls mit einer metallorganischen Verbindung der Formel V unterworfen.

Vorzugsweise erfolgt die Umsetzung in Gegenwart katalytisch aktiver Mengen eines Übergangsmetalls der Nebengruppe VIII des Periodensystems (Gruppe 10 nach IUPAC), z. B. Nickel, Palladium oder Platin, insbesondere in Gegenwart eines Palladiumkatalysators. Geeignete Katalysatoren sind beispielsweise Palladium-Phosphin-Komplexe wie Tetrakis(triphenylphosphin)palladium(0), PdCl₂(o-tolyl₃P)₂, Bis(triphenylphosphin)palladium(II)-chlorid, der [1,1'-Bis(diphenylphosphino)ferrocen]palladium(II)-chlorid-Dichlormethan-Komplex, Bis-[1,2-bis(diphenylphosphin)ethan]palladium(0) und [1,4-Bis(diphenylphosphin)butan]palladium(II)-chlorid, Palladium auf Aktivkohle in Gegenwart von Phosphin-Verbindungen sowie Palladium(II)-Verbindungen wie Palladium(II)chlorid oder Bis(acetonitril)palladium(II)-chlorid, in Gegenwart von Phosphinverbindungen wie Triphenylphosphin, 1,1'-Bis(diphenylphosphino)ferrocen, 1,2-Bis(diphenylphosphin)ethan, 1,3-Bis(diphenylphosphin)propan und 1,4-Bis(diphenylphosphin)butan. Die Menge an Katalysator beträgt üblicherweise 10 bis 150 mol-%, bezogen auf die Verbindungen der Formeln lila und IIIb.

Insbesondere geeignete metallorganische Verbindungen IV sind eine entsprechend substituierte Arylboronsäure und Arylboronsäureester (Verbindungen IV mit Met = B(OH)₂ oder B(OR')(OR") mit R', R" = C₁-C₄-Alkyl oder R' und R" stehen zusammen für C₂-C₄-Alkylen stehen, das gegebenenfalls 1, 2, 3 oder 4 Substituenten trägt, die ausgewählt sind unter C₁-C₄-Alkyl).

Die Umsetzung erfolgt unter den Bedingungen einer Suzuki-Kupplung, wie sie z. B. aus Suzuki et al., Chem. Rev., 1995, 95, 2457-2483 und der darin zitierten Literatur bekannt sind. Die Arylboronsäuren und deren Ester sind entweder literaturbekannt, käuflich erhältlich oder lassen sich aus den entsprechenden Arylmagnesiumverbindungen durch Umsetzung mit entsprechenden Borsäureestern herstellen. Geeignete metallorganische Verbindungen IV sind weiterhin Alkylboronsäure oder Alkylboronsäureester

Geeignete metallorganische Verbindungen IV sind insbesondere auch Arylstanne, Cycloalkylstannane, Alkinylstannane, Alkenylstannane oder Alkylstannane (Verbindungen IV mit Met = Sn(R*)₃ mit R* = C₁-C₄-Alkyl). Die Umsetzung erfolgt dann unter den Bedingungen einer Stille-Kupplung, wie sie z. B. aus D. Milstein, J. K. Stille, J. Am. Chem. Soc. 1978, 100, S. 3636-3638 oder V. Farina, V. Krishnamurthy, W. J. Scott, Org. React. 1997, 50, 1-652 bekannt sind. Stannane der Formel IV sind entweder bekannt oder lassen sich nach allgemein bekannten Verfahren darstellen.

Geeignete metallorganische Verbindungen IV sind weiterhin zinkorganische Verbindungen (Verbindungen IV mit Met = Zn-Hal mit Hal = Cl, Br, insbesondere Br). Die Umsetzung erfolgt dann unter den Bedingungen einer Negishi-Kupplung, wie sie z. B. aus A. Lützen, M. Hapke, Eur. J. Org. Chem., 2002, 2292-2297 bekannt sind. Arylzink-Verbindungen der Formel IV oder Alkylzink-Verbindungen der Formel IV sind entweder bekannt oder lassen sich nach allgemein bekannten Verfahren darstellen.

Die Umsetzung von IIIa und IIIb mit der metallorganischen Verbindung IV erfolgt insbesondere im Falle der Suzuki-Kupplung unter basischen Bedingungen. Geeignete Basen sind Alkalimetallcarbonate und Alkalimetallhydrogencarbonate wie Natriumcarbonat, Kaliumcarbonat, Cäsiumcarbonat, Natriumhydrogencarbonat, Erdalkalimetallcarbonate und Erdalkalimetallhydrogencarbonate, wie Magnesiumcarbonat oder Magnesiumhydrogencarbonat oder tertiäre Amine, wie Triethylamin, Trimethylamin, Triisopropylamin oder N-Ethyl-N-diisopropylamin.

Üblicherweise erfolgt die Kupplung der Verbindungen lila und IIIb mit der Verbindung IV in einem Lösungsmittel. Als Lösungsmittel sind organische Solventien wie Aromaten, z. B. Toluol, Mesitylen, acyclische Ether, z. B. 1,2-Dimethoxyethan, cyclische Ether wie Tetrahydrofuran oder 1,4-Dioxan, Polyalkylenglykole wie Diethylenglykol, Carbonsäurenitrile wie Acetonitril, Propionitril, Carbonsäureamide wie Dimethylformamid oder Dimethylacetamid geeignet. Bei der Suzuki-Kupplung können die vorgenannten Lösungsmittel auch im Gemisch mit Wasser eingesetzt werden, z. B. kann das Verhältnis von organischem Lösungsmittel zu Wasser im Bereich von 5 : 1 bis 1 : 5 liegen.

Pro mol auszutauschendes Halogenatom wird mindestens ein mol der metallorganischen Verbindung IV eingesetzt. Es kann vorteilhaft sein, pro mol auszutauschendem Halogenatom einen 5 bis 30%igen molaren Überschuss an metallorganischer Verbindung der Formel IV zu verwenden.

Falls Z von Z* verschieden ist, wird anschließend eine weitere Kupplung mit einer metallorganischen Verbindung der Formel V durchgeführt. Verfahrenstechnisch geht man wie bei der Umsetzung der Verbindung der Formeln lila und IIIb mit der metallorganischen Verbindung IV vor.

### Schritt c)

Die Halogenierung von Verbindungen der Formeln VIa und Vlb erfolgt üblicherweise mit einem Bromierungsmittel oder einem Chlorierungsmittel. Geeignete Bromierungsmittel oder Chlorierungsmittel sind die in Schritt a) genannten. In der Regel beträgt das Molverhältnis von Bromierungsmittel zu halogenierender Verbindung der Formeln VIa und VIb 10 : 1 bis 30: 1, vorzugsweise 15 : 1 bis 25 : 1.

Schritt c) des erfindungsgemäßen Verfahrens wird üblicherweise in Gegenwart eines Lösungsmittels bei erhöhten Temperaturen vorgenommen. Geeignete Lösungsmittel sind aprotische Lösungsmittel wie halogenierte Aromaten wie Chlorbenzol oder Dichlorbenzole oder halogenierte Kohlenwasserstoffe. Geeignet sind weiterhin wässrige aprotische Lösungsmittel.

Es kann vorteilhaft sein, Schritt c) in Gegenwart von katalytischen Mengen lod durchzuführen.

Die Reaktionstemperatur in Schritt c) beträgt üblicherweise 50 °C bis zur Siedetemperatur des Lösungsmittels, vor allem 80 bis 150 °C.

### Schritt d)

Geeignete Verfahrensbedingungen zur Cyano-Dehalogenierung sind in J. March, Advanced Organic Chemistry, 4. Auflage, Verlag John Wiley & Sons (1992), S. 660-661 sowie in der WO 2004/029028 beschrieben. Dazu zählt beispielsweise die Umsetzung mit Kupfercyanid. Geeignet sind weiterhin Alkalicyanide, wie Kaliumcyanid und Natriumcyanid, sowie Zinkcyanid. Üblicherweise wird die Cyanidquelle im Überschuss eingesetzt. Die Umsetzung erfolgt in der Regel in polaren aprotischen Lösungsmitteln in Gegenwart von Übergangsmetallen wie Pd(II)-salzen, Pd-, Kupfer- oder Nickelkomplexen. Der Palladium-Katalysator kann in situ aus Pd(0)-Komplexen wie (Tris-(dibenzylidenaceton)-dipalladium(0) und 1,1'-Bis-(diphenylphosphino)-ferrocen hergestellt werden. Bevorzugte polare aprotische Lösungsmittel sind Dimethylformamid, N-Methylpyrrolidon, (CH₃)₂SO, Dimethylsulfon und Sulfolan. Die Umsetzung wird üblicherweise bei Temperaturen von 80 bis 160, bevorzugt 100 bis 140, insbesondere bevorzugt 130 bis 150 °C durchgeführt. Das Molverhältnis von auszutauschendem Halogenatom zu Zinkcyanid beträgt üblicherweise 1 : 1 bis 1 : 3, vorzugsweise 1,5 : 2,5. Alternativ kann man auch Kupfercyanid in N-Methylpyrrolidon oder Sulfolan verwenden in Abwesenheit eines Katalysators.

### Schritt e)

Gegebenenfalls wird das in Schritt d) erhaltene Reaktionsgemisch, das wenigstens eine Perylen-Verbindung der Formel I-A oder Mischungen davon enthält, einer teilweise oder vollständigen Auftrennung und/oder einem Reinigungsschritt unterzogen. Die Auftrennung und/oder Reinigung in Schritt e) kann nach üblichen, dem Fachmann bekannten Verfahren erfolgen, wie Extraktion, Destillation, Umkristallisation, Auftrennung an geeigneten stationären Phasen sowie einer Kombination dieser Maßnahmen erfolgen.

Es kann vorteilhaft sein, eine teilweise oder vollständige Auftrennung der erhaltenen Isomere bereits nach dem Reaktionsschritt a) und/oder b) und/oder c) vorzunehmen.

Gemäß einer ersten speziellen Ausführungsform dieses Gegenstandes ist eine Zusammensetzung bevorzugt, worin in der Verbindung der Formel I-A
einer der Substituenten Z für Cyano steht und der andere Substituent Z für Phenyl steht, das unsubstituiert ist;
einer der Substituenten Z* für Cyano steht und der andere Substituent Z* für Phenyl steht, das unsubstituiert ist;
R¹, R⁴, R⁵ und R⁸ jeweils für Wasserstoff stehen;
zwei der Substituenten R², R³, R⁶ oder R⁷ für Cyano stehen und die anderen Substituenten R², R³, R⁶ oder R⁷ für Wasserstoff stehen.

Gemäß einer zweiten speziellen Ausführungsform dieses Gegenstandes ist eine Zusammensetzung bevorzugt, worin in der Verbindung der Formel I-A
einer der Substituenten Z für Cyano steht und der andere Substituent Z für Phenyl steht, das 1, 2 oder 3 C₁-C₄-Alkylgruppen trägt;
einer der Substituenten Z* für Cyano steht und der andere Substituent Z* für Phenyl steht, das 1, 2 oder 3 C₁-C₄-Alkylgruppen trägt;
R¹, R⁴, R⁵ und R⁸ jeweils für Wasserstoff stehen;
zwei der Substituenten R², R³, R⁶ oder R⁷ für Cyano stehen und die anderen Substituenten R², R³, R⁶ oder R⁷ für Wasserstoff stehen.

Gemäß einer dritten speziellen Ausführungsform dieses Gegenstandes ist eine Zusammensetzung bevorzugt, worin in der Verbindung der Formel I-A
einer der Substituenten Z für Cyano steht und der andere Substituent Z für C₁-C₆-Alkyl steht;
einer der Substituenten Z* für Cyano steht und der andere Substituent Z* für C₁-C₆-Alkyl steht;
R¹, R⁴, R⁵ und R⁸ jeweils für Wasserstoff stehen;
zwei der Substituenten R², R³, R⁶ oder R⁷ für Cyano stehen und die anderen Substituenten R², R³, R⁶ oder R⁷ für Wasserstoff stehen.

Ein weiterer Gegenstand ist eine Zusammensetzung, enthaltend mindestens eine cyanierte Perylen-Verbindung der Formel I, welche der Formel I-B entspricht 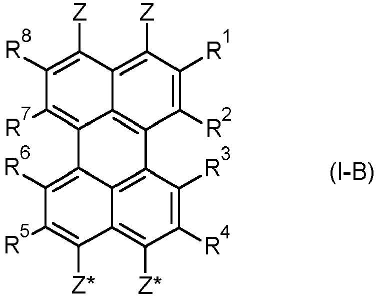 worin
einer der Substituenten Z für Cyano steht und der andere Substituent Z für COOR⁹ steht;
einer der Substituenten Z* für Cyano steht und der andere Substituent Z* für COOR⁹ steht;
R¹, R⁴, R⁵, und R⁸ für Wasserstoff stehen;
einer der Substituenten R², R³, R⁶ oder R⁷ für Cyano steht und die anderen Substituenten R², R³, R⁶ oder R⁷ für Wasserstoff stehen;
R⁹ für Wasserstoff, C₁-C₁₀-Alkyl, C₂-C₁₀-Alkenyl, C₂-C₁₀-Alkinyl, C₃-C₁₂-Cycloalkyl oder C₆-C₁₄-Aryl steht, wobei
   C₁-C₁₀-Alkyl, C₂-C₁₀-Alkenyl, C₂-C₁₀-Alkinyl unsubstituiert sind oder eine oder mehrere gleiche oder verschiedene Substituenten R^{a} tragen, C₃-C₁₂-Cycloalkyl unsubstituiert ist oder eine oder mehrere gleiche oder verschiedene Substituenten R^{b} trägt und
   C₆-C₁₄-Aryl unsubstituiert ist oder ein- oder mehrere gleiche oder verschiedene Substituenten R^{Ar} trägt, wobei R^{a}, R^{b} und R^{Ar} wie zuvor definiert sind
oder Mischungen davon,
erhältlich nach einem Verfahren, bei dem man
   f) eine Mischung aus Perylen-Verbindungen der Formeln VIIIa und VIIIb 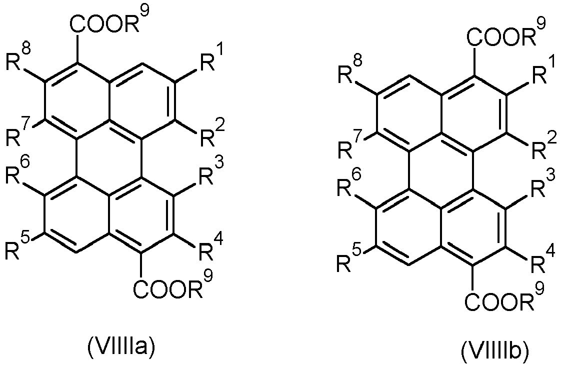 worin
      R¹, R², R³, R⁴, R⁵, R⁶, R⁷ und R⁸ für Wasserstoff; und
      R⁹ wie zuvor definiert ist
      halogeniert unter Erhalt eines Reaktionsgemischs, das Verbindungen der Formeln IXa und IXb, 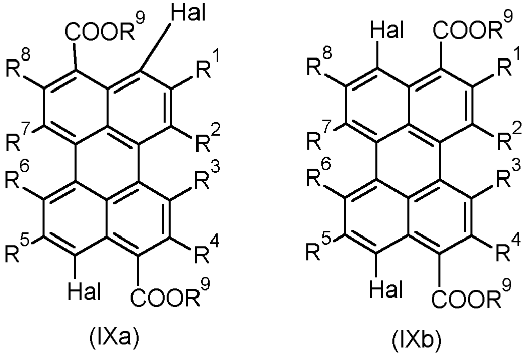 worin
         R¹, R², R³, R⁴, R⁵, R⁶, R⁷ und R⁸ für Wasserstoff oder unter Chlor und Brom ausgewähltes Halogen stehen, wobei die Substituenten R¹, R², R³, R⁴, R⁵, R⁶, R⁷ und R⁸, die nicht für Wasserstoff stehen, entweder alle für Chlor oder alle für Brom stehen;
         Hal für unter Chlor und Brom ausgewähltes Halogen steht, wobei die Substituenten Hal entweder alle für Chlor oder alle für Brom stehen; und
         R⁹ wie zuvor definiert ist;
         oder Mischungen davon, enthält;
   g) die in dem in Schritt f) erhaltenen Reaktionsgemisch enthaltenen Verbindungen der Formeln IXa und IXb einer Substitution von Halogen durch Cyanogruppen, sowie gegebenenfalls teilweise durch Wasserstoff unterzieht unter Erhalt wenigstens einer Verbindung der Formel I-B oder Mischungen davon; und
   h) gegebenenfalls die in dem in Schritt g) erhaltenen Reaktionsgemisch enthaltene wenigstens eine Verbindung der Formel I-B oder Mischungen davon mindestens einem Auftrennungs- und/oder Reinigungsschritt unterzieht.

### Schritt f)

Verfahrenstechnisch wird Schritt f) wie Schritt c) durchgeführt.

### Schritt g)

Verfahrenstechnisch wird Schritt g) wie Schritt d) durchgeführt.

Gemäß einer speziellen Ausführungsform dieses Gegenstandes ist eine Zusammensetzung bevorzugt, worin in der Verbindung der Formel I-B
einer der Substituenten Z für Cyano steht und der andere Substituent Z für C₁-C₆-Alkoxycarbonyl steht;
einer der Substituenten Z* für Cyano steht und der andere Substituent Z* für C₁-C₆-Alkoxycarbonyl steht;
R¹, R⁴, R⁵ und R⁸ jeweils für Wasserstoff stehen;
einer der Substituenten R², R³, R⁶ oder R⁷ für Cyano steht und die anderen Substituenten R², R³, R⁶ oder R⁷ jeweils für Wasserstoff stehen.

Die erfindungsgemäße Verbindung der Formel I und Mischungen davon oder Zusammensetzungen, enthaltend mindestens eine cyanierte Perylen-Verbindung der Formel I und Mischungen davon, wie zuvor definiert eignen sich als Fluoreszenzfarbstoff in Farbkonvertern, für optische Label, zur unsichtbaren Markierung von Produkten, als Fluoreszenzfarbstoffe, vorzugsweise als Fluoreszenzlabel für Biomoleküle, als Pigmente, als Fluoreszenzfarbstoff in einem auf Fluoreszenzkonversion beruhenden Display; in einem lichtsammelnden Kunststoffteil, welches gegebenenfalls mit einer Solarzelle kombiniert ist; als Pigmentfarbstoff in elektrophoretischen Displays; als Fluoreszenzfarbstoff in einer auf Chemolumineszenz basierenden Anwendung.

Die erfindungsgemäße Verbindung der Formel I und Mischungen davon oder Zusammensetzungen, enthaltend mindestens eine cyanierte Perylen-Verbindung der Formel I und Mischungen davon wie zuvor definiert eignen sich besonders vorteilhaft als Fluoreszenzfarbstoff in einem auf Fluoreszenzkonversion beruhenden Display. Derartige Displays umfassen im Allgemeinen ein transparentes Substrat, einen auf dem Substrat befindlichen Fluoreszenzfarbstoff und eine Strahlungsquelle. Übliche Strahlungsquellen senden blaues (color-by-blue) oder UV-Licht (color-by-uv) aus. Die Farbstoffe absorbieren entweder das blaue oder das UV-Licht und werden als Grünemitter eingesetzt. In diesen Displays wird z. B. das rote Licht erzeugt, indem der Rotemitter durch einen blaues oder UV-Licht absorbierenden Grünemitter angeregt wird. Geeignete color-by-blue-Displays sind z. B. in der WO 98/28946 beschrieben. Geeignete color-by- uv-Displays werden z. B. von W. A. Crossland, I. D. Sprigle und A. B. Davey in Photoluminescent LCDs (PL-LCD) using phosphors Cambridge University and Screen Technology Ltd., Cambridge, UK, beschrieben.

Die erfindungsgemäße Verbindung der Formel I und Mischungen davon oder Zusammensetzungen, enthaltend mindestens eine cyanierte Perylen-Verbindung der Formel I und Mischungen davon, wie zuvor definiert eignen sich weiterhin besonders als Fluoreszenzemitter in OLEDs, in denen sie entweder durch Elektrolumineszenz oder durch einen entsprechenden Phosphoreszenzemitter über Förster Energietransfer (FRET) angeregt werden.

Die erfindungsgemäße Verbindung der Formel I und Mischungen davon oder Zusammensetzungen, enthaltend mindestens eine cyanierte Perylen-Verbindung der Formel I und Mischungen davon, wie zuvor definiert eignen sich weiterhin besonders in Chemolumineszenzanwendungen. Dazu zählen so genannte "Glow Sticks". Zu deren Herstellung kann mindestens eine Verbindung der Formel (I) z. B. in einem Alkylphthalat gelöst werden. Eine Anregung der Chemolumineszenz kann durch Mischung eines Oxalsäureesters mit Wasserstoffperoxid erfolgen, z. B. nachdem diese beiden zunächst separaten Komponenten durch Zerbrechen eines Glases gemischt werden. Die resultierende Reaktionsenergie führt zur Anregung und Fluoreszenz der Farbstoffe. Derartige Glow Sticks können als Notlicht, z. B. beim Angeln, in Seenotrettungswesten oder anderen Sicherheitsanwendungen eingesetzt werden.

Die erfindungsgemäße Verbindung der Formel I und Mischungen davon oder Zusammensetzungen, enthaltend mindestens eine cyanierte Perylen-Verbindung der Formel I und Mischungen davon, wie zuvor definiert eignen sich insbesondere als Fluoreszenzfarbstoff in Farbkonvertern für Solarzellen.

Ein weiterer Gegenstand der vorliegenden Erfindung sind Farbkonverter, enthaltend mindestens ein Polymer als Matrixmaterial und mindestens eine cyanierte Perylen-Verbindung der Formel I oder Mischungen davon wie zuvor definiert oder Zusammensetzungen, enthaltend mindestens eine cyanierte Perylen-Verbindung der Formel I und Mischungen davon wie zuvor definiert als Fluoreszenzfarbstoff.

Ein weiterer Gegenstand der vorliegenden Erfindung sind Farbkonverter, enthaltend (i) mindestens ein Polymer als Matrixmaterial und (ii) mindestens eine erfindungsgemäße cyanierte Perylen-Verbindung der Formel I oder Mischungen davon oder Zusammensetzungen, enthaltend mindestens eine cyanierte Perylen-Verbindung der Formel I und Mischungen davon wie zuvor definiert als Fluoreszenzfarbstoff.

### Komponente (i)

Geeignete Polymere sind prinzipiell alle Polymere, die in der Lage sind, die mindestens eine cyanierte Perylen-Verbindung der Formel I oder Mischungen davon in ausreichender Menge zu lösen oder homogen zu verteilen.

Geeignete Polymere können anorganische Polymere oder organische Polymere sein.

Geeignete anorganische Polymere sind zum Beispiel Silikate oder Siliziumdioxid. Voraussetzung für die Verwendung von anorganischen Polymeren ist, dass die mindestens eine cyanierte Perylen-Verbindung der Formel I oder Mischungen davon hierin zersetzungsfrei gelöst oder homogen verteilt werden kann. Dieses kann im Falle von Silikaten oder Siliziumdioxid zum Beispiel durch Abscheiden des Polymers aus einer Wasserglaslösung geschehen.

Gemäß einer bevorzugten Ausführungsform bestehen die organischen Polymere im Wesentlichen aus Polystyrol, Polycarbonat, Polymethylmethacrylat, Polyvinylpyrrolidon, Polymethacrylat, Polyvinylacetat, Polyvinylchlorid, Polybuten, Polyethylenglykol, Silikon, Polyacrylat, Epoxidharz, Polyvinylalkohol, EthylenVinylalkohol-Copolymer (EVOH), Polyacrylnitril, Polyvinylidenchlorid (PVDC), Polystyrolacrylnitril (SAN), Polybutylenterephthalat (PBT), Polyethylenterephthalat (PET), Polyvinylbutyrat (PVB), Polyvinylchlorid (PVC), Polyamide, Polyoxymethylene, Polyimide, Polyetherimid oder Mischungen hiervon.

Bevorzugt besteht das mindestens eine Polymer im Wesentlichen aus Polystyrol (PS), Polycarbonat (PC), Polymethylmethacrylat (PMMA), Polyethylenterephthalat (PET) oder Mischungen hiervon.

Ganz besonders bevorzugt besteht das mindestens eine Polymer im Wesentlichen aus Polyethylenterephthalat, Polystyrol oder Polycarbonat.

Polyethylenterephthalat ist durch Kondensation von Ethylenglykol mit Terephthalsäure erhältlich.

Als Polystyrol verstanden seien hierbei unter anderem alle Homo- oder Copolymere, die durch Polymerisation von Styrol und/oder Derivaten des Styrols entstehen. Derivate des Styrols sind zum Beispiel Alkylstyrole wie alpha-Methylstyrol, ortho-, meta-, para-Methylstyrol, para-Butylstyrol insbesondere para-tert-Butylstyrol, Alkoxystyrol wie para-Methoxystyrol, para-Butoxystyrol, para-tert-Butoxystyrol.

In der Regel haben geeignete Polystyrole eine mittlere molare Masse Mₙ von 10.000 bis 1.000.000 g/mol (bestimmt durch GPC) bevorzugt 20.000 bis 750.000 g/mol, besonders bevorzugt 30.000 bis 500.000 g/mol.

In einer bevorzugten Ausführungsform besteht die Matrix des Farbkonverters im Wesentlichen oder vollständig aus einem Homopolymer von Styrol oder Styrolderivaten.

In weiteren bevorzugten Ausführungsformen der Erfindung besteht die Matrix im Wesentlichen oder vollständig aus einem Styrol-Copolymer, die im Rahmen dieser Anmeldung ebenfalls als Polystyrol angesehen werden. Styrol Copolymere können als weitere Bestandteile zum Beispiel Butadien, Acrylnitril, Maleinsäureanhydrid, Vinylcarbazol oder Ester der Acryl-, Methacryl- oder Itaconsäure als Monomere enthalten. Geeignete Styrolcopolymere enthalten in der Regel mindestens 20 Gew.-% Styrol bevorzugt mindestens 40 und besonders bevorzugt mindestens 60 Gew.-% Styrol. In einer anderen Ausführungsform enthalten sie mindestens 90 Gew.-% Styrol.

Bevorzugte Styrol Copolymere sind Styrol-Acrylnitril-Copolymere (SAN) und AcrylnitrilButadien-Styrol-Copolymere (ABS), Styrol-1,1'-Diphenylethen-Copolymere, Acrylester-Styrol-Acrylnitril-Copolymere (ASA), Methylmethacrylat-Acrylnitril-Butadien-StyrolCopolymere (MABS).

Ein weiteres bevorzugtes Polymer ist alpha-Methylstyrol - Acrylnitril-Copolymer (AMSAN).

Die Styrol Homo- oder Copolymere können zum Beispiel durch radikalische Polymerisation, kationische Polymerisation, anionische Polymerisation oder unter dem Einfluss metallorganischer Katalysatoren (zum Beispiel Ziegler-Natta Katalyse) hergestellt werden. Dies kann zu isotaktischem, syndiotaktischem, ataktischem Polystyrol bzw. Copolymeren führen. Bevorzugt werden sie durch radikalische Polymerisation hergestellt. Die Polymerisation kann als Suspensionspolymerisation, Emulsionspolymerisation, Lösungspolymerisation oder Massepolymerisation durchgeführt werden.

Die Herstellung geeigneter Polystyrole ist zum Beispiel beschrieben in Oscar Nuyken, Polystyrenes and Other Aromatic Polyvinyl Compounds, in Kricheldorf, Nuyken, Swift, New York 2005, S. 73-150 und darin zitierte Literaturstellen; sowie in Elias, Macromolecules, Weinheim 2007, S. 269-275.

Polycarbonate sind Polyester der Kohlensäure mit aromatischen oder aliphatischen Dihydroxyverbindungen. Bevorzugte Dihydroxyverbindungen sind zum Beispiel Methylendiphenylendihydroxyverbindungen wie zum Beispiel Bisphenol A.

Eine Möglichkeit der Herstellung von Polycarbonaten ist die Umsetzung geeigneter Dihydroxyverbindungen mit Phosgen in einer Grenzflächenpolymerisation. Eine andere Möglichkeit ist die Umsetzung mit Diestern der Kohlensäure wie Diphenylcarbonat in einer Kondensationspolymerisation.

Die Herstellung geeigneter Polycarbonate ist zum Beispiel beschrieben in Elias, Macromolecules, Weinheim 2007, S. 343-347.

In einer bevorzugten Ausführungsform werden Polymere verwendet, die unter Ausschluss von Sauerstoff polymerisiert worden sind. Bevorzugt enthielten die Monomere in Summe während der Polymerisation höchstens 1000 ppm Sauerstoff, besonders bevorzugt höchstens 100 ppm und insbesondere bevorzugt höchstens 10 ppm.

Geeignete Polymere können als weitere Bestandteile Additive wie Flammschutzmittel, Antioxidantien, Lichtschutzmittel, UV-Absorber, Radikalfänger, Antistatika enthalten. Derartige Stabilisatoren sind dem Fachmann bekannt.

Geeignete Antioxidantien oder Radikalfänger sind zum Beispiel Phenole, insbesondere sterisch gehinderte Phenole wie Butylhydroxyanisol (BHA) oder Butylhydroxytoluol (BHT), oder sterisch gehinderte Amine (HALS). Derartige Stabilisatoren werden zum Beispiel von der Firma BASF unter dem Handelsnamen Irganox® vertrieben. In einigen Fällen können Antioxidantien und Radikalfänger durch Sekundärstabilisatoren wie Phosphite oder Phosphonite ergänzt werden, wie sie zum Beispiel von der Firma BASF unter dem Handelsnamen Irgafos® vertrieben werden.

Geeignete UV Absorber sind zum Beispiel Benzotriazole wie 2-(2-Hydroxyphenyl)-2H-benzotriazole (BTZ), Triazine wie (2-Hydroxyphenyl)-s-triazine (HPT), Hydroxybenzophenone (BP) oder Oxalanilide. Derartige UV Absorber werden zum Beispiel von der Firma BASF unter dem Handelsnamen Uvinul® vertrieben.

In einer bevorzugten Ausführungsform wird TiO₂ als alleiniger UV-Absorber eingesetzt.

In einer bevorzugten Ausführungsform der Erfindung enthalten geeignete Polymere keine Antioxidantien oder Radikalfänger.

In einer weiteren Ausführungsform der Erfindung handelt es sich bei geeigneten Polymeren um transparente Polymere.

In einer anderen Ausführungsform handelt es sich bei geeigneten Polymeren um opake Polymere.

Die genannten Polymere dienen als Matrixmaterial für geeignete organische Fluoreszenzfarbstoffe.

### Komponente (ii)

Komponente (ii) enthält mindestens eine cyanierte Perylen-Verbindung der Formel I oder Mischungen davon oder eine Zusammensetzung, enthaltend mindestens eine cyanierte Perylen-Verbindung der Formel I oder Mischungen davon. Insbesondere bevorzugt ist die cyanierte Perylen-Verbindung der Formel I ausgewählt unter Verbindungen der Formeln (1), (2), (3), (4), (5), (6), (7), (8), (9), (10), (11), (12), (13), (14), (15), (16), (17), (18), (19), (20) 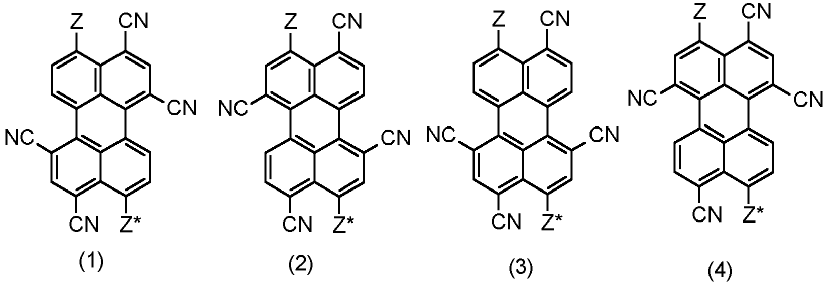 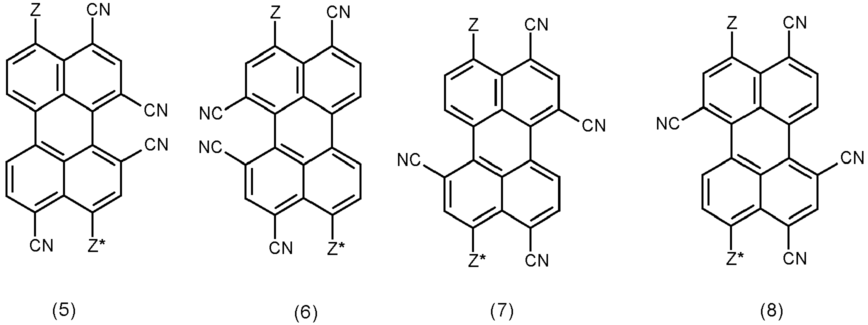 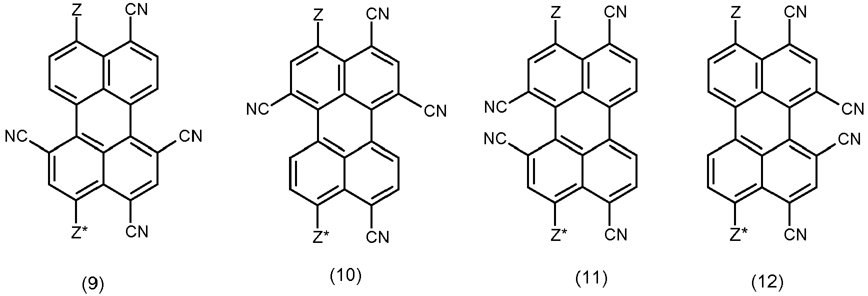 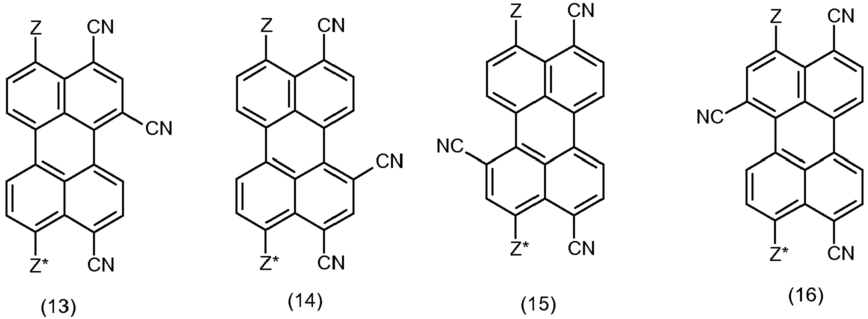 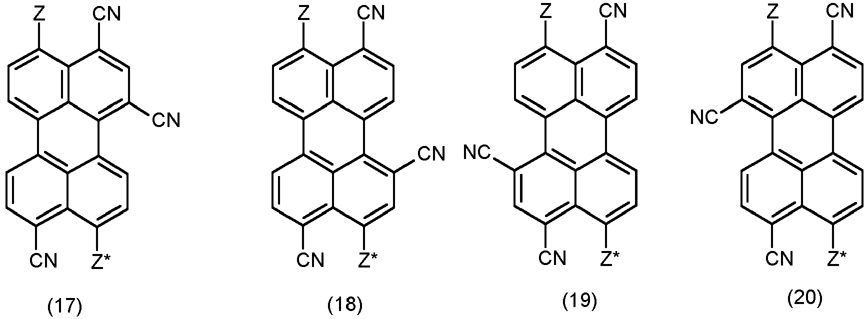 worin
- Z: ausgewählt ist unter C₁-C₆-Alkyl, C₁-C₆-Alkoxycarbonyl, Phenyl und Phenyl, das 1, 2 oder 3 C₁-C₄-Alkylgruppen trägt; und
- Z*: ausgewählt ist unter C₁-C₆-Alkyl, C₁-C₆-Alkoxycarbonyl, Phenyl und Phenyl, das 1, 2 oder 3 C₁-C₄-Alkylgruppen trägt.

Hierunter speziell bevorzugt sind cyanierte Perylen-Verbindungen der Formeln (1) bis (20), worin Z und Z* jeweils die gleiche Bedeutung aufweisen.

Speziell bevorzugt sind cyanierte Perylen-Verbindungen der Formeln (1), (2), (3), (4), (5), (6), (7), (8), (9), (10), (11), (12) und Mischungen davon, worin Z und Z* jeweils für Isopropyl stehen.

Gleichermaßen speziell bevorzugt sind cyanierte Perylen-Verbindungen der Formeln (1), (2), (3), (4), (5), (6), (7), (8), (9), (10), (11), (12) und Mischungen davon, worin Z und Z* jeweils für Phenyl stehen.

Gleichermaßen speziell bevorzugt sind cyanierte Perylen-Verbindungen der Formeln (1), (2), (3), (4), (5), (6), (7), (8), (9), (10), (11), (12) und Mischungen davon, worin Z und Z* jeweils für 2-Methylphenyl stehen.

Gleichermaßen speziell bevorzugt sind cyanierte Perylen-Verbindungen der Formeln (1), (2), (3), (4), (5), (6), (7), (8), (9), (10), (11), (12) und Mischungen davon, worin Z und Z* jeweils für 2,6-Dimethylphenyl stehen.

Gleichermaßen speziell bevorzugt sind cyanierte Perylen-Verbindungen der Formeln (13), (14), (15), (16), (17), (18), (19), (20) und Mischungen davon, worin Z und Z* jeweils für Isobutylcarboxy stehen.

Die cyanierte Perylen-Verbindung der Formel I oder Mischungen davon oder die Zusammensetzung enthaltend mindestens eine cyanierte Perylen-Verbindung der Formel I und Mischungen davon können entweder in dem Polymer gelöst vorliegen oder als homogen verteilte Mischung. Bevorzugt liegt die Perylen-Verbindung der Formel I oder Mischungen davon in dem Polymer gelöst vor. Ebenfalls bevorzug liegt die Zusammensetzung, die mindestens eine cyanierte Perylen-Verbindung der Formel I oder Mischungen davon enthält, in dem Polymer gelöst vor.

In einer bevorzugten Ausführungsform enthalten erfindungsgemäße Farbkonverter noch weitere Fluoreszenzfarbmittel. Geeignete weitere Fluoreszenzfarbmittel sind beispielsweise rot fluoreszierende Fluoreszenzfarbmittel. In vielen Fällen werden Fluoreszenzfarbmittel so miteinander kombiniert, dass Farbkonverter erhalten werden, welche blaues Licht mit guter Farbwiedergabe in weißes Licht umwandeln können.

Geeignete weitere Fluoreszenzfarbmittel sind zum Beispiel anorganische Fluoreszenzfarbmittel. Hierunter besonders bevorzugt sind solche aus der Klasse der mit Seltenen Erden dotierten Aluminate, Silicate, Nitride und Granate. Solche weitere anorganische Leuchtfarbmittel sind zum Beispiel die in "Luminescence - from Theory to Applications", Cees Ronda [Hrsg.], Wiley-VCH, 2008, Kapitel 7, "Luminescent Materials for Phosphor-Converted LEDs", Th. Jüstel, Seite 179-190, genannten.

Granate sind Verbindungen der allgemeinen Formel X₃Y₂[ZO₄]₃, worin Z ein zweiwertiges Kation wie Ca, Mg, Fe, Mn ist, Y ist ein dreiwertiges Kation wie Al, Fe, Cr, Seltene Erden und Z ist Si, Al, Fe³⁺, Ga³⁺. Vorzugsweise ist das Granat Yttrium-Aluminium-Granat Y₃Al₅O₁₂ dotiert mit Ce³⁺, Gd³⁺, Sm³⁺, Eu²⁺, Eu ³⁺, Dy³⁺,Tb³⁺ oder Mischungen davon.

Geeignete Nitride sind beispielsweise in der US 8,274,215 beschrieben, worauf hiermit im vollen Umfang Bezug genommen wird. Geeignete Silicate sind beispielsweise in der US 7,906,041 und US 7,311,858 beschrieben, worauf hiermit im vollen Umfang Bezug genommen wird.

Geeignete Aluminate sind beispielsweise in der US 7,755,276 beschrieben, worauf hiermit im vollen Umfang Bezug genommen wird.

Geeignete Aluminat-Phosphore der Formel SrLu₂₋ₓAl₄O₁₂:Ceₓ, worin x für einen Wert aus dem Bereich von 0,01 bis 0,15 steht, sind aus der WO2012010244 bekannt. Leuchtstoffe der Zusammensetzung MLn₂QR₄O₁₂, wobei M für mindestens eines der Elemente Mg, Ca, Sr oder Ba steht, Ln für mindestens eines der Elemente Sc, Y, La, Ce, Pr, Nd, Sm, Eu, Gd, Tb, Dy, Ho, Er, Tm, Yb und Lu; Q für eines der Elemente Si, Ge, Sn, und Pb und R schließlich für mindestens eines der Elemente B, Al, Ga, In und Tl sind aus der US 2004/0062699 bekannt.

Weiterhin sind alle organischen roten oder rosafarbenen Fluoreszenzfarbstoffe besonders geeignet. In einer weiteren Ausführungsform umfassen weitere Fluoreszenzfarbmittel weitere orange oder gelb fluoreszierende Fluoreszenzfarbstoffe. Geeignete organische fluoreszierende Rotfarbstoffe haben zum Beispiel die allgemeine Formel X 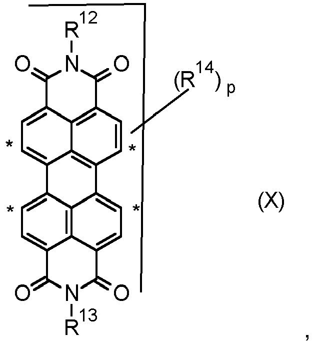 wobei
- p: für 1 bis 4 steht,
- R¹², R¹³: unabhängig voneinander für C₁-C₃₀-Alkyl, C₃-C₈-Cycloalkyl, C₆-C₁₄-Aryl, Hetaryl, C₆-C₁₄-Aryl-C₁-C₁₀-alkylen stehen, wobei in den drei letztgenannten Resten der aromatische Ring unsubstituiert ist oder ein- oder mehrfach durch C₁-C₁₀-Alkyl substituiert ist, und
- R¹⁴: für C₁-C₃₀-Alkoxy oder C₆-C₁₄-Aryloxy, das unsubstituiert ist oder ein- oder mehrfach durch C₁-C₁₀-Alkyl substituiert ist, steht, wobei die Reste R¹⁴ sich an einer oder mehrerer der mit * gekennzeichneten Positionen befinden.

Bevorzugt sind R¹² und R¹³ unabhängig voneinander ausgewählt unter C₁-C₁₀-Alkyl, 2,6-Di-(C₁-C₁₀-alkyl)aryl und 2,4-Di-(C₁-C₁₀-alkyl)aryl. Besonders bevorzugt sind R¹² und R¹³ identisch. Ganz besonders stehen R¹² und R¹³ jeweils für 2,6-Diisopropylphenyl oder 2,4-Di-tert-butylphenyl.

R¹⁴ ist bevorzugt Phenoxy oder C₁-C₁₀-Alkylphenoxy, besonders bevorzugt 2,6-Dialkylphenoxy, 2,4-Dialkylphenoxy. Insbesondere bevorzugt ist R¹⁴ Phenoxy, 2,6-Diisopropylphenoxy, 2,4-Di-tert-butylphenoxy oder 4-tert-Octylphenoxy.

Insbesondere sind geeignete weitere organische Fluoreszenzfarbstoffe ausgewählt unter den Verbindungen der Formeln X-1, X-2 und X-3, 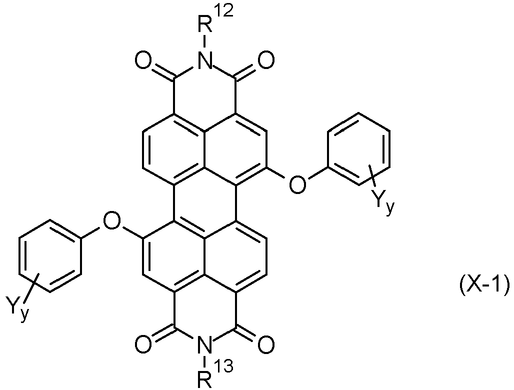 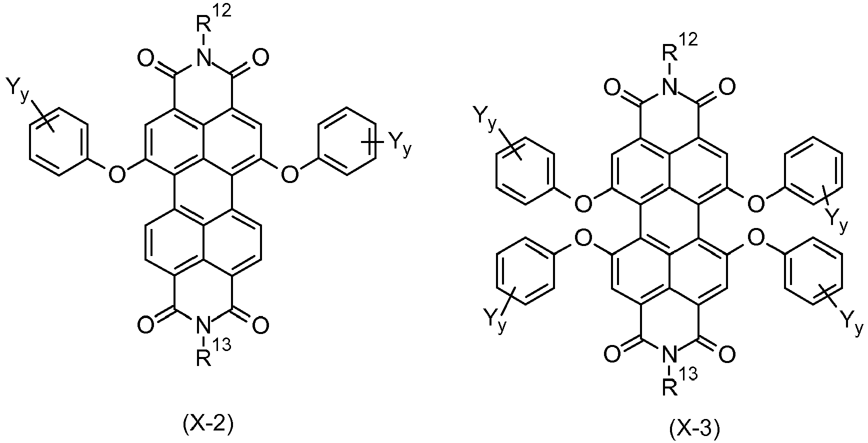 worin
R¹² und R¹³ die oben genannten Bedeutungen aufweisen und insbesondere die als bevorzugt genannten,
Y für lineares oder verzweigtes C₁-C₁₀-Alkyl steht; und
y für 0, 1, 2 oder 3 steht.

Weitere Beispiele für besonders geeignete weitere organische Fluoreszenzfarbstoffe sind die in der WO2007/006717 auf S. 1, Z. 5 bis S. 22, Z. 6 genannten Perylenderivate.

Besonders geeignete weitere organische Fluoreszenzfarbstoffe sind: N,N'-Bis(2,6-diisopropylphenyl)-1,6,7,12-tetraphenoxyperylen-3,4;9,10-tetracarbonsäurediimid, N,N'-Bis(2,6-diisopropylphenyl)-1,7-di(2,6-diisopropylphenoxy)perylen-3,4;9,10-tetracarbonsäurediimid, N,N'-Bis(2,6-diisopropylphenyl)-1,6-di(2,6-diiso-propylphenoxy)perylen-3,4;9,10-tetracarbonsäurediimid, N,N'-(Bis(2,6-diisopropylphenyl)-1,7-di(p-tert-octylphenoxy)-perylen-3,4;9,10-tetracarbonsäurediimid, N,N'-(Bis(2,6-diisopropylphenyl)-1,6-di(p-tert-octylphenoxy)-perylen-3,4;9,10-tetracarbonsäurediimid, N,N' Bis-(2,6-diisopropylphenyl)-1,7-diphenoxy-perylen-3,4;9,10-tetracarbonsäurediimid, N,N' Bis-(2,6-diisopropylphenyl)-1,6-diphenoxy-perylen-3,4;9,10-tetracarbonsäurediimid. Vorzugsweise ist der weitere organische Fluoreszenzfarbstoff ausgewählt unter N,N'-Bis(2,6-diisopropylphenyl)-1,7-di(2,6-diisopropylphenoxy)perylen-3,4;9,10-tetracarbonsäurediimid, N,N'-Bis(2,6-diisopropylphenyl)-1,6-di(2,6-diisopropylphenoxy)perylen-3,4;9,10-tetracarbonsäurediimid und Mischungen davon.

In einer weiteren Ausführungsform enthalten erfindungsgemäße Farbkonverter zusätzlich mindestens einen weiteren organischen Fluoreszenzfarbstoff der Formel XI und XII 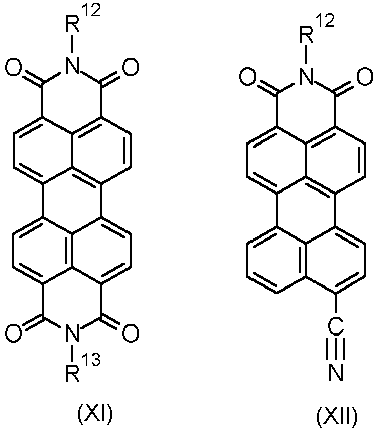 wobei R¹² und R¹³ dieselbe Bedeutung haben wie oben.

In einer Ausführungsform der Erfindung haben erfindungsgemäße Farbkonverter einen Schichtaufbau. Sie können dabei entweder einen Einschichtaufbau aufweisen oder einen Mehrschichtaufbau, in der Regel aus mehreren Polymerschichten, die einen oder mehrere Fluoreszenzfarbmittel und/oder Streukörper enthalten.

In einer Ausführungsform bestehen die Farbkonverter aus mehreren Polymerschichten, welche zu einem Verbund zusammenlaminiert worden sind und wobei sich die verschiedenen Fluoreszenzfarbmittel und/oder Streukörper in unterschiedlichen Polymerschichten befinden können.

Enthalten erfindungsgemäße Farbkonverter mehr als ein Fluoreszenzfarbmittel, so können in einer Ausführungsform der Erfindung mehrere Fluoreszenzfarbmittel in einer Schicht nebeneinander vorliegen.

In einer anderen Ausführungsform liegen die verschiedenen Fluoreszenzfarbmittel in verschiedenen Schichten vor.

In einer bevorzugten Ausführungsform enthalten erfindungsgemäße Farbkonverter zusätzlich mindestens einen weiteren organischen Fluoreszenzfarbstoff gemäß Formel (X), Streukörper auf der Basis von TiO₂ und mindestens ein Polymer, das im Wesentlichen aus Polystyrol, Polyethylenterephthalat (PET) oder Polycarbonat besteht.

In einer weiteren bevorzugten Ausführungsform enthalten erfindungsgemäße Farbkonverter zusätzlich mindestens einen weiteren organischen Fluoreszenzfarbstoff gemäß Formel (X) und mindestens einen weiteren organischen Fluoreszenzfarbstoff gemäß den Formeln (XI) oder (XII), Streukörper auf der Basis von TiO₂ und mindestens ein Polymer, das im Wesentlichen aus Polystyrol, Polyethylenterephthalat oder Polycarbonat besteht.

In einer besonders bevorzugten Ausführungsform enthalten erfindungsgemäße Farbkonverter mindestens eine Verbindung der Formel I oder Mischungen davon oder eine Zusammensetzung, enthaltend mindestens eine Verbindung der Formel I oder Mischungen davon, einen weiteren roten organischen Fluoreszenzfarbstoff ausgewählt aus N,N'-Bis(2,6-diisopropylphenyl)-1,6,7,12-tetraphenoxyperylen-3,4;9,10-tetracarbonsäurediimid, N,N'-Bis(2,6-diisopropylphenyl)-1,7-di(2,6-diisopropylphenoxy)perylen-3,4;9,10-tetracarbonsäurediimid, N,N'-Bis(2,6-diisopropylphenyl)-1,6-di(2,6-diiso-propylphenoxy)perylen-3,4;9,10-tetracarbonsäurediimid, sowie mindestens einen weiteren organischen Fluoreszenzfarbstoff ausgewählt aus N,N'-Bis(2,6-diisopropylphenyl)-perylen-3,4;9,10-tetracarbonsäurediimid oder N'-(2,6-Diisopropylphenyl)-perylen-9-cyano-3,4-dicarbonsäurediimid, einen Streukörper auf der Basis von TiO₂ und mindestens ein Polymer, das im Wesentlichen aus Polystyrol, Polyethylenterephthalat oder Polycarbonat besteht.

Typischerweise liegt die Konzentration an erfindungsgemäßen organischen Fluoreszenzfarbstoff der Formel I oder Mischungen davon bei 0,001 bis 0,5 Gew.-%, bevorzugt 0,005 bis 0,2 Gew.-%, ganz besonders bevorzugt 0,01 bis 0,1 Gew.-%, jeweils bezogen auf die eingesetzte Menge an Polymer. Typischerweise liegt die Konzentration des roten organischen Fluoreszenzfarbstoffs bei 0,0001 bis 0,5 Gew.-%, bevorzugt 0,002 bis 0,1 Gew.-%, ganz besonders bevorzugt 0,005 bis 0,05 Gew.-%, bezogen auf die Menge des eingesetzten Polymers.

Das Verhältnis von mindestens einem erfindungsgemäßen organischen Fluoreszenzfarbstoff der Formel I oder Mischungen davon zu mindestens einem weiteren roten organischen Fluoreszenzfarbstoff liegt üblicherweise im Bereich von 4 : 1 bis 15 : 1, vorzugsweise 6 : 1 bis 12 : 1.

In einer ganz besonders bevorzugten Ausführungsform enthalten erfindungsgemäße Farbkonverter
- als organischen Fluoreszenzfarbstoff mindestens eine erfindungsgemäße Verbindung der Formel I, vorzugsweise ausgewählt unter Verbindungen der Formeln (1), (2), (3), (4), (5), (6), (7), (8), (9), (10), (11), (12) und Mischungen davon, oder eine Zusammensetzung, enthaltend mindestens eine cyanierte Perylen-Verbindung der Formel I, vorzugsweise ausgewählt unter Verbindungen der Formeln (1), (2), (3), (4), (5), (6), (7), (8), (9), (10), (11), (12) und Mischungen davon;
- des Weiteren als roten organischen Fluoreszenzfarbstoff N,N'-Bis(2,6-diisopropylphenyl)-1,7-di(2,6-diisopropylphenoxy)perylen-3,4;9,10-tetracarbonsäurediimid, und/oder N,N'-Bis(2,6-diisopropylphenyl)-1,6-di(2,6-diisopropylphenoxy)perylen-3,4;9,10-tetracarbonsäurediimid;
- Streukörper auf der Basis von TiO₂; und
- mindestens ein Polymer, das im Wesentlichen aus Polystyrol besteht.

Hierunter speziell bevorzugt sind Perylen-Verbindungen der Formeln (1), (2), (3), (4), (5), (6), (7), (8), (9), (10), (11), (12) und Mischungen davon, worin Z und Z* die gleiche Bedeutung aufweisen.

In einer weiteren ganz besonders bevorzugten Ausführungsform enthalten erfindungsgemäße Farbkonverter
- als organischen Fluoreszenzfarbstoff mindestens eine erfindungsgemäße Verbindung der Formel I, vorzugsweise ausgewählt unter Verbindungen der Formeln (13), (14), (15), (16), (17), (18), (19), (20) und Mischungen davon, oder eine Zusammensetzung, enthaltend mindestens eine cyanierte Perylen-Verbindung der Formel I, vorzugsweise ausgewählt unter Verbindungen der Formeln (13), (14), (15), (16), (17), (18), (19), (20) und Mischungen davon;
- des Weiteren als roten organischen Fluoreszenzfarbstoff N,N'-Bis(2,6-diisopropylphenyl)-1,7-di(2,6-diisopropylphenoxy)perylen-3,4;9,10-tetracarbonsäurediimid, und/oder N,N'-Bis(2,6-diisopropylphenyl)-1,6-di(2,6-diisopropylphenoxy)perylen-3,4;9,10-tetracarbonsäurediimid;
- Streukörper auf der Basis von TiO₂; und
- mindestens ein Polymer, das im Wesentlichen aus Polystyrol besteht.

Hierunter speziell bevorzugt sind Perylen-Verbindungen der Formeln (13), (14), (15), (16), (17), (18), (19), (20) und Mischungen davon, worin Z und Z* die gleiche Bedeutung aufweisen.

In einer weiteren ganz besonders bevorzugten Ausführungsform enthalten erfindungsgemäße Farbkonverter
- als organischen Fluoreszenzfarbstoff mindestens eine erfindungsgemäße Verbindung der Formel I, vorzugsweise ausgewählt unter Verbindungen der Formeln (1), (2), (3), (4), (5), (6), (7), (8), (9), (10), (11), (12) und Mischungen davon, oder eine Zusammensetzung, enthaltend mindestens eine cyanierte Perylen-Verbindung der Formel I, vorzugsweise ausgewählt unter Verbindungen der Formeln (1), (2), (3), (4), (5), (6), (7), (8), (9), (10), (11), (12) und Mischungen davon;
- des Weiteren als roten organischen Fluoreszenzfarbstoff N,N'-Bis(2,6-diisopropylphenyl)-1,7-di(2,6-diisopropylphenoxy)perylen-3,4;9,10-tetracarbonsäurediimid, und/oder N,N'-Bis(2,6-diisopropylphenyl)-1,6-di(2,6-diisopropylphenoxy)perylen-3,4;9,10-tetracarbonsäurediimid;
- Streukörper auf der Basis von TiO₂; und
- mindestens ein Polymer, das im Wesentlichen aus PET besteht.

Hierunter speziell bevorzugt sind Perylen-Verbindungen der Formeln (1), (2), (3), (4), (5), (6), (7), (8), (9), (10), (11), (12) und Mischungen davon, worin Z und Z* die gleiche Bedeutung aufweisen.

In einer weiteren ganz besonders bevorzugten Ausführungsform enthalten erfindungsgemäße Farbkonverter
- als organischen Fluoreszenzfarbstoff mindestens eine erfindungsgemäße Verbindung der Formel I, vorzugsweise ausgewählt unter Verbindungen der Formeln (13), (14), (15), (16), (17), (18), (19), (20) und Mischungen davon, oder eine Zusammensetzung, enthaltend mindestens eine cyanierte Perylen-Verbindung der Formel I, vorzugsweise ausgewählt unter Verbindungen der Formeln (13), (14), (15), (16), (17), (18), (19), (20) und Mischungen davon;
- des Weiteren als roten organischen Fluoreszenzfarbstoff N,N'-Bis(2,6-diisopropylphenyl)-1,7-di(2,6-diisopropylphenoxy)perylen-3,4;9,10-tetracarbonsäure-diimid, und/oder N,N'-Bis(2,6-diisopropylphenyl)-1,6-di(2,6-diisopropylphenoxy)-perylen-3,4;9,10-tetracarbonsäurediimid;
- Streukörper auf der Basis von TiO₂; und
- mindestens ein Polymer, das im Wesentlichen aus PET besteht.

Hierunter speziell bevorzugt sind Perylen-Verbindungen der Formeln (13), (14), (15), (16), (17), (18), (19), (20) und Mischungen davon, worin Z und Z* die gleiche Bedeutung aufweisen.

In einer weiteren ganz besonders bevorzugten Ausführungsform enthalten erfindungsgemäße Farbkonverter
- als organischen Fluoreszenzfarbstoff mindestens eine erfindungsgemäße Verbindung der Formel I, vorzugsweise ausgewählt unter Verbindungen der Formeln (1), (2), (3), (4), (5), (6), (7), (8), (9), (10), (11), (12) und Mischungen davon, oder eine Zusammensetzung, enthaltend mindestens eine cyanierte Perylen-Verbindung der Formel I, vorzugsweise ausgewählt unter Verbindungen der Formeln (1), (2), (3), (4), (5), (6), (7), (8), (9), (10), (11), (12) und Mischungen davon;
- des Weiteren als roten organischen Fluoreszenzfarbstoff N,N'-Bis(2,6-diisopropylphenyl)-1,7-di(2,6-diisopropylphenoxy)perylen-3,4;9,10-tetracarbonsäurediimid, und/oder N,N'-Bis(2,6-diisopropylphenyl)-1,6-di(2,6-diisopropyl-phenoxy)perylen-3,4;9,10-tetracarbonsäurediimid;
- Streukörper auf der Basis von TiO₂; und
- mindestens ein Polymer, das im Wesentlichen aus Polycarbonat besteht.

Hierunter speziell bevorzugt sind Perylen-Verbindungen der Formeln (1), (2), (3), (4), (5), (6), (7), (8), (9), (10), (11), (12) und Mischungen davon, worin Z und Z* die gleiche Bedeutung aufweisen.

In einer weiteren ganz besonders bevorzugten Ausführungsform enthalten erfindungsgemäße Farbkonverter
- als organischen Fluoreszenzfarbstoff mindestens eine erfindungsgemäße Verbindung der Formel I, vorzugsweise ausgewählt unter Verbindungen der Formeln (13), (14), (15), (16), (17), (18), (19), (20) und Mischungen davon, oder eine Zusammensetzung, enthaltend mindestens eine cyanierte Perylen-Verbindung der Formel I, vorzugsweise ausgewählt unter Verbindungen der Formeln (13), (14), (15), (16), (17), (18), (19), (20) und Mischungen davon;
- des Weiteren als roten organischen Fluoreszenzfarbstoff N,N'-Bis(2,6-diisopropylphenyl)-1,7-di(2,6-diisopropylphenoxy)perylen-3,4;9,10-tetracarbonsäurediimid, und/oder N,N'-Bis(2,6-diisopropylphenyl)-1,6-di(2,6-diisopropyl-phenoxy)perylen-3,4;9,10-tetracarbonsäurediimid;
- Streukörper auf der Basis von TiO₂; und
- mindestens ein Polymer, das im Wesentlichen aus Polycarbonat besteht.

Hierunter speziell bevorzugt sind Perylen-Verbindungen der Formeln (13), (14), (15), (16), (17), (18), (19), (20) und Mischungen davon, worin Z und Z* die gleiche Bedeutung aufweisen.

Hat der Farbkonverter einen Mehrschichtaufbau, umfasst in einer Ausführungsform eine Schicht mindestens einen roten Fluoreszenzfarbstoff und eine andere Schicht mindestens einen erfindungsgemäßen Fluoreszenzfarbstoff der Formel I oder Mischungen davon.

In einer Ausführungsform liegt der mindestens eine rote organische Fluoreszenzfarbstoff in der der LED zugewandten Schicht des Farbkonverters vor. In einer anderen Ausführungsform liegt der mindestens eine grüne oder grüngelbe Fluoreszenzfarbstoff in der der LED zugewandten Schicht des Farbkonverters vor.

In einer weiteren Ausführungsform befindet sich in der der LED zugewandten Schicht ein Streukörper, darüber befinden sich ein Farbkonverter und darüber wiederum gegebenenfalls eine weitere Schicht mit einem Streukörper.

In einer bevorzugten Ausführungsform hat der Farbkonverter einen Zweischichtaufbau mit einer rot fluoreszierenden Schicht und einer grüngelb fluoreszierenden Schicht enthaltend mindestens einen erfindungsgemäß enthaltenen Fluoreszenzfarbstoff, wobei die rote Schicht der blauen Lichtquelle zugewandt ist. In dieser Ausführungsform enthalten beide Schichten TiO₂ als Streukörper.

Eine weitere bevorzugte Ausführungsform für Farbkonverter hat einen einschichtigen Aufbau, wobei mindestens ein erfindungsgemäß enthaltener Fluoreszenzfarbstoff der Formel I und Mischungen davon und mindestens ein roter Fluoreszenzfarbstoff gemäß Formel (XII) und Streukörper in einer Schicht umfasst sind. Der Streukörper ist vorzugsweise Titandioxid. In dieser Ausführungsform besteht das Polymer vorzugsweise aus Polystyrol, PET oder Polycarbonat.

In einer Ausführungsform ist mindestens eine Polymerschicht des Farbkonverters mit Glasfasern mechanisch verstärkt.

Erfindungsgemäße Farbkonverter können in beliebiger geometrischer Anordnung vorliegen. Die Farbkonverter können zum Beispiel in Form von Folien, Scheiben oder Plättchen vorliegen. Ebenso kann die organische Fluoreszenzfarbmittel enthaltende Matrix in Tropfen- oder Halbkugelform oder in Form von Linsen mit konvexen und/oder konkaven, ebenen oder sphärischen Oberflächen vorliegen.

Als Ausgießung bezeichnet man die Ausführungsform, wenn LEDs oder LEDs enthaltende Bauteile vollständig mit einer organischen Fluoreszenzfarbstoff enthaltendem Polymer ausgegossen oder umhüllt werden.

In einer Ausführungsform der Erfindung sind die organischen Fluoreszenzfarbstoff enthaltenden Polymerschichten (Matrices) 25 bis 200 Mikrometer dick, bevorzugt 35 bis 150 µm und besonders 50 bis 100 µm.

In einer anderen Ausführungsform sind die organischen Fluoreszenzfarbstoff enthaltenden Polymerschichten 0,2 bis 5 Millimeter dick, bevorzugt 0,3 bis 3 mm, besonders bevorzugt 0,4 bis 1 mm.

Bestehen die Farbkonverter aus einer Schicht oder haben sie einen Schichtaufbau, so sind die einzelnen Schichten in einer bevorzugten Ausführungsform durchgehend und weisen keine Löcher oder Unterbrechungen auf.

Die Konzentration der organischen Fluoreszenzfarbstoffe im Polymer wird in Abhängigkeit von der Dicke des Farbkonverters und der Art des Polymers eingestellt. Wird eine dünne Polymerschicht verwendet, so ist die Konzentration des organischen Fluoreszenzfarbstoffs in der Regel höher als bei einer dicken Polymerschicht.

In einer bevorzugten Ausführungsform enthält mindestens eine der Fluoreszenzfarbstoff enthaltenden Schichten bzw. Matrices Streukörper für Licht.

In einer weiteren bevorzugten Ausführungsform des Mehrschichtaufbaus liegen mehrere Fluoreszenzfarbstoff-enthaltende Schichten sowie eine oder mehrere Streumittel enthaltende Schichten ohne Fluoreszenzfarbstoff vor.

Geeignete Streukörper sind anorganische Weißpigmente wie zum Beispiel Titandioxid, Bariumsulfat, Litophone, Zinkoxid, Zinksulfid, Calciumcarbonat mit einer mittleren Teilchengröße nach DIN 13320 von 0,01 bis 10 µm, bevorzugt 0,1 bis 1 µm, besonders bevorzugt 0,15 bis 0,4 µm.

Streukörper sind typischerweise in einer Menge von 0,01 bis 4,0 Gew.-%, bevorzugt 0,05 bis 2 Gew.-%, besonders bevorzugt 0,1 bis 1 Gew.-%, jeweils bezogen auf das Polymer der Streukörper enthaltenden Schicht umfasst.

Erfindungsgemäße Farbkonverter können gegebenenfalls weitere Bestandteile umfassen wie eine Trägerschicht.

Trägerschichten dienen dazu, dem Farbkonverter mechanische Stabilität zu verleihen. Die Art des Materials der Trägerschichten ist nicht ausschlaggebend, solange dieses transparent ist und die gewünschte mechanische Festigkeit aufweist. Geeignete Materialien für Trägerschichten sind zum Beispiel Glas oder transparente, starre, organische Polymer wie Polycarbonat, Polystyrol oder Polymethacrylate oder Polymethylmethacrylate.

Trägerschichten haben in der Regel eine Dicke von 0,1 mm bis 10 mm, bevorzugt 0,3 mm bis 5 mm, besonders bevorzugt 0,5 mm bis 2 mm.

In einer Ausführungsform der Erfindung weisen erfindungsgemäße Farbkonverter mindestens eine Barriereschicht gegen Sauerstoff und/oder Wasser auf, wie dieses in WO 2012/152812 offenbart ist. Beispiele für geeignete Barrierematerialien für Barriereschichten sind zum Beispiel Glas, Quarz, Metalloxide, SiO₂, ein Multischichtsystem aus alternierenden Schichten von Al₂O₃- und SiO₂-Schichten, Titannitrid, SiO₂/Metalloxid Multischichtmaterialien, Polyvinylalkohol, Polyacrylnitril, Polyvinylidenchlorid (PVDC), Flüssigkristallpolymeren (LCP), Polystyrolacrylnitril (SAN), Polybutylenterephthalat (PBT), Polybutylennaphthalat (PBN), Polyethylenterephthalat (PET), Polyethylenenaphthalat (PEN), Polyvinylbutyrat (PBT), Polyvinylchlorid (PVC), Polyamide, Polyoxymethylene, Polyimide, Polyetherimide, Epoxidharze, Polymere, die sich von Ethylenvinylacetat (EVA) ableiten und Polymere, die sich von Ethylenvinylalkohol (EVOH) ableiten.

Ein bevorzugtes Material für Barriereschichten ist Glas oder ein Multischichtsystem aus alternierenden Schichten von Al₂O₃- und SiO₂-Schichten.

Bevorzugt haben geeignete Barriereschichten eine geringe Durchlässigkeit für Sauerstoff.

Besonders bevorzugt haben geeignete Barriereschichten eine geringe Durchlässigkeit für Sauerstoff und Wasser.

Erfindungsgemäße Farbkonverter eignen sich insbesondere für die Konversion von blauem Licht in grüngelbes Licht.

Insbesondere eignen sie sich zur Konversion von durch blaue LEDs emittiertem Licht. Geeignete LEDs sind zum Beispiel solche auf der Basis von Galliumnitrid (GaN) oder Indiumgalliumnitrid (InGaN). Ebenfalls möglich ist die Verwendung zur Konversion von durch Quecksilberlampen, durch organische Leuchtdioden (OLEDs) oder von UV-LEDs erzeugtem Licht.

Sie eignen sich weiterhin für Anwendungen als Lichtsammelsystem (Fluoreszenzkollektor) in der Photovoltaik sowie in Fluoreszenzkonversionssolarzellen.

In einer weiteren Ausführungsform werden die erfindungsgemäßen Farbkonverter für die Konversion von blauem Licht verwendet.

In einer weiteren Ausführungsform wird der Farbkonverter zur Konversion von Licht verwendet, das von einer blauen Diode erzeugt wurde, wobei anstelle von Ce:YAG als Strahlungskonversionsstoff mindestens eine Verbindung der Formel I oder Mischungen davon als Fluoreszenzfarbstoff verwendet wird oder eine Zusammensetzung, enthaltend mindestens eine Verbindung der Formel I oder Mischungen davon. Bevorzugt enthält der Farbkonverter als Fluoreszenzfarbstoff neben der erfindungsgemäßen Verbindung der Formel I oder Mischungen davon einen roten organischen Fluoreszenzfarbstoff. Der rote organische Fluoreszenzfarbstoff ist vorzugsweise ausgewählt unter den Verbindungen der Formeln X, XI und XII. In dieser Ausführungsform liegt die blaue LED und der Farbkonverter in einer Remote Phosphor Anordnung vor. Die Farbwiedergabe einer solchen LED genügt hohen Anforderungen.

In einer weiteren Ausführungsform wird der Farbkonverter zur Konversion von Licht verwendet, das von einer blauen Diode erzeugt wurde, wobei mindestens eine Verbindung der Formel I oder Mischungen davon oder eine Zusammensetzung, enthaltend mindestens eine Verbindung der Formel I oder Mischungen davon als Fluoreszenzfarbstoff in Kombination mit mindestens einem anorganischen Fluoreszenzfarbmittel ausgewählt unter mit Seltenen Erden dotierten Aluminaten, Silicaten, Nitriden und Granaten, insbesondere mit Cer dotiertes Yttrium-Aluminium-Granat, eingesetzt wird. In dieser Ausführungsform liegt die blaue LED und der Farbkonverter in einer remote phosphor Anordnung vor.

Erfindungsgemäße Farbkonverter zeigen bei Bestrahlung mit Licht, insbesondere mit blauem LED Licht, eine hohe Quantenausbeute. Weiterhin haben sie eine hohe Photostabilität bei Belichtung mit blauem Licht. Ferner sind sie stabil gegenüber Sauerstoff, und Wasser. Sie emittieren angenehmes Licht mit einer guten Farbwiedergabe. Ein weiterer Vorteil ist, dass Farbkonverter bereitgestellt werden können, die keine Seltenen Erden enthalten. Erfindungsgemäße Farbkonverter, die cyanierte Verbindungen der Formel I oder Mischungen davon oder eine Zusammensetzung, enthaltend mindestens eine Verbindung der Formel I oder Mischungen davon zusammen mit Seltenen Erden dotierten anorganischen Fluoreszenzmitteln enthalten, verbessern den Farbwiedergabewert einer mit einer blauen LED hergestellten Beleuchtungsvorrichtung, die Ce:YAG als Konvertermaterial enthalten.

Erfindungsgemäße Farbkonverter können nach unterschiedlichen Verfahren hergestellt werden.

In einer Ausführungsform umfasst das Verfahren zur Herstellung von erfindungsgemäßen Farbkonvertern das Lösen des mindestens einen Polymers und des mindestens einen organischen Fluoreszenzfarbstoffs in einem Lösungsmittel und anschließendes Entfernen des Lösungsmittels.

In einer anderen Ausführungsform umfasst das Verfahren zur Herstellung von erfindungsgemäßen Farbkonvertern das Extrudieren des mindestens einen organischen Fluoreszenzfarbstoffs mit dem mindestens einen Polymer.

Ein weiterer Gegenstand der Erfindung sind Beleuchtungsvorrichtungen umfassend mindestens eine LED und mindestens einen erfindungsgemäßen Farbkonverter. Die mindestens eine LED ist bevorzugt blau und emittiert Licht vorzugsweise in einem Wellenlängenbereich von 400 bis 500 nm, bevorzugt 420 bis 480 nm, besonders bevorzugt 440 bis 470 nm, ganz besonders bevorzugt bei 445 bis 460 nm.

In einer Ausführungsform enthalten erfindungsgemäße Beleuchtungsvorrichtungen genau eine LED. In einer anderen Ausführungsform enthalten erfindungsgemäße Beleuchtungsvorrichtungen mehrere LEDs.

In einer Ausführungsform enthalten erfindungsgemäße Beleuchtungsvorrichtungen mehrere LEDs, die alle blau sind. In einer anderen Ausführungsform enthalten erfindungsgemäße Beleuchtungsvorrichtungen mehrere LEDs, wobei mindestens eine LED blau ist und mindestens eine LED nicht blau ist, sondern Licht einer anderen Farbe, zum Beispiel rot, emittiert.

Darüber hinaus ist die Art der verwendeten LED für die erfindungsgemäßen Beleuchtungsvorrichtungen nicht entscheidend. In einer bevorzugten Ausführungsform beträgt die Leistungsdichte der verwendeten LED weniger als 20 mW/cm², bevorzugt weniger als 15 mW/cm². Die Verwendung von LEDs höherer Leistungsdichten wie 25 oder 30 mW/cm² ist ebenfalls möglich. Durch eine höhere Leistungsdichte der LED kann jedoch die Lebensdauer der Fluoreszenzfarbstoffe und der Farbkonverter vermindert werden.

Erfindungsgemäße Farbkonverter können in Kombination mit LEDs in nahezu jeder geometrischen Form und unabhängig vom Aufbau der Beleuchtungsvorrichtung verwendet werden.

In einer Ausführungsform liegen Farbkonverter und LED in einer phosphor on a chip Anordnung vor.

Bevorzugt werden erfindungsgemäße Farbkonverter in einem remote phosphor Aufbau verwendet. Hierbei ist der Farbkonverter von der LED räumlich getrennt. In der Regel beträgt der Abstand zwischen LED und Farbkonverter von 0,1 cm bis 50 cm, bevorzugt 0,2 bis 10 cm und ganz besonders bevorzugt 0,5 bis 2 cm. Zwischen Farbkonverter und LED können sich unterschiedliche Medien wie Luft, Edelgase, Stickstoff oder andere Gase oder Mischungen hiervon befinden.

Der Farbkonverter kann dabei zum Beispiel konzentrisch um die LED angeordnet sein oder eine planare Geometrie haben. Er kann zum Beispiel als Plättchen, Scheibe, Folie, in Tropfenform oder als Ausgießung vorliegen.

Erfindungsgemäße Beleuchtungsvorrichtungen sind geeignet zur Beleuchtung in Innenräumen, im Außenbereich, von Büroräumen, von Fahrzeugen, in Taschenlampen, Spielekonsolen, Straßenlaternen, Verkehrsleuchtzeichen.

Erfindungsgemäße Beleuchtungsvorrichtungen zeigen eine hohe Quantenausbeute. Weiterhin haben sie eine lange Lebensdauer, insbesondere eine hohe Photostabilität bei Belichtung mit blauem Licht. Sie emittieren angenehmes Licht mit einer guten Farbwiedergabe.

### Beispiele

Es wurden verschiedene Fluoreszenzfarbstoffe synthetisiert. Aus den gemäß den Beispielen hergestellten Fluoreszenzfarbstoffen wurden Farbkonverter hergestellt. Hierzu wurde diese wie im Folgenden beschrieben in eine Matrix aus einem Polymer eingearbeitet. Als Polymer wurde PMMA, (Plexiglas® 6N der Firma Evonik), Polystyrol (PS168 N von BASF) und PC (Macrolon® 2808 von Bayer) verwendet.

Herstellung der Farbkonverter zur Prüfung der Farbstoffe:
In ca. 5 mL Methylenchlorid wurden ca. 2,5 g Polymer sowie 0,02 Gew.-% Farbstoff gelöst und darin 0,5 Gew.-% TiO₂ dispergiert, jeweils bezogen auf die eingesetzte Menge Polymer. Die erhaltene Lösung/Dispersion wurde mit einem Kastenrakel (Nassfilmdicke 400 µm) auf eine Glasoberfläche gerakelt. Nach Abtrocknen des Lösemittels wurde der Film vom Glas abgelöst und über Nacht im Vakuumtrockenschrank bei 50 °C getrocknet. Aus diesem 80 bis 85 µm dicken Film wurden je zwei kreisrunde Folienstücke mit einem Durchmesser von 15 mm ausgestanzt, die dann als Messproben dienten.

Fluoreszenzquantenausbeuten (FQA) der Messproben wurden mit dem Quantenausbeute-Messsystem Modell C9920-02 (Fa. Hamamatsu) vermessen. Dabei wurden die Proben in einer Integrationskugel (Ulbricht-Kugel) jeweils mit Licht von 450 bis 455 nm beleuchtet. Durch Vergleich mit der Referenzmessung in der Ulbricht-Kugel ohne Probe werden der nicht-absorbierte Anteil des Anregungslichts und das von der Probe ausgesendete Fluoreszenzlicht mittels CCD-Spektrometer ermittelt. Durch Integration der Intensitäten über das Spektrum des nicht-absorbierten Anregungslichts bzw. über das des emittierten Fluoreszenzlichts erhält man jeweils den Absorptionsgrad bzw. die Fluoreszenzintensität bzw. die Fluoreszenzquantenausbeute der Probe.

### Beispiel 1

Mischung von Verbindungen der Formeln (1.a) und (1.b) 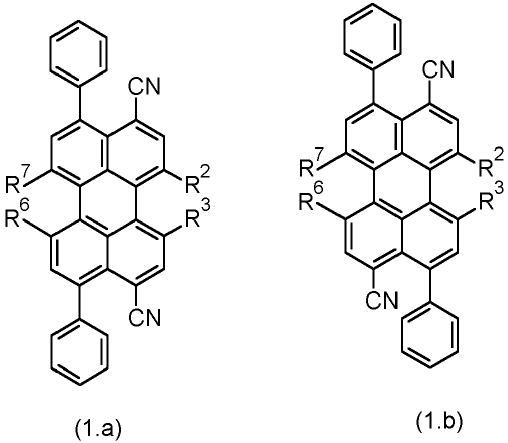 worin
zwei der Substituenten R², R³, R⁶ und R⁷ für Wasserstoff stehen; und
zwei der Substituenten R², R³, R⁶ und R⁷ für Cyano stehen.

### 1.1 3,9-Dibromperylen und 3,10-Dibromperylen

Eine Mischung aus 14,9 g (59 mmol) Perylen, 400 ml Essigsäure und 18,9 g (236 mmol) Brom wurde vier Stunden bei 40 °C gerührt. Das überschüssige Brom wurde anschließend durch Zugabe von Natriumthiosulfatlösung zerstört. Der Niederschlag wurde abfiltriert, mit Wasser gewaschen und im Vakuum getrocknet. Man erhielt 25,46 g (quant.) der Titelverbindungen als gelblichen Niederschlag.

### 1.2 3,9-Diphenylperylen und 3,10-Diphenylperylen

Eine Mischung aus 1,23 g (3 mmol) 3,9-Dibromperylen und 3,10-Dibromperylen aus Beispiel 1.1, 30 ml Toluol, 1,46 g (12 mmol) Phenylboronsäure, 2,49 g (18 mmol) Kaliumcarbonat, 8 ml Wasser, 0,24 g (0,2 mmol) Tetrakistriphenylphosphinpalladium wurde 60 Stunden auf 90 °C erhitzt. Nach Abkühlen des Reaktionsgemisches wurde mit Toluol verdünnt, die Phasen getrennt und die Toluolphase über eine Säulenfiltration mit Kieselgel gereinigt. Man erhielt 1,1 g (92 %) der Titelverbindung als gelben Feststoff. Rf (Petrolether : Toluol 5 : 1) = 0,31.

### 1.3 Mischung von Verbindungen der Formeln (1.3a) und (1.3b)

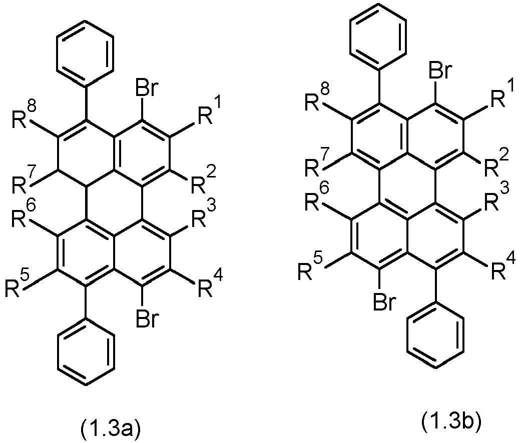

worin
wenigstens zwei der Substituenten R¹, R², R³, R⁴, R⁵, R⁶, R⁷ und R⁸ für Brom stehen;
und die anderen Substituenten R¹, R², R³, R⁴, R⁵, R⁶, R⁷ und R⁸ für Wasserstoff stehen.

Zu einer Mischung aus 202 mg (0,5 mmol) 3,9-Diphenylperylen und 3,10-Diphenylperylen aus Beispiel 1.2 und 25 ml Chlorbenzol gab man 5 ml Wasser, 10 ml Chlorbenzol und 800 mg (10 mmol) Brom und erhitzte 22 Stunden bei Rückfluss. Das Reaktionsgemisch wurde abgekühlt. Danach gab man bei Raumtemperatur 200 ml verdünnte Natriumthiosulfat Lösung zu, versetzte mit Essigester, trennte die Phasen und engte ein, wobei man die Titelverbindungen mit einem Rf (Petrolether: Toluol 5 : 1): 0,61 erhielt.

### 1.4 Mischung von Verbindungen der Formeln (1.a) und (1.b)

280 mg (0,5 mmol) des in 1.3 erhalten Gemischs, 896 mg (10 mmol) Cu-cyanid und 30 ml NMP (N-Methylpyrrolidon) wurden 6 h bei 100 °C und weitere 16 h bei 150 °C gerührt. Nach Abkühlen auf Raumtemperatur wurde das Gemisch auf verdünnte HCl gefällt, abgesaugt, mit Wasser gewaschen und bei 60 °C im Vakuum getrocknet. Der Rückstand wurde an Kieselgel chromatographiert (Eluent: Toluol: Essigester 100 : 1). Man erhielt 17 mg der Titelverbindungen.
Absorption: λ ₘₐₓ (CH₂Cl₂): 497 nm;
Emission: λ ₘₐₓ (CH₂Cl₂): 563 nm
FQA (Polystyrol): 92 %
Halbwertzeit T80 (80 mW/cm²): 59 Tage in Polystyrol

### Beispiel 2

Mischung von Verbindungen der Formeln (2.a) und (2.b), 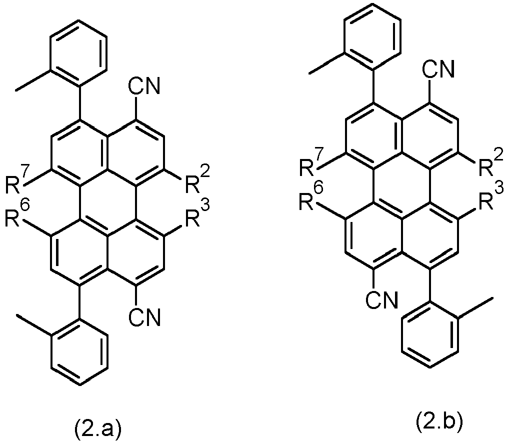 worin
zwei der Substituenten R², R³, R⁶ und R⁷ für Wasserstoff stehen; und
zwei der Substituenten R², R³, R⁶ und R⁷ für Cyano stehen.

### 2.1 3,9-Bis(o-tolyl)perylen und 3,10-Bis(o-tolyl)perylen

Eine Mischung aus 4,10 g (10 mmol) 3,9-Dibromperylen und 3,10-Dibromperylen aus Beispiel 1.1, 100 ml Toluol, 5,44 g (40 mmol) 2-Methylphenylboronsäure, 8,3 g (60 mmol) Kaliumcarbonat, 15 ml Wasser, 2,32 g (2 mmol) Tetrakistriphenylphosphinpalladium wurde 34 Stunden auf 90 °C erhitzt. Nach Abkühlen des Reaktionsgemisches wurde mit Toluol verdünnt, die Phasen getrennt und die Toluolphase über eine Säulenfiltration mit Kieselgel gereinigt. Man erhielt 5,54 g der Titelverbindung als gelben Feststoff, Rf (Petrolether : Toluol 5 : 1) = 0,33, und ein Nebenprodukt mit Rf (Petrolether : Toluol 5 : 1) = 0,09.

### 2.2 Mischung von Verbindungen der Formeln (2.2a) und (2.2b)

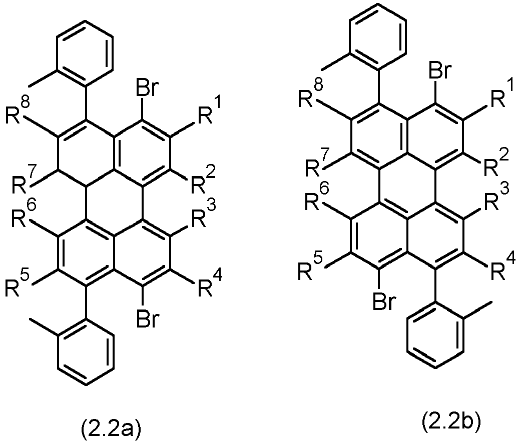

worin
wenigstens zwei der Substituenten R¹, R², R³, R⁴, R⁵, R⁶, R⁷ und R⁸ für Brom stehen;
und die anderen Substituenten R¹, R², R³, R⁴, R⁵, R⁶, R⁷ und R⁸ für Wasserstoff stehen.

Zu der in Beispiel 2.1 isolierten Mischung aus 3,9-Bis(o-tolyl)perylen und 3,10-Bis(o-tolyl)perylen (1,30 g, 3 mmol) in 100 ml Chlorbenzol gab man bei 40 °C 10 ml Wasser, 10 ml Chlorbenzol und 4,8 g (60 mmol) Brom. Man rührte das Gemisch 16 Stunden bei 70 °C und 7 Stunden bei 80 °C. Man ließ auf Raumtemperatur abkühlen, verdünnte mit 200 ml Toluol, gab verdünnte Natriumthiosulfat Lösung zu, trennte die Phasen und engte ein, wobei man einen Rückstand erhielt. Es entstanden als Hauptprodukte tribromiertes und tetrabromiertes 3,9-Bis(o-tolyl)perylen und tribromiertes und tetrabromiertes 3,10-Bis(o-tolyl)perylen und als Nebenprodukte dibromiertes und pentabromiertes 3,9-Bis(o-tolyl)perylen und 3,10-Bis(o-tolyl)perylen. Rf (Petrolether: Toluol 5 : 1) 0,49, 0,61, 0,38.

### 2.3 Mischung von Verbindungen der Formeln (2.a) und (2.b)

1,12 g des in Beispiel 2.2 erhaltenen Rückstands, 2,69 mg (30 mmol) Cu-cyanid und 50 ml N-Methylpyrrolidon wurden 3 Stunden bei 100 °C und danach 16 Stunden bei 150 °C gerührt. Man ließ auf Raumtemperatur abkühlen und gab verdünnte HCl zu. Man saugte den ausgefallenen Niederschlag ab, wusch mit Wasser und trocknete bei 60 °C im Vakuum. Man reinigte die rohe Titelverbindung an Kieselgel (Laufmittel Petrolether: THF 10 : 1).
Rf (Petrolether : THF, 5 : 1) = 0,19.
Absorption: λ ₘₐₓ (CH₂Cl₂): 489 nm;
Emission: λ ₘₐₓ (CH₂Cl₂): 547 nm
FQA (Polystyrol): 90-91 %;
FQA (Polycarbonat): 90-91 %;
Halbwertzeit T80 (80 mW/cm²): 50 Tage in Polystyrol;
Halbwertzeit T80 (80 mW/cm²): 61 Tage in Polycarbonat.

### Beispiel 3

Mischung von Verbindungen der Formeln (3.a) und (3.b) 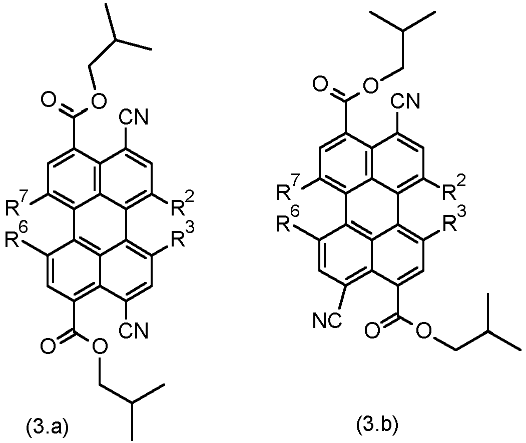 worin
drei der Substituenten R², R³, R⁶ und R⁷ für Wasserstoff stehen; und
einer der Substituenten R², R³, R⁶ und R⁷ für Cyano steht.

### 3.1 Mischung von Verbindungen der Formeln (3.1a) und (3.1b)

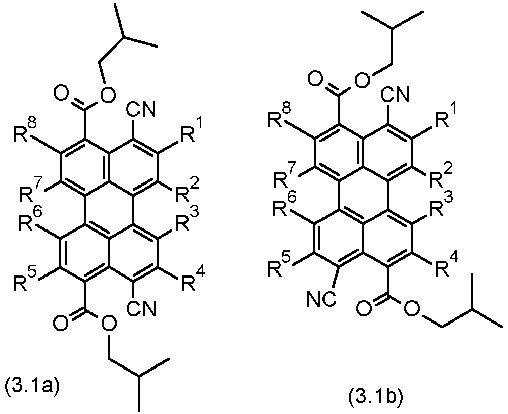

worin
wenigstens einer der Substituenten R¹, R², R³, R⁴, R⁵, R⁶, R⁷ und R⁸ für Brom steht;
und die anderen Substituenten R¹, R², R³, R⁴, R⁵, R⁶, R⁷ und R⁸ für Wasserstoff stehen.

Eine Mischung aus 4,52 g (10 mmol) Diisobutylperylen-3,9-dicarboxylat und Diisobutylperylen-3,10-dicarboxylat, 150 ml Chlorbenzol, 100 ml Wasser, 16 g (200 mmol) Brom und etwas lod wurden 3 h bei leichten Rückfluss (ca. 87 °C) gerührt. Danach wurde das Reaktionsgemisch abgekühlt, auf verdünnte HCl gegossen und die Phasen getrennt. Die organische Phase wurde eingeengt. Der Rückstand hatte einen Rf (Toluol : Essigester 10 : 1) = 0,73.

### 3.2 Herstellung einer Mischung von Verbindungen der Formeln (3.a) und (3.b)

Eine Mischung von 6 g (7,8 mmol) des in Beispiel 3.1 erhaltenen Rückstandes, 6,9 g (6,9 mmol) Cu(I)-cyanid und 150 ml NMP wurden 4 Std. bei 170 °C gerührt. Das Reaktionsgemisch wurde auf Raumtemperatur abgekühlt, Ammoniakwasser zugegeben und gegen Methylenchlorid extrahiert. Der Rückstand der vereinigten organischen Phasen wurde durch eine Säulenfiltration über Kieselgel (Laufmittel: Toluol) gereinigt.
Absorption: λ ₘₐₓ (CH₂Cl₂): 481 nm;
Emission: λ ₘₐₓ (CH₂Cl₂): 511 nm
Halbwertzeit T80 (80 mW/cm²): 13 Tage in Polystyrol;
Halbwertzeit T80 (80 mW/cm²): 40 Tage in Polycarbonat.
FQA (Polystyrol): 93 %;
FQA (Polycarbonat): 93 %.

## Patentansprüche

1. Cyanierte Perylen-Verbindung der Formel I 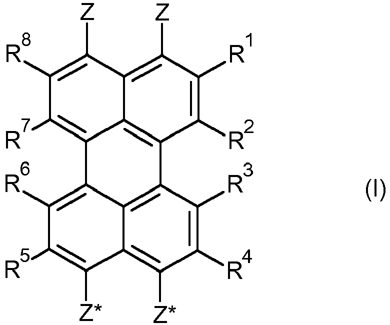 worin
einer der Substituenten Z für Cyano steht und der andere Substituent Z für CO₂R⁹, CONR¹⁰R¹¹, C₁-C₁₈-Alkyl, C₂-C₁₈-Alkenyl, C₂-C₁₈-Alkinyl, C₃-C₁₂-Cycloalkyl oder C₆-C₁₄-Aryl steht, wobei
C₁-C₁₈-Alkyl, C₂-C₁₈-Alkenyl, C₂-C₁₈-Alkinyl unsubstituiert sind oder eine oder mehrere, gleiche oder verschiedene Substituenten Z^{a} tragen, C₃-C₁₂-Cycloalkyl unsubstituiert ist oder eine oder mehrere gleiche oder verschiedene Substituenten Z^{b} trägt, und
C₆-C₁₄-Aryl unsubstituiert ist oder ein- oder mehrere gleiche oder verschiedene Substituenten Z^{Ar} trägt;
einer der Substituenten Z* für Cyano steht und der andere Substituent Z* für CO₂R⁹, CONR¹⁰R¹¹, C₁-C₁₈-Alkyl, C₂-C₁₈-Alkenyl, C₂-C₁₈-Alkinyl, C₃-C₁₂-Cycloalkyl oder C₆-C₁₄-Aryl steht, wobei
C₁-C₁₈-Alkyl, C₂-C₁₈-Alkenyl, C₂-C₁₈-Alkinyl unsubstituiert sind oder eine oder mehrere, gleiche oder verschiedene Substituenten Z^{a} tragen, C₃-C₁₂-Cycloalkyl unsubstituiert ist oder eine oder mehrere gleiche oder verschiedene Substituenten Z^{b} trägt, und
C₆-C₁₄-Aryl unsubstituiert ist oder ein- oder mehrere gleiche oder verschiedene Substituenten Z^{Ar} trägt;
R¹, R², R³, R⁴, R⁵, R⁶, R⁷ und R⁸ unabhängig voneinander ausgewählt sind unter Wasserstoff, Cyano, Brom und Chlor,
unter der Maßgabe, dass 1, 2, 3, 4, 5, 6, 7 oder 8 der Substituenten R¹, R², R³, R⁴, R⁵, R⁶, R⁷ oder R⁸ für Cyano steht;
wobei
R⁹ für Wasserstoff, C₁-C₁₀-Alkyl, C₂-C₁₀-Alkenyl, C₂-C₁₀-Alkinyl, C₃-C₁₂-Cycloalkyl oder C₆-C₁₄-Aryl steht, wobei
C₁-C₁₀-Alkyl, C₂-C₁₀-Alkenyl, C₂-C₁₀-Alkinyl unsubstituiert sind oder eine oder mehrere gleiche oder verschiedene Substituenten R^{a} tragen,
C₃-C₁₂-Cycloalkyl unsubstituiert ist oder eine oder mehrere gleiche oder verschiedene Substituenten R^{b} trägt und
C₆-C₁₄-Aryl unsubstituiert ist oder ein- oder mehrere gleiche oder verschiedene Substituenten R^{Ar} trägt;
R¹⁰ und R¹¹, unabhängig voneinander, für Wasserstoff, C₁-C₁₀-Alkyl, C₂-C₁₀-Alkenyl, C₂-C₁₀-Alkinyl, C₃-C₁₂-Cycloalkyl oder C₆-C₁₄-Aryl stehen, wobei
C₁-C₁₀-Alkyl, C₂-C₁₀-Alkenyl, C₂-C₁₀-Alkinyl unsubstituiert sind oder eine oder mehrere gleiche oder verschiedene Substituenten R^{a} tragen,
C₃-C₁₂-Cycloalkyl unsubstituiert ist oder eine oder mehrere gleiche oder verschiedene Substituenten R^{b} trägt und
C₆-C₁₄-Aryl unsubstituiert ist oder ein- oder mehrere gleiche oder verschiedene Substituenten R^{Ar} trägt;
jedes Z^{a} unabhängig voneinander für Halogen, Hydroxy, NR^{10a}R^{11a}, C₁-C₁₀-Alkoxy, C₁-C₁₀-Haloalkoxy, C₁-C₁₀-Alkylthio, C₃-C₁₂-Cycloalkyl, C₆-C₁₄-Aryl, C(=O)R^{9a}, C(=O)OR^{9a} oder C(O)NR^{10a}R^{11a} steht, wobei
C₃-C₁₂-Cycloalkyl unsubstituiert ist oder eine oder mehrere gleiche oder verschiedene Substituenten R^{b} trägt und
C₆-C₁₄-Aryl unsubstituiert ist oder ein- oder mehrere gleiche oder verschiedene Substituenten R^{Ar} trägt;
jedes Z^{b} und jedes Z^{Ar} unabhängig voneinander für Halogen, Hydroxy, NR^{10a}R^{11a}, C₁-C₁₀-Alkyl, C₁-C₁₀-Alkoxy, C₁-C₁₀-Haloalkoxy, C₁-C₁₀-Alkylthio, C(=O)R^{9a}, C(=O)OR^{9a} oder C(O)NR^{10a}R^{11a} steht;
jedes R^{a} unabhängig voneinander für Halogen, Hydroxy, C₁-C₁₀-Alkoxy C₃-C₁₂-Cycloalkyl oder C₆-C₁₄-Aryl steht;
jedes R^{b} unabhängig voneinander für Halogen, Hydroxy, C₁-C₁₀-Alkyl, C₁-C₁₀-Alkoxy, C₁-C₁₀-Haloalkoxy, C₁-C₁₀-Alkylthio, C₂-C₁₀-Alkenyl, C₂-C₁₀-Alkinyl, C₃-C₁₂-Cycloalkyl oder C₆-C₁₄-Aryl steht;
jedes R^{Ar} unabhängig voneinander für Halogen, Hydroxy, C₁-C₁₀-Alkyl, C₁-C₁₀-Alkoxy, C₁-C₁₀-Haloalkoxy, C₁-C₁₀-Alkylthio, C₂-C₁₀-Alkenyl, C₂-C₁₀-Alkinyl, C₃-C₁₂-Cycloalkyl oder C₆-C₁₄-Aryl steht;
R^{9a} für Wasserstoff, C₁-C₁₀-Alkyl, C₂-C₁₀-Alkenyl, C₂-C₁₀-Alkinyl, C₃-C₁₂-Cycloalkyl oder C₆-C₁₄-Aryl steht; und
R^{10a}, R^{11a} unabhängig voneinander für Wasserstoff, C₁-C₁₀-Alkyl, C₂-C₁₀-Alkenyl, C₂-C₁₀-Alkinyl, C₃-C₁₂-Cycloalkyl oder C₆-C₁₄-Aryl stehen,
und Mischungen davon.

2. Cyanierte Perylen-Verbindung der Formel I nach Anspruch 1 und Mischungen davon, worin ein oder zwei der Substituenten R¹, R², R³, R⁴, R⁵, R⁶, R⁷ oder R⁸ für Cyano stehen und die anderen Substituenten R¹, R², R³, R⁴, R⁵, R⁶, R⁷ oder R⁸ für Wasserstoff stehen.

3. Cyanierte Perylen-Verbindung der Formel I nach Anspruch 1 oder 2 und Mischungen davon, worin einer der Substituenten Z und einer der Substituenten Z* unabhängig voneinander ausgewählt sind unter C₁-C₁₀-Alkyl, CO₂R⁹, Phenyl-C₁-C₁₀-alkyl und Phenyl, wobei Phenyl und der Phenylteil von Phenyl-C₁-C₁₀-alkyl unsubstituiert sind oder einen oder mehrere unter C₁-C₆-Alkyl ausgewählte Substituenten tragen, und wobei R⁹ wie zuvor definiert ist.

4. Cyanierte Perylen-Verbindung der Formel I nach einem der vorhergehenden Ansprüche ausgewählt unter Verbindungen der Formeln (1), (2), (3), (4), (5), (6), (7), (8), (9), (10), (11), (12), (13), (14), (15), (16), (17), (18), (19), (20) 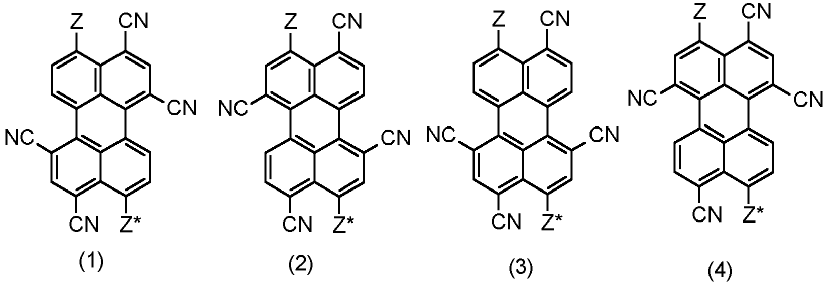 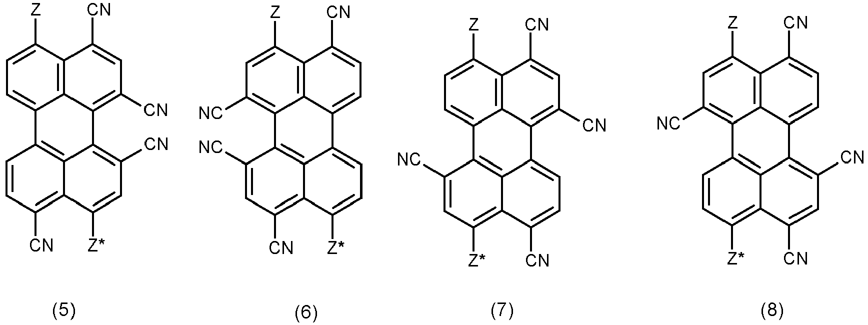 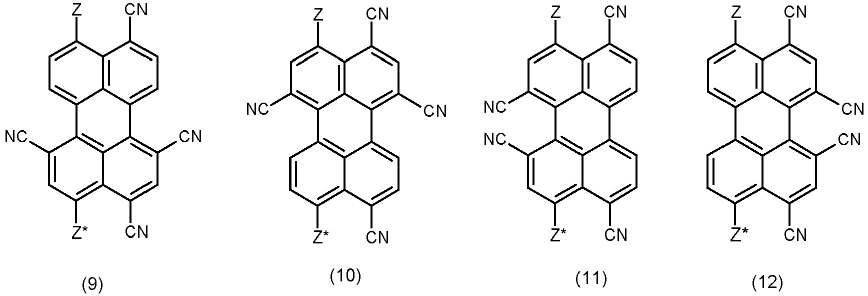 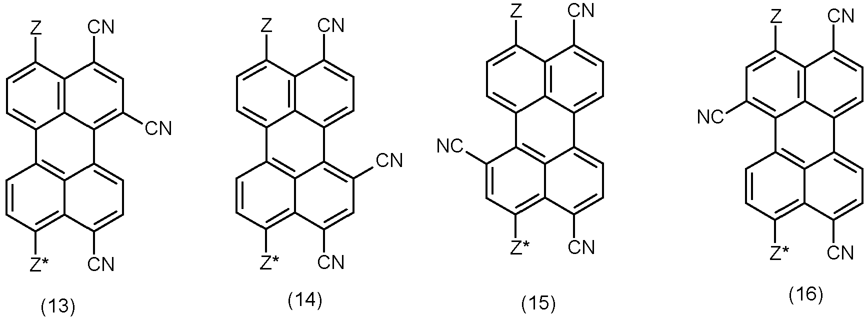 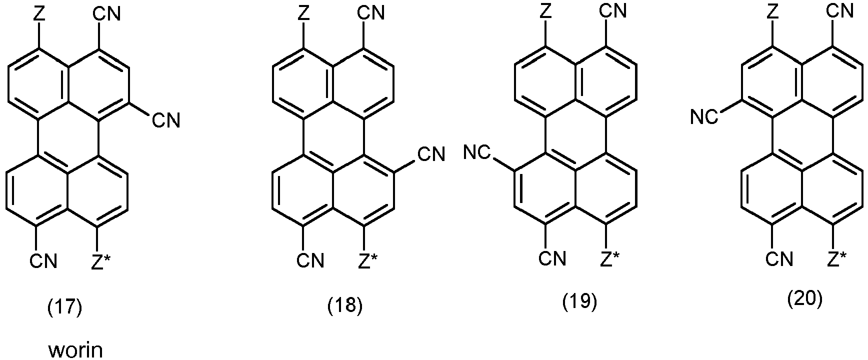
Z ausgewählt ist unter C₁-C₆-Alkyl, C₁-C₆-Alkoxycarbonyl, Phenyl und Phenyl, das 1, 2 oder 3 C₁-C₄-Alkylgruppen trägt; und
Z* ausgewählt ist unter C₁-C₆-Alkyl, C₁-C₆-Alkoxycarbonyl, Phenyl und Phenyl, das 1, 2 oder 3 C₁-C₄-Alkylgruppen trägt;
und Mischungen davon.

5. Zusammensetzung, enthaltend mindestens eine cyanierte Perylen-Verbindung der Formel I nach einem der Ansprüche 1 bis 4 oder Mischungen davon.

6. Zusammensetzung nach Anspruch 5, enthaltend mindestens eine cyanierte Perylen-Verbindung der Formel I-A, 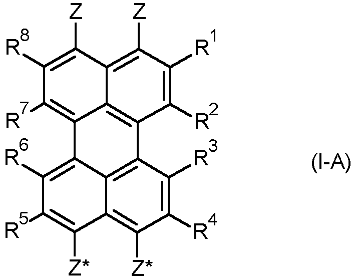 worin
einer der Substituenten Z für Cyano steht und der andere Substituent Z für C₁-C₁₈-Alkyl, C₂-C₁₈-Alkenyl, C₂-C₁₈-Alkinyl, C₃-C₁₂-Cycloalkyl oder C₆-C₁₄-Aryl steht, wobei
C₁-C₁₈-Alkyl, C₂-C₁₈-Alkenyl, C₂-C₁₈-Alkinyl unsubstituiert sind oder eine oder mehrere, gleiche oder verschiedene Substituenten Z^{a} tragen, wobei Z^{a} die zuvor genannten Bedeutungen aufweist,
C₃-C₁₂-Cycloalkyl unsubstituiert ist oder eine oder mehrere gleiche oder verschiedene Substituenten Z^{b} trägt, wobei Z^{b} die zuvor genannten Bedeutungen aufweist; und
C₆-C₁₄-Aryl unsubstituiert ist oder ein- oder mehrere gleiche oder verschiedene Substituenten Z^{Ar} trägt, wobei Z^{Ar} die zuvor genannten Bedeutungen aufweist;
einer der Substituenten Z* für Cyano steht und der andere Substituent Z* für C₁-C₁₈-Alkyl, C₂-C₁₈-Alkenyl, C₂-C₁₈-Alkinyl, C₃-C₁₂-Cycloalkyl oder C₆-C₁₄-Aryl steht, wobei
C₁-C₁₈-Alkyl, C₂-C₁₈-Alkenyl, C₂-C₁₈-Alkinyl unsubstituiert sind oder eine oder mehrere, gleiche oder verschiedene Substituenten Z^{a} tragen, wobei Z^{a} die zuvor genannten Bedeutungen aufweist,
C₃-C₁₂-Cycloalkyl unsubstituiert ist oder eine oder mehrere gleiche oder verschiedene Substituenten Z^{b} trägt, wobei Z^{b} die zuvor genannten Bedeutungen aufweist; und
C₆-C₁₄-Aryl unsubstituiert ist oder ein- oder mehrere gleiche oder verschiedene Substituenten Z^{Ar} trägt, wobei Z^{Ar} die zuvor genannten Bedeutungen aufweist;
R¹, R⁴, R⁵ und R⁸ jeweils für Wasserstoff stehen;
zwei der Substituenten R², R³, R⁶ oder R⁷ für Wasserstoff stehen; und die anderen Substituenten R², R³, R⁶ oder R⁷ für Cyano stehen;
oder Mischungen davon,
erhältlich nach einem Verfahren, bei dem man
a) Perylen der Formel II 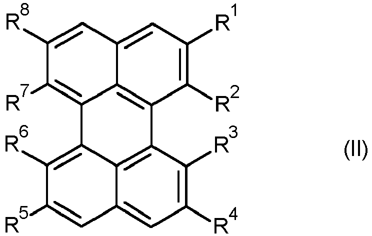 worin
R¹, R², R³, R⁴, R⁵, R⁶, R⁷ und R⁸ jeweils für Wasserstoff stehen;
halogeniert unter Erhalt einer Mischung aus 3,9-Dihalogenperylen der Formel IIIa und 3,10-Dihalogenperylen der Formel IIIb 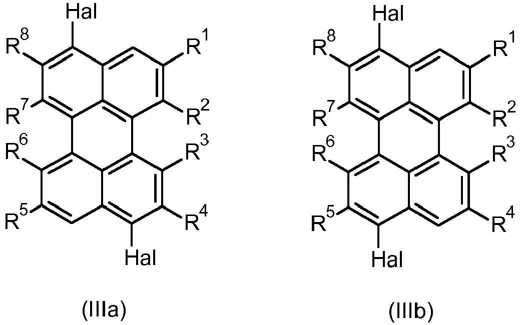 worin
R¹, R², R³, R⁴, R⁵, R⁶, R⁷ und R⁸ jeweils für Wasserstoff stehen; und
Hal jeweils alle für Chlor oder Brom stehen;
b) die in Schritt a) erhaltene Mischung aus Verbindungen der Formeln lila und IIIb mit einer metallorganischen Verbindung der Formel IV
Z-Met (IV)
und gegebenenfalls mit einer metallorganischen Verbindung der Formel V
Z*-Met (V)
worin
Z ausgewählt ist unter C₁-C₁₈-Alkyl, C₂-C₁₈-Alkenyl, C₂-C₁₈-Alkinyl, C₃-C₁₂-Cycloalkyl und C₆-C₁₄-Aryl, wobei C₁-C₁₈-Alkyl, C₂-C₁₈-Alkenyl, C₂-C₁₈-Alkinyl unsubstituiert sind oder eine oder mehrere, gleiche oder verschiedene Substituenten Z^{a} tragen,
C₃-C₁₂-Cycloalkyl unsubstituiert ist oder eine oder mehrere gleiche oder verschiedene Substituenten Z^{b} trägt, und
C₆-C₁₄-Aryl unsubstituiert ist oder ein- oder mehrere gleiche oder verschiedene Substituenten Z^{Ar} trägt;
Z* ausgewählt ist unter C₁-C₁₈-Alkyl, C₂-C₁₈-Alkenyl, C₂-C₁₈-Alkinyl, C₃-C₁₂-Cycloalkyl und C₆-C₁₄-Aryl, wobei C₁-C₁₈-Alkyl, C₂-C₁₈-Alkenyl, C₂-C₁₈-Alkinyl unsubstituiert sind oder eine oder mehrere, gleiche oder verschiedene Substituenten Z^{a} tragen,
C₃-C₁₂-Cycloalkyl unsubstituiert ist oder eine oder mehrere gleiche oder verschiedene Substituenten Z^{b} trägt, und
C₆-C₁₄-Aryl unsubstituiert ist oder ein- oder mehrere gleiche oder verschiedene Substituenten Z^{Ar} trägt;
wobei Z* auch die gleiche Bedeutung wie Z besitzen kann;
Met für B(OH)₂, B(OR')(OR"), Zn-Hal oder Sn(R*)₃ steht, worin
R' und R" unabhängig voneinander für Wasserstoff, C₁-C₃₀-Alkyl, C₅-C₈-Cycloalkyl, C₆-C₁₄-Aryl oder Heteroaryl stehen oder R' und R" zusammen für C₂-C₄-Alkylen stehen, das
gegebenenfalls 1, 2, 3, 4, 5, 6, 7 oder 8 Substituenten trägt, die ausgewählt sind unter C₁-C₄-Alkyl, C₅-C₈-Cycloalkyl, C₆-C₁₄-Aryl und Heteroaryl;
Hal für Chlor oder Brom steht; und
R* für C₁-C₈-Alkyl oder Phenyl steht;
umsetzt unter Erhalt einer Mischung aus Verbindungen der Formeln VIa und Vlb, 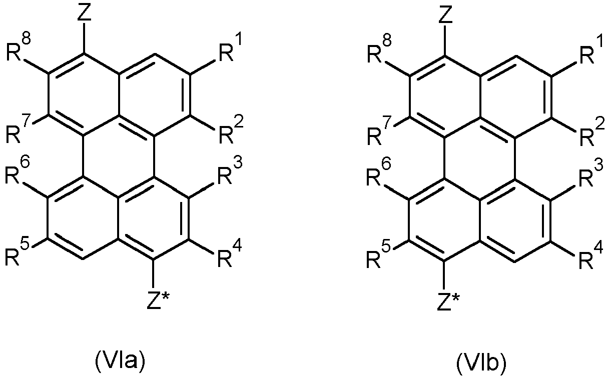 worin
R¹, R², R³, R⁴, R⁵, R⁶, R⁷ und R⁸ jeweils für Wasserstoff stehen; und
Z und Z* wie zuvor definiert sind;
c) die in Schritt b) erhaltene Mischung aus Verbindungen der Formeln VIa und Vlb halogeniert unter Erhalt eines Reaktionsgemischs, das Verbindungen der Formeln Vlla und VIIb 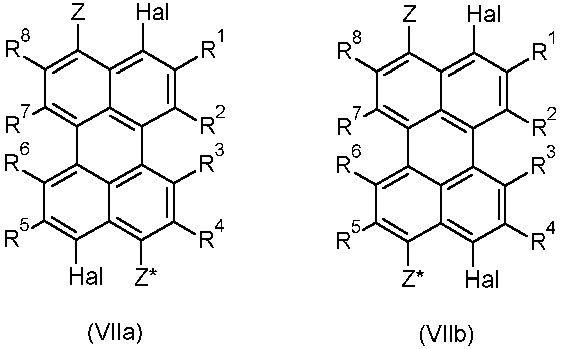 worin
Z und Z* wie zuvor definiert sind,
Hal für unter Chlor und Brom ausgewähltes Halogen steht, wobei die Substituenten Hal entweder alle für Chlor oder alle für Brom stehen;
R¹, R², R³, R⁴, R⁵, R⁶, R⁷ und R⁸ für Wasserstoff oder unter Chlor und Brom ausgewähltes Halogen stehen, wobei die Substituenten R¹, R², R³, R⁴, R⁵, R⁶, R⁷ und R⁸, die nicht für Wasserstoff stehen, entweder alle für Chlor oder alle für Brom stehen;
enthält;
d) die in dem in Schritt c) erhaltenen Reaktionsgemisch enthaltenen Verbindungen der Formel VIIa und VIIb einer Substitution von Halogen durch Cyano, sowie gegebenenfalls teilweise durch Wasserstoff, unterzieht unter Erhalt wenigstens einer Verbindung der Formel I-A oder Mischungen davon; und
e) gegebenenfalls die in dem in Schritt d) erhaltenen Reaktionsgemisch enthaltene wenigstens eine Verbindung der Formel I-A oder Mischungen davon mindestens einem Auftrennungs- und/oder Reinigungsschritt unterzieht.

7. Zusammensetzung nach Anspruch 5 enthaltend mindestens eine cyanierte Perylen-Verbindung der Formel I-B, 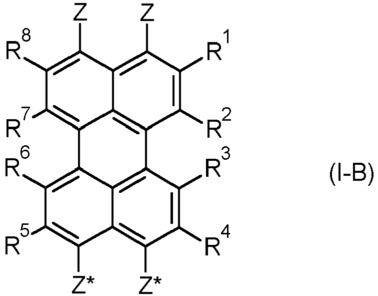 worin
einer der Substituenten Z für Cyano steht und der andere Substituent Z für COOR⁹ steht;
einer der Substituenten Z* für Cyano steht und der andere Substituent Z* für COOR⁹ steht;
R¹, R⁴, R⁵ und R⁸ jeweils für Wasserstoff stehen;
einer der Substituenten R², R³, R⁶ oder R⁷ für Cyano steht und die anderen Substituenten R², R³, R⁶ oder R⁷ für Wasserstoff stehen; und
R⁹ für Wasserstoff, C₁-C₁₀-Alkyl, C₂-C₁₀-Alkenyl, C₂-C₁₀-Alkinyl, C₃-C₁₂-Cycloalkyl oder C₆-C₁₄-Aryl steht, wobei
C₁-C₁₀-Alkyl, C₂-C₁₀-Alkenyl, C₂-C₁₀-Alkinyl, unsubstituiert sind oder eine oder mehrere gleiche oder verschiedene Substituenten R^{a} tragen, C₃-C₁₂-Cycloalkyl unsubstituiert ist oder eine oder mehrere gleiche oder verschiedene Substituenten R^{b} trägt, und
C₆-C₁₄-Aryl unsubstituiert ist oder ein- oder mehrere gleiche oder verschiedene Substituenten R^{Ar} trägt,
wobei R^{a}, R^{b} und R^{Ar} wie zuvor definiert sind;
oder Mischungen davon,
erhältlich nach einem Verfahren, bei dem man
f) eine Mischung aus Perylen-Verbindungen der Formeln VIIIa und VIIIb 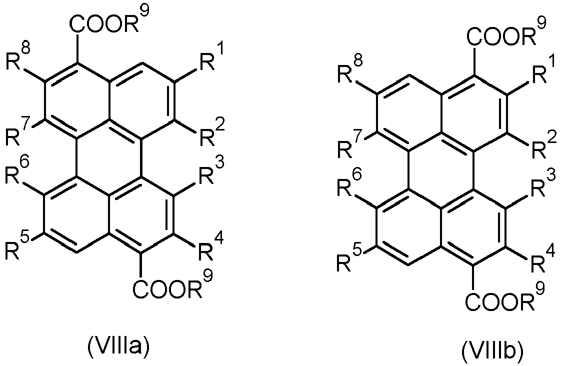 worin
R¹, R², R³, R⁴, R⁵, R⁶, R⁷ und R⁸ jeweils für Wasserstoff stehen; und
R⁹ wie zuvor definiert ist
halogeniert unter Erhalt eines Reaktionsgemischs, das Verbindungen der Formeln IXa und IXb 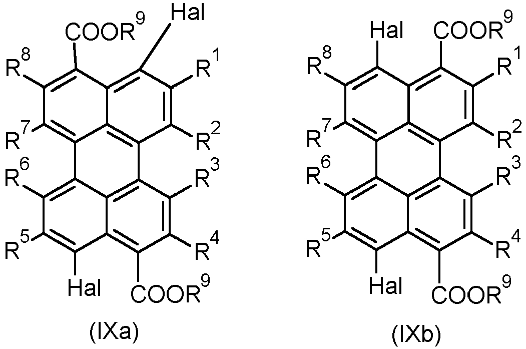 worin
R¹, R², R³, R⁴, R⁵, R⁶, R⁷ und R⁸ für Wasserstoff oder unter Chlor und Brom ausgewähltes Halogen stehen, wobei die Substituenten R¹, R², R³, R⁴, R⁵, R⁶, R⁷ und R⁸, die nicht für Wasserstoff stehen, entweder alle für Chlor oder alle für Brom stehen;
Hal für unter Chlor und Brom ausgewähltes Halogen steht, wobei die Substituenten Hal entweder alle für Chlor oder alle für Brom stehen; und
R⁹ wie zuvor definiert ist;
enthält;
g) die in dem in Schritt f) erhaltenen Reaktionsgemisch enthaltenen Verbindungen der Formeln IXa und IXb einer Substitution von Halogen durch Cyanogruppen, sowie gegebenenfalls teilweise durch Wasserstoff unterzieht unter Erhalt wenigstens einer Verbindung der Formel I-B oder Mischungen davon; und
h) gegebenenfalls die in dem in Schritt g) erhaltenen Reaktionsgemisch enthaltene wenigstens eine Verbindung der Formel I-B oder Mischungen davon mindestens einem Auftrennungs- und/oder Reinigungsschritt unterzieht.

8. Verwendung der cyanierten Perylen-Verbindungen der Formel I nach einem der Ansprüche 1 bis 4 und Mischungen davon oder eine Zusammensetzung nach einem der Ansprüche 5 bis 7 in Farbkonvertern, für optische Label, zur unsichtbaren Markierung von Produkten, als Fluoreszenzfarbstoffe, vorzugsweise als Fluoreszenzlabel für Biomoleküle, als Pigmente, als Fluoreszenzfarbstoff in einem auf Fluoreszenzkonversion beruhenden Display; in einem lichtsammelnden Kunststoffteil, welches gegebenenfalls mit einer Solarzelle kombiniert ist; als Pigmentfarbstoff in elektrophoretischen Displays; als Fluoreszenzfarbstoff in einer auf Chemolumineszenz basierenden Anwendung.

9. Farbkonverter, enthaltend (i) als Matrix mindestens ein Polymer, im Wesentlichen bestehend aus Polystyrol, Polycarbonat, Polymethylmethacrylat, Polyvinylpyrrolidon, Polymethacrylat, Polyvinylacetat, Polyvinylchlorid, Polybuten, Polyethylenglykol, Silikon, Polyacrylat, Epoxidharz, Polyvinylalkohol, EthylenVinylalkohol-Copolymer (EVOH), Polyacrylnitril, Polyvinylidenchlorid (PVDC), Polystyrolacrylnitril (SAN), Polybutylenterephthalat (PBT), Polyethylenterephthalat (PET), Polyvinylbutyrat (PVB), Polyvinylchlorid (PVC), Polyamide, Polyoxymethylene, Polyimide, Polyetherimid oder Mischungen hiervon,und (ii) als Fluoreszenzfarbstoff mindestens eine cyanierte Perylen-Verbindung der Formel I nach einem der Ansprüche 1 bis 4 oder Mischungen davon oder eine Zusammensetzung nach einem der Ansprüche 5 bis 7.

10. Farbkonverter nach Anspruch 9, wobei die Komponente (ii) mindestens eine cyanierte Perylen-Verbindung ausgewählt unter Verbindungen der Formeln (1) bis (20) 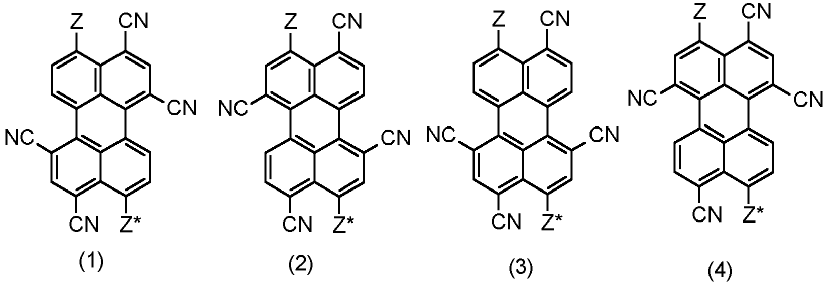 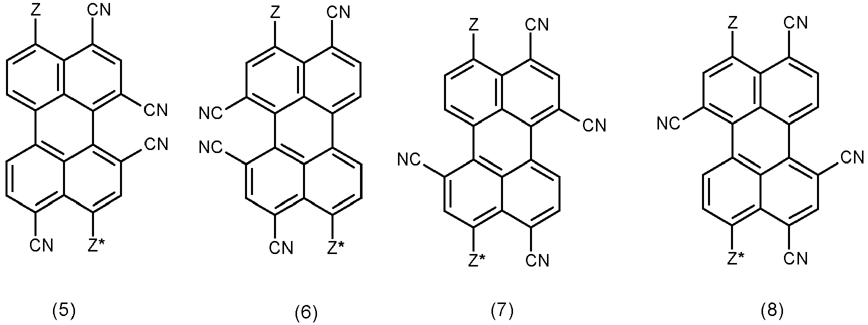 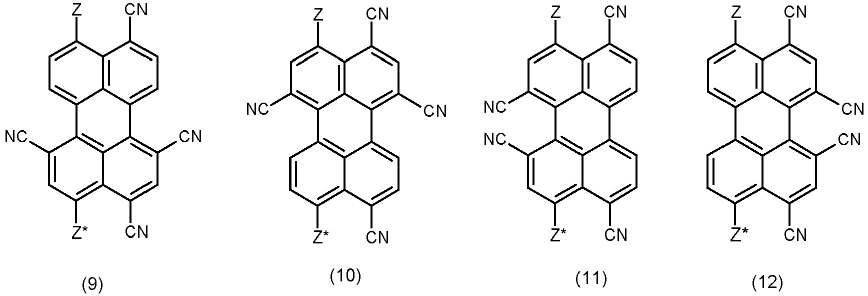 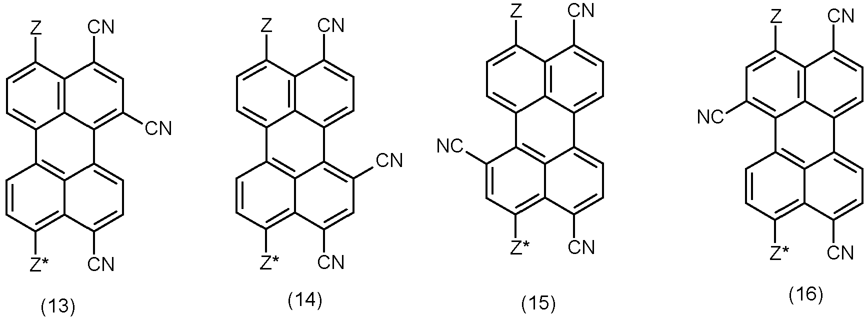 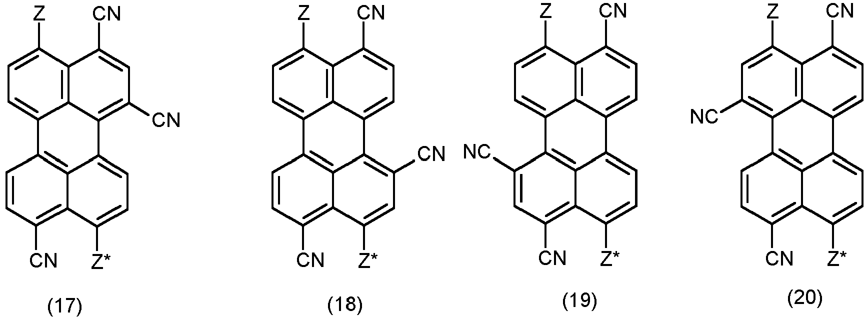 worin
Z ausgewählt ist unter C₁-C₆-Alkyl, C₁-C₆-Alkoxycarbonyl, Phenyl und Phenyl, das 1, 2 oder 3 C₁-C₄-Alkylgruppen trägt; und
Z* ausgewählt ist unter C₁-C₆-Alkyl, C₁-C₆-Alkoxycarbonyl, Phenyl und Phenyl, das 1, 2 oder 3 C₁-C₄-Alkylgruppen trägt;
und Mischungen davon, oder eine Zusammensetzung, enthaltend mindestens eine cyanierte Perylen-Verbindung der Formel I ausgewählt unter den Verbindungen (1) bis (20) und Mischungen davon, enthält.

11. Farbkonverter nach Anspruch 9 oder 10, enthaltend mindestens einen weiteren organischen Fluoreszenzfarbstoff ausgewählt unter Verbindungen oder Mischungen der Formeln X, XI und XII 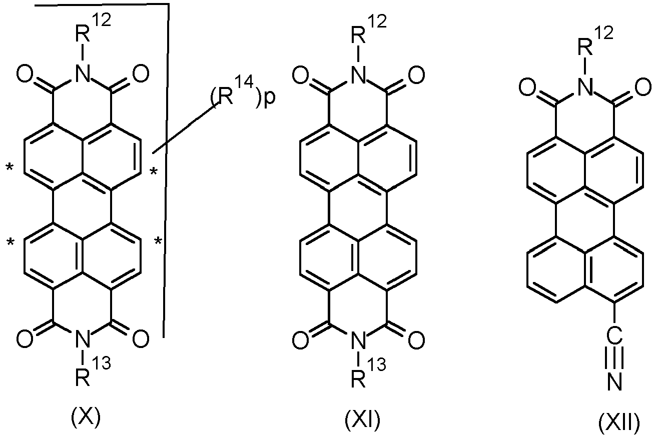 worin
p für 1 bis 4 steht,
R¹², R¹³ unabhängig voneinander für C₁-C₃₀-Alkyl, C₃-C₈-Cycloalkyl, C₆-C₁₄-Aryl, Hetaryl, C₆-C₁₄-Aryl-C₁-C₁₀-alkylen stehen, wobei in den drei letztgenannten Resten der aromatische Ring unsubstituiert ist oder ein- oder mehrfach durch C₁-C₁₀-Alkyl substituiert ist,
R¹⁴ für C₁-C₃₀-Alkoxy oder C₆-C₁₄-Aryloxy, das unsubstituiert ist oder ein- oder mehrfach durch C₁-C₁₀-Alkyl substituiert ist, steht,
wobei die Reste R¹⁴ sich an einer oder mehrerer der mit * gekennzeichneten Positionen befinden.

12. Farbkonverter nach Anspruch 11, wobei der weitere organische Fluoreszenzfarbstoff ausgewählt ist unter N,N'-Bis(2,6-diisopropylphenyl)-1,6,7,12-tetraphenoxyperylen-3,4;9,10-tetracarbonsäurediimid, N,N'-Bis(2,6-diisopropylphenyl)-1,7-di(2,6-diisopropylphenoxy)perylen-3,4;9,10-tetracarbonsäurediimid, N,N'-Bis(2,6-diisopropylphenyl)-1,6-di(2,6-diisopropylphenoxy)perylen-3,4;9,10-tetracarbonsäurediimid, N,N'-(Bis(2,6-diisopropylphenyl)-1,7-di(p-tert-octylphenoxy)-perylen-3,4;9,10-tetracarbonsäurediimid, N,N'-(Bis(2,6-diisopropylphenyl)-1,6-di(p-tert-octylphenoxy)-perylen-3,4;9,10-tetracarbonsäurediimid, N,N' Bis-(2,6-diisopropylphenyl)-1,7-diphenoxy-perylen-3,4;9,10-tetracarbonsäurediimid, N,N' Bis-(2,6-diisopropylphenyl)-1,6-diphenoxy-perylen-3,4;9,10-tetracarbonsäurediimid und Mischungen davon.

13. Farbkonverter nach einem der Ansprüche 9 bis 12, enthaltend als weiteres Fluoreszenzfarbmittel mindestens ein anorganisches Fluoreszenzfarbmittel ausgewählt unter den mit Seltenen Erden dotierten Aluminaten, Silicaten, Nitriden und Granaten, insbesondere mit Cer dotierter Yttrium-Aluminium-Granat.

14. Verwendung von Farbkonvertern wie in einem der Ansprüche 9 bis 13 definiert zur Konversion von durch LEDs erzeugtem Licht.

15. Beleuchtungsvorrichtung, umfassend mindestens eine LED und mindestens einen Farbkonverter gemäß einem der Ansprüche 9 bis 13.

## Claims

1. A cyanated perylene compound of the formula I 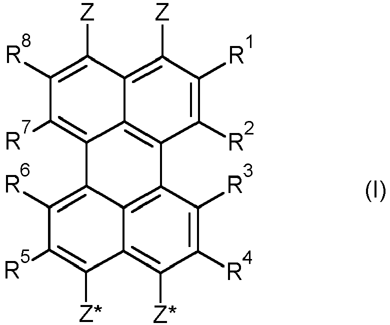 in which
one of the Z substituents is cyano and the other Z substituent is CO₂R⁹, CONR¹⁰R¹¹, C₁-C₁₈-alkyl, C₂-C₁₈-alkenyl, C₂-C₁₈-alkynyl, C₃-C₁₂-cycloalkyl or C₆-C₁₄-aryl, where
C₁-C₁₈-alkyl, C₂-C₁₈-alkenyl, C₂-C₁₈-alkynyl are unsubstituted or bear one or more identical or different Z^{a} substituents,
C₃-C₁₂-cycloalkyl is unsubstituted or bears one or more identical or different Z^{b} substituents, and
C₆-C₁₄-aryl is unsubstituted or bears one or more identical or different Z^{Ar} substituents;
one of the Z* substituents is cyano and the other Z* substituent is CO₂R⁹, CONR¹⁰R¹¹, C₁-C₁₈-alkyl, C₂-C₁₈-alkenyl, C₂-C₁₈-alkynyl, C₃-C₁₂-cycloalkyl or C₆-C₁₄-aryl, where
C₁-C₁₈-alkyl, C₂-C₁₈-alkenyl, C₂-C₁₈-alkynyl are unsubstituted or bear one or more identical or different Z^{a} substituents,
C₃-C₁₂-cycloalkyl is unsubstituted or bears one or more identical or different Z^{b} substituents, and
C₆-C₁₄-aryl is unsubstituted or bears one or more identical or different Z^{Ar} substituents;
R¹, R², R³, R⁴, R⁵, R⁶, R⁷ and R⁸ are each independently selected from hydrogen, cyano, bromine and chlorine,
with the proviso that 1, 2, 3, 4, 5, 6, 7 or 8 of the R¹, R², R³, R⁴, R⁵, R⁶, R⁷ or R⁸ substituents are cyano;
where
R⁹ is hydrogen, C₁-C₁₀-alkyl, C₂-C₁₀-alkenyl, C₂-C₁₀-alkynyl, C₃-C₁₂-cycloalkyl or C₆-C₁₄ -aryl, where
C₁-C₁₀-alkyl, C₂-C₁₀-alkenyl, C₂-C₁₀-alkynyl are unsubstituted or bear one or more identical or different R^{a} substituents, C₃-C₁₂-cycloalkyl is unsubstituted or bears one or more identical or different R^{b} substituents and
C₆-C₁₄-aryl is unsubstituted or bears one or more identical or different R^{Ar} substituents;
R¹⁰ and R¹¹ are each independently hydrogen, C₁-C₁₀-alkyl, C₂-C₁₀-alkenyl, C₂-C₁₀-alkynyl, C₃-C₁₂-cycloalkyl or C₆-C₁₄-aryl, where C₁-C₁₀-alkyl, C₂-C₁₀-alkenyl, C₂-C₁₀-alkynyl are unsubstituted or bear one or more identical or different R^{a} substituents, C₃-C₁₂-cycloalkyl is unsubstituted or bears one or more identical or different R^{b} substituents and
C₆-C₁₄-aryl is unsubstituted or bears one or more identical or different R^{Ar} substituents;
each Z^{a} is independently halogen, hydroxyl, NR^{10a}R^{11a}, C₁-C₁₀-alkoxy, C₁-C₁₀-haloalkoxy, C₁-C₁₀-alkylthio, C₃-C₁₂-cycloalkyl, C₆-C₁₄-aryl, C(=O)R^{9a}, C(=O)OR^{9a} or C(O)NR^{10a}R^{11a}, where C₃-C₁₂-cycloalkyl is unsubstituted or bears one or more identical or different R^{b} substituents and
C₆-C₁₄-aryl is unsubstituted or bears one or more identical or different R^{Ar} substituents;
each Z^{b} and each Z^{Ar} is independently halogen, hydroxyl, NR^{10a}R^{11a}, C₁-C₁₀-alkyl, C₁-C₁₀-alkoxy, C₁-C₁₀-haloalkoxy, C₁-C₁₀-alkylthio, C(=O)R^{9a}, C(=O)OR^{9a} or C(O)NR^{10a}R^{11a};
each R^{a} is independently halogen, hydroxyl, C₁-C₁₀-alkoxy, C₃-C₁₂-cycloalkyl or C₆-C₁₄-aryl;
each R^{b} is independently halogen, hydroxyl, C₁-C₁₀-alkyl, C₁-C₁₀-alkoxy, C₁-C₁₀-haloalkoxy, C₁-C₁₀-alkylthio, C₂-C₁₀-alkenyl, C₂-C₁₀-alkynyl, C₃-C₁₂-cycloalkyl or C₆-C₁₄-aryl;
each R^{Ar} is independently halogen, hydroxyl, C₁-C₁₀-alkyl, C₁-C₁₀-alkoxy, C₁-C₁₀-haloalkoxy, C₁-C₁₀-alkylthio, C₂-C₁₀-alkenyl, C₂-C₁₀-alkynyl, C₃-C₁₂-cycloalkyl or C₆-C₁₄-aryl;
R^{9a} is hydrogen, C₁-C₁₀-alkyl, C₂-C₁₀-alkenyl, C₂-C₁₀-alkynyl, C₃-C₁₂-cycloalkyl or C₆-C₁₄-aryl; and
R^{10a}, R^{11a} are each independently hydrogen, C₁-C₁₀-alkyl, C₂-C₁₀-alkenyl, C₂-C₁₀-alkynyl, C₃-C₁₂-cycloalkyl or C₆-C₁₄-aryl,
and mixtures thereof.

2. A cyanated perylene compound of the formula I according to claim 1 and mixtures thereof, in which one or two of the R¹, R², R³, R⁴, R⁵, R⁶, R⁷ or R⁸ substituents are cyano and the other R¹, R², R³, R⁴, R⁵, R⁶, R⁷ or R⁸ substituents are hydrogen.

3. A cyanated perylene compound of the formula I according to claim 1 or 2 and mixtures thereof, in which one of the Z substituents and one of the Z* substituents are independently selected from C₁-C₁₀-alkyl, CO₂R⁹, phenyl-C₁-C₁₀-alkyl and phenyl, where phenyl and the phenyl moiety of phenyl-C₁-C₁₀-alkyl are unsubstituted or bear one or more substituents selected from C₁-C₆-alkyl, and where R⁹ is as defined above.

4. A cyanated perylene compound of the formula I according to any of the preceding claims, selected from compounds of the formulae (1), (2), (3), (4), (5), (6), (7), (8), (9), (10), (11), (12), (13), (14), (15), (16), (17), (18), (19), (20) 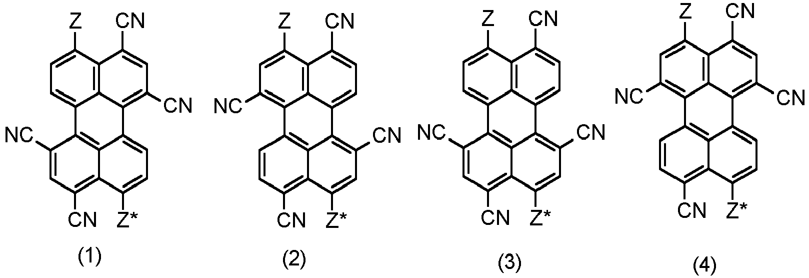 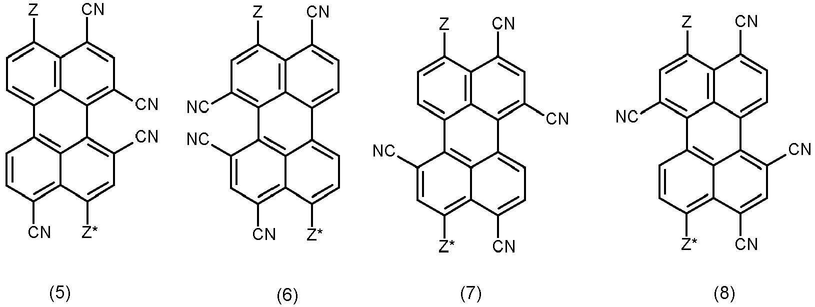 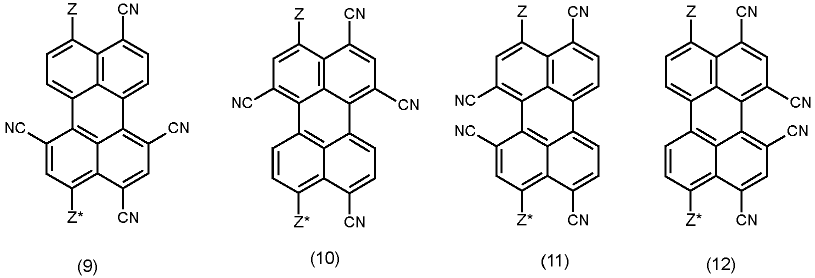 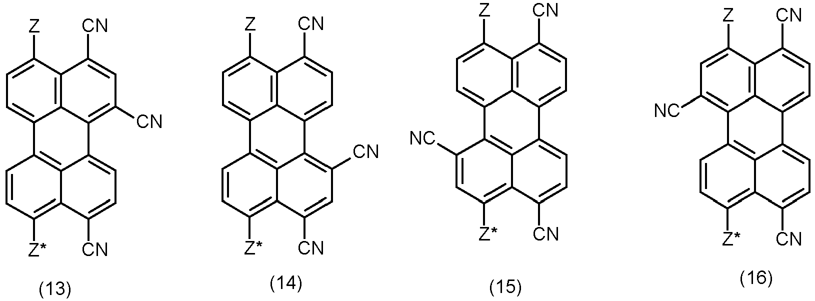 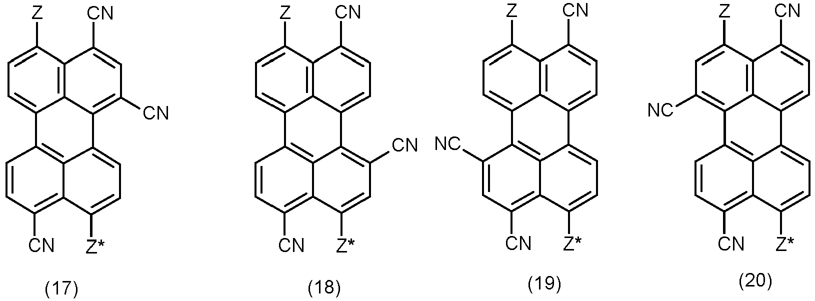 in which
Z is selected from C₁-C₆-alkyl, C₁-C₆-alkoxycarbonyl, phenyl, and phenyl bearing 1, 2 or 3 C₁-C₄-alkyl groups; and
Z* is selected from C₁-C₆-alkyl, C₁-C₆-alkoxycarbonyl, phenyl, and phenyl bearing 1, 2 or 3 C₁-C₄-alkyl groups;
and mixtures thereof.

5. A composition comprising at least one cyanated perylene compound of the formula I according to any of claims 1 to 4 or mixtures thereof.

6. The composition according to claim 5, comprising at least one cyanated perylene compound of the formula I-A 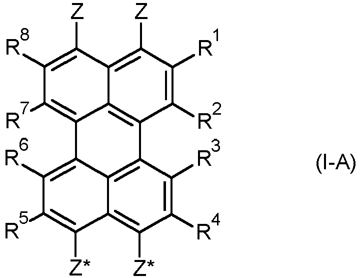 in which
one of the Z substituents is cyano and the other Z substituent is C₁-C₁₈-alkyl, C₂-C₁₈-alkenyl, C₂-C₁₈-alkynyl, C₃-C₁₂-cycloalkyl or C₆-C₁₄-aryl, where
C₁-C₁₈-alkyl, C₂-C₁₈-alkenyl, C₂-C₁₈-alkynyl are unsubstituted or bear one or more identical or different Z^{a} substituents, where Z^{a} is as defined above,
C₃-C₁₂-cycloalkyl is unsubstituted or bears one or more identical or different Z^{b} substituents, where Z^{b} is as defined above; and
C₆-C₁₄-aryl is unsubstituted or bears one or more identical or different Z^{Ar} substituents, where Z^{Ar} is as defined above;
one of the Z* substituents is cyano and the other Z* substituent is C₁-C₁₈-alkyl, C₂-C₁₈-alkenyl, C₂-C₁₈-alkynyl, C₃-C₁₂-cycloalkyl or C₆-C₁₄-aryl, where
C₁-C₁₈-alkyl, C₂-C₁₈-alkenyl, C₂-C₁₈-alkynyl are unsubstituted or bear one or more identical or different Z^{a} substituents, where Z^{a} is as defined above,
C₃-C₁₂-cycloalkyl is unsubstituted or bears one or more identical or different Z^{b} substituents, where Z^{b} is as defined above; and
C₆-C₁₄-aryl is unsubstituted or bears one or more identical or different Z^{Ar} substituents, where Z^{Ar} is as defined above;
R¹, R⁴, R⁵ and R⁸ are each hydrogen;
two of the R², R³, R⁶ or R⁷ substituents are hydrogen; and the other R², R³, R⁶ or R⁷ substituents are cyano;
or mixtures thereof,
obtainable by a process in which
a) perylene of the formula II 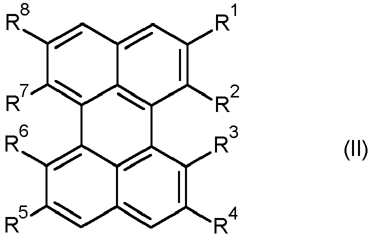 in which
R¹, R², R³, R⁴, R⁵, R⁶, R⁷ and R⁸ are each hydrogen;
is halogenated to obtain a mixture of 3,9-dihaloperylene of the formula IIIa and 3,10-dihaloperylene of the formula IIIb 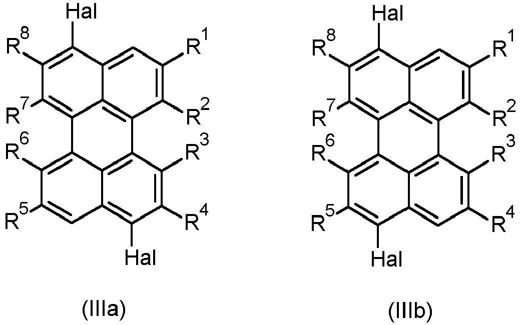 in which
R¹, R², R³, R⁴, R⁵, R⁶, R⁷ and R⁸ are each hydrogen; and
Hal are each all chlorine or bromine;
b) the mixture of compounds of the formulae IIIa and IIIb obtained in step a) is reacted with an organometallic compound of the formula IV
Z-Met (IV)
and optionally with an organometallic compound of the formula V
Z*-Met (V)
in which
Z is selected from C₁-C₁₈-alkyl, C₂-C₁₈-alkenyl, C₂-C₁₈-alkynyl, C₃-C₁₂-cycloalkyl and C₆-C₁₄-aryl, where C₁-C₁₈-alkyl, C₂-C₁₈-alkenyl, C₂-C₁₈-alkynyl are unsubstituted or bear one or more identical or different Z^{a} substituents,
C₃-C₁₂-cycloalkyl is unsubstituted or bears one or more identical or different Z^{b} substituents, and
C₆-C₁₄-aryl is unsubstituted or bears one or more identical or different Z^{Ar} substituents;
Z* is selected from C₁-C₁₈-alkyl, C₂-C₁₈-alkenyl, C₂-C₁₈-alkynyl, C₃-C₁₂-cycloalkyl and C₆-C₁₄-aryl, where C₁-C₁₈-alkyl, C₂-C₁₈-alkenyl, C₂-C₁₈-alkynyl are unsubstituted or bear one or more identical or different Z^{a} substituents,
C₃-C₁₂-cycloalkyl is unsubstituted or bears one or more identical or different Z^{b} substituents, and
C₆-C₁₄-aryl is unsubstituted or bears one or more identical or different Z^{Ar} substituents;
where Z* may also be as defined for Z;
Met is B(OH)₂, B(OR')(OR"), Zn-Hal or Sn(R*)₃, in which
R' and R" are each independently hydrogen, C₁-C₃₀-alkyl, C₅-C₈-cycloalkyl, C₆-C₁₄-aryl or heteroaryl or R' and R" together are C₂-C₄-alkylene which optionally bears 1, 2, 3, 4, 5, 6, 7 or 8 substituents selected from C₁-C₄-alkyl, C₅-C₈-cycloalkyl, C₆-C₁₄-aryl and heteroaryl;
Hal is chlorine or bromine; and
R* is C₁-C₈-alkyl or phenyl;
to obtain a mixture of compounds of the formulae VIa and VIb 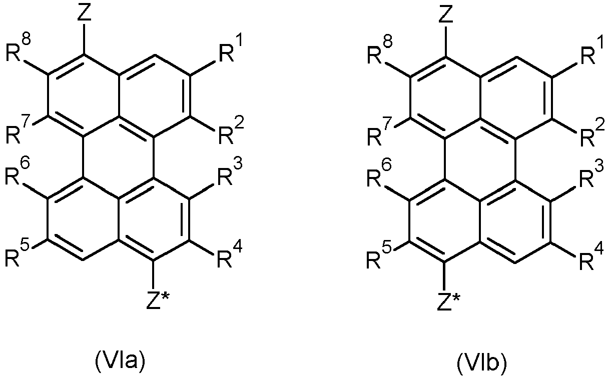 in which
R¹, R², R³, R⁴, R⁵, R⁶, R⁷ and R⁸ are each hydrogen; and
Z and Z* are each as defined above;
c) the mixture of compounds of the formulae VIa and VIb obtained in step b) is halogenated to obtain a reaction mixture comprising compounds of the formulae VIIa and VIIb 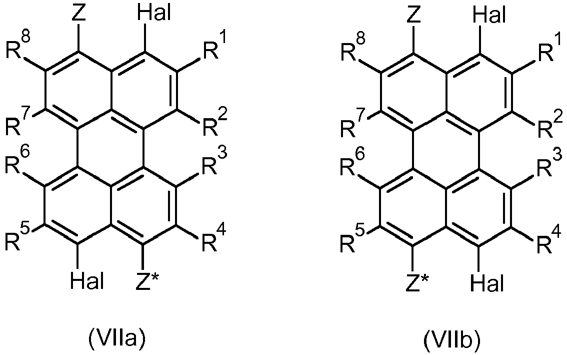 in which
Z and Z* are each as defined above,
Hal is halogen selected from chlorine and bromine, where the Hal substituents are either all chlorine or all bromine;
R¹, R², R³, R⁴, R⁵, R⁶, R⁷ and R⁸ are each hydrogen or halogen selected from chlorine and bromine, where the R¹, R², R³, R⁴, R⁵, R⁶, R⁷ and R⁸ substituents that are not hydrogen are either all chlorine or all bromine;
d) the compounds of the formulae VIIa and VIIb present in the reaction mixture obtained in step c) are subjected to a substitution of halogen for cyano, and optionally partly for hydrogen, to obtain at least one compound of the formula I-A or mixtures thereof; and
e) the at least one compound of the formula I-A or mixtures thereof present in the reaction mixture obtained in step d) is optionally subjected to at least one separation and/or purification step.

7. The composition according to claim 5, comprising at least one cyanated perylene compound of the formula I-B 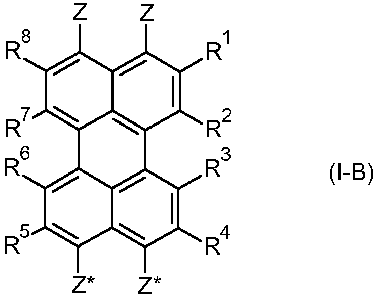 in which
one of the Z substituents is cyano and the other Z substituent is COOR⁹;
one of the Z* substituents is cyano and the other Z* substituent is COOR⁹;
R¹, R⁴, R⁵ and R⁸ are each hydrogen;
one of the R², R³, R⁶ or R⁷ substituents is cyano and the other R², R³, R⁶ or R⁷ substituents are hydrogen; and
R⁹ is hydrogen, C₁-C₁₀-alkyl, C₂-C₁₀-alkenyl, C₂-C₁₀-alkynyl, C₃-C₁₂-cycloalkyl or C₆-C₁₄-aryl, where C₁-C₁₀-alkyl, C₂-C₁₀-alkenyl, C₂-C₁₀-alkynyl are unsubstituted or bear one or more identical or different R^{a} substituents,
C₃-C₁₂-cycloalkyl is unsubstituted or bears one or more identical or different R^{b} substituents and
C₆-C₁₄-aryl is unsubstituted or bears one or more identical or different R^{Ar} substituents, where R^{a}, R^{b} and R^{Ar} are each as defined above;
or mixtures thereof,
obtainable by a process in which
f) a mixture of perylene compounds of the formulae VIIIa and VIIIb 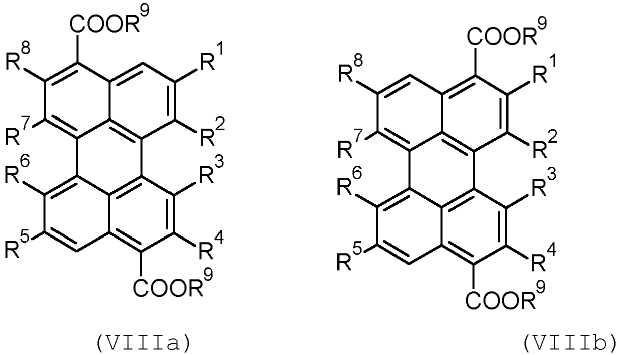 in which
R¹, R², R³, R⁴, R⁵, R⁶, R⁷ and R⁸ are each hydrogen; and
R⁹ is as defined above
is halogenated to obtain a reaction mixture comprising compounds of the formulae IXa and IXb 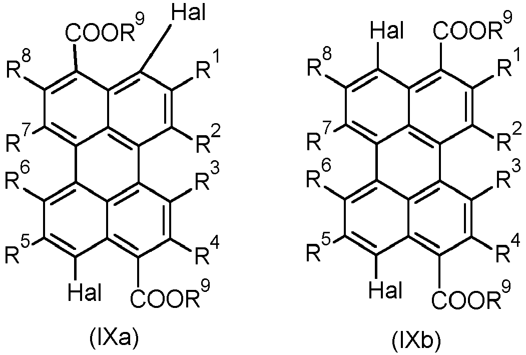 in which
R¹, R², R³, R⁴, R⁵, R⁶, R⁷ and R⁸ are each hydrogen or halogen selected from chlorine and bromine, where the R¹, R², R³, R⁴, R⁵, R⁶, R⁷ and R⁸ substituents that are not hydrogen are either all chlorine or all bromine;
Hal is halogen selected from chlorine and bromine, where the Hal substituents are either all chlorine or all bromine; and
R⁹ is as defined above;
g) the compounds of the formulae IXa and IXb present in the reaction mixture obtained in step f) are subjected to a substitution of halogen for cyano groups, and optionally partly for hydrogen, to obtain at least one compound of the formula I-B or mixtures thereof; and
h) the at least one compound of the formula I-B or mixtures thereof present in the reaction mixture obtained in step g) is optionally subjected to at least one separation and/or purification step.

8. The use of the cyanated perylene compounds of the formula I according to any of claims 1 to 4 and mixtures thereof or a composition according to any of claims 5 to 7 in color converters, for optical labels, for invisible marking of products, as fluorescent dyes, preferably as fluorescent labels for biomolecules, as pigments, as a fluorescent dye in a display based on fluorescence conversion; in a light-collecting plastics part optionally combined with a solar cell; as a pigment dye in electrophoretic displays; as a fluorescent dye in an application based on chemoluminescence.

9. A color converter comprising (i) at least one polymer as matrix, consisting essentially of polystyrene, polycarbonate, polymethylmethacrylate, polyvinylpyrrolidone, polymethacrylate, polyvinyl acetate, polyvinyl chloride, polybutene, polyethylene glycol, silicone, polyacrylate, epoxy resin, polyvinyl alcohol, ethylene vinyl alcohol copolymer (EVOH), polyacrylonitrile, polyvinylidene chloride (PVDC), polystyreneacrylonitrile (SAN), polybutylene terephthalate (PBT), polyethylene terephthalate (PET), polyvinyl butyrate (PVB), polyvinyl chloride (PVC), polyamides, polyoxymethylenes, polyimides, polyetherimide or mixtures thereof, and (ii) at least one cyanated perylene compound of the formula I according to any of claims 1 to 4 or mixtures thereof or a composition according to any of claims 5 to 7 as fluorescent dye.

10. The color converter according to claim 9, wherein component (ii) comprises at least one cyanated perylene compound selected from compounds of the formulae (1) to (20) 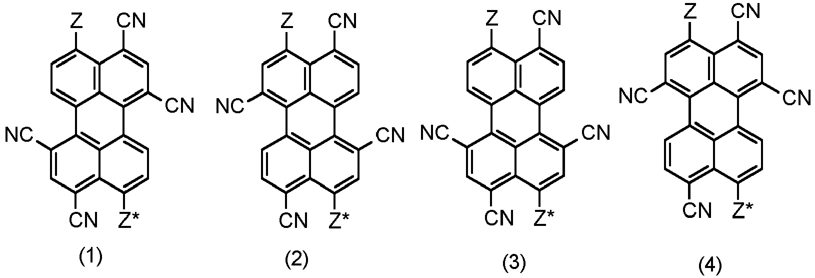 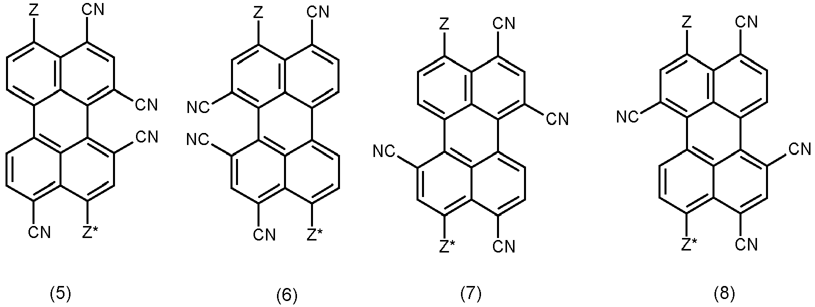 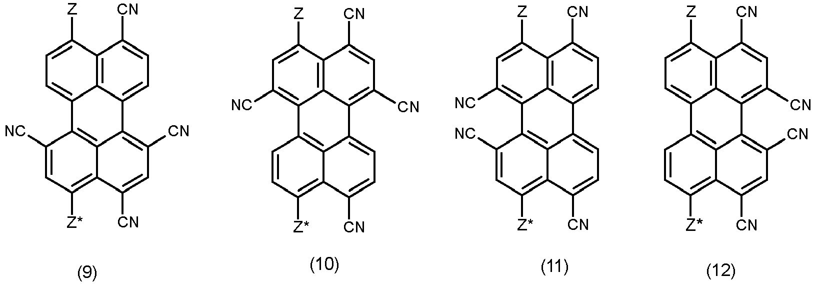 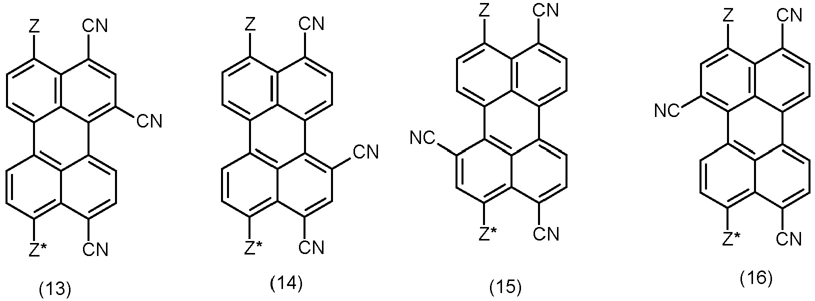 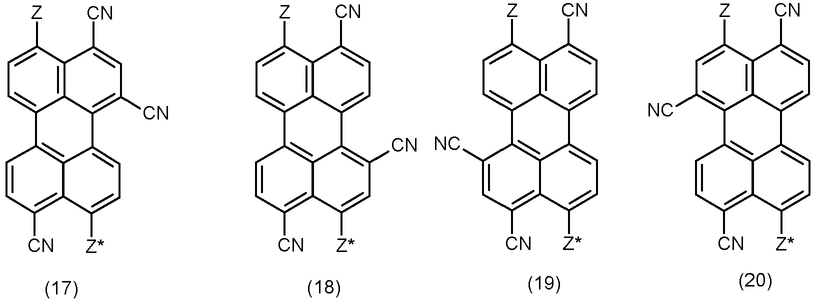 in which
Z is selected from C₁-C₆-alkyl, C₁-C₆-alkoxycarbonyl, phenyl, and phenyl bearing 1, 2 or 3 C₁-C₄-alkyl groups; and
Z* is selected from C₁-C₆-alkyl, C₁-C₆-alkoxycarbonyl, phenyl, and phenyl bearing 1, 2 or 3 C₁-C₄-alkyl groups;
and mixtures thereof,
or a composition comprising at least one cyanated perylene compound of the formula I selected from the compounds (1) to (20) and mixtures thereof.

11. The color converter according to claim 9 or 10, comprising at least one further organic fluorescent dye selected from compounds or mixtures of the formulae X, XI and XII 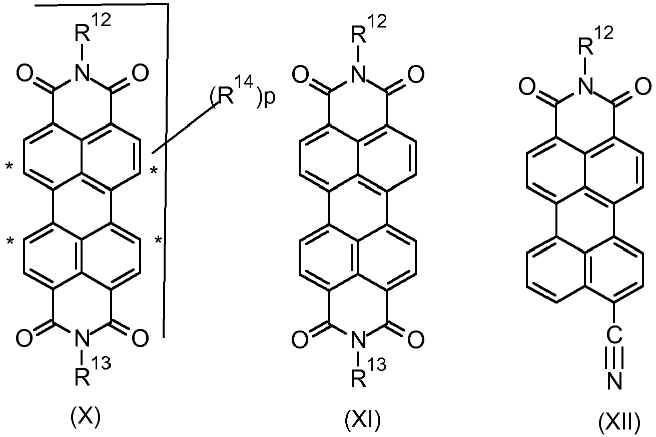 in which
p is 1 to 4,
R¹², R¹³ are each independently C₁-C₃₀-alkyl, C₃-C₈-cycloalkyl, C₆-C₁₄-aryl, hetaryl, C₆-C₁₄-aryl-C₁-C₁₀-alkylene, where the aromatic ring in the three latter radicals is unsubstituted or mono-or polysubstituted by C₁-C₁₀-alkyl,
R¹⁴ is C₁-C₃₀-alkoxy or C₆-C₁₄-aryloxy which is unsubstituted or mono- or polysubstituted by C₁-C₁₀-alkyl,
where the R¹⁴ radicals are at one or more of the positions indicated by *.

12. The color converter according to claim 11, wherein the further organic fluorescent dye is selected from N,N'-bis(2,6-diisopropylphenyl)-1,6,7,12-tetraphenoxyperylene-3,4;9,10-tetracarboximide, N,N'-bis(2,6-diisopropylphenyl)-1,7-di(2,6-diisopropylphenoxy)perylene-3,4;9,10-tetracarboximide, N,N'-bis(2,6-diisopropylphenyl)-1,6-di(2,6-diisopropylphenoxy)perylene-3,4;9,10-tetracarboximide, N,N'-bis(2,6-diisopropylphenyl)-1,7-di(p-tert-octylphenoxy)perylene-3,4;9,10-tetracarboximide, N,N'-bis(2,6-diisopropylphenyl)-1,6-di(p-tert-octylphenoxy)perylene-3,4;9,10-tetracarboximide, N,N'-bis(2,6-diisopropylphenyl)-1,7-diphenoxyperylene-3,4;9,10-tetracarboximide, N,N'-bis(2,6-diisopropylphenyl)-1,6-diphenoxyperylene-3,4;9,10-tetracarboximide and mixtures thereof.

13. The color converter according to any of claims 9 to 12, comprising, as a further fluorescent colorant, at least one inorganic fluorescent colorant selected from the rare earth-doped aluminates, silicates, nitrides and garnets, especially cerium-doped yttrium aluminum garnet.

14. The use of color converters as defined in any of claims 9 to 13 for conversion of light generated by LEDs.

15. A lighting device comprising at least one LED and at least one color converter according to any of claims 9 to 13.

## Revendications

1. Composé cyanuré à base de pérylène de formule I 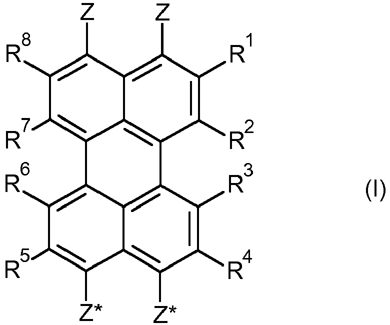 dans laquelle
un des substituants Z représente cyano et l'autre substituant Z représente CO₂R⁹, CONR¹⁰R¹¹, C₁-C₁₈-alkyle, C₂-C₁₈-alcényle, C₂-C₁₈-alcynyle, C₃-C₁₂-cycloalkyle ou C₆-C₁₄ -aryle,
C₁-C₁₈-alkyle, C₂-C₁₈-alcényle, C₂-C₁₈-alcynyle étant non substitués ou portant un ou plusieurs substituants Z^{a} identiques ou différents, C₃-C₁₂-cycloalkyle étant non substitué ou portant un ou plusieurs substituants Z^{b} identiques ou différents et
C₆-C₁₄-aryle étant non substitué ou portant un ou plusieurs substituants Z^{Ar} identiques ou différents ;
un des substituants Z* représente cyano et l'autre substituant Z* représente CO₂R⁹, CONR¹⁰R¹¹, C₁-C₁₈-alkyle,
C₂-C₁₈-alcényle, C₂-C₁₈-alcynyle, C₃-C₁₂-cycloalkyle ou C₆-C₁₄ -aryle,
C₁-C₁₈-alkyle, C₂-C₁₈-alcényle, C₂-C₁₈-alcynyle étant non substitués ou portant un ou plusieurs substituants Z^{a} identiques ou différents, C₃-C₁₂-cycloalkyle étant non substitué ou portant un ou plusieurs substituants Z^{b} identiques ou différents et
C₆-C₁₄-aryle étant non substitué ou portant un ou plusieurs substituants Z^{Ar} identiques ou différents ;
R¹, R², R³, R⁴, R⁵, R⁶, R⁷ et R⁸ sont choisis, indépendamment les uns des autres, parmi hydrogène, cyano, brome et chlore,
à condition que 1, 2, 3, 4, 5, 6, 7 ou 8 substituants parmi R¹, R², R³, R⁴, R⁵, R⁶, R⁷ ou R⁸ représente (nt) cyano ;
R⁹ représentant hydrogène, C₁-C₁₀-alkyle, C₂-C₁₀-alcényle, C₂-C₁₀-alcynyle, C₃-C₁₂-cycloalkyle ou C₆-C₁₄-aryle
C₁-C₁₀-alkyle, C₂-C₁₀-alcényle, C₂-C₁₀-alcynyle étant non substitués ou portant un ou plusieurs substituants R^{a} identiques ou différents, C₃-C₁₂-cycloalkyle étant non substitué ou portant un ou plusieurs substituants R^{b} identiques ou différents et
C₆-C₁₄-aryle étant non substitué ou portant un ou plusieurs substituants R^{Ar} identiques ou différents ;
R¹⁰ et R¹¹, indépendamment l'un de l'autre, représentant hydrogène, C₁-C₁₀-alkyle, C₂-C₁₀-alcényle, C₂-C₁₀-alcynyle, C₃-C₁₂-cycloalkyle ou C₆-C₁₄-aryle
C₁-C₁₀-alkyle, C₂-C₁₀-alcényle, C₂-C₁₀-alcynyle étant non substitués ou portant un ou plusieurs substituants R^{a} identiques ou différents, C₃-C₁₂-cycloalkyle étant non substitué ou portant un ou plusieurs substituants R^{b} identiques ou différents et
C₆-C₁₄-aryle étant non substitué ou portant un ou plusieurs substituants R^{Ar} identiques ou différents ;
- chaque Z^{a} représentant, indépendamment, halogène, hydroxy, NR^{10a}R^{11a}, C₁-C₁₀-alcoxy, C₁-C₁₀-halogénoalcoxy, C₁-C₁₀-alkylthio, C₃-C₁₂-cycloalkyle, C₆-C₁₄-aryle, C(=O)R^{9a}, C(=O)OR^{9a} ou C(O)NR^{10a}R^{11a},
C₃-C₁₂-cycloalkyle étant non substitué ou portant un ou plusieurs substituants R^{b} identiques ou différents et
C₆-C₁₄-aryle étant non substitué ou portant un ou plusieurs substituants R^{Ar} identiques ou différents ;
- chaque Z^{b} et chaque Z^{Ar} représentant, indépendamment l'un de l'autre, halogène, hydroxy, NR^{10a}R^{11a}, C₁-C₁₀-alkyle, C₁-C₁₀-alcoxy, C₁-C₁₀-halogénoalcoxy, C₁-C₁₀-alkylthio, C(=O)R^{9a}, C(=O)OR^{9a} ou C(O)NR^{10a}R^{11a} ;
- chaque R^{a} représentant, indépendamment, halogène, hydroxy, C₁-C₁₀-alcoxy, C₃-C₁₂-cycloalkyle ou C₆-C₁₄-aryle ;
- chaque R^{b} représentant, indépendamment, halogène, hydroxy, C₁-C₁₀-alkyle, C₁-C₁₀-alcoxy, C₁-C₁₀-halogénoalcoxy, C₁-C₁₀-alkylthio, C₂-C₁₀-alcényle, C₂-C₁₀-alcynyle, C₃-C₁₂-cycloalkyle ou C₆-C₁₄-aryle ;
- chaque R^{Ar} représentant, indépendamment, halogène, hydroxy, C₁-C₁₀-alkyle, C₁-C₁₀-alcoxy, C₁-C₁₀-halogénoalcoxy, C₁-C₁₀-alkylthio, C₂-C₁₀-alcényle, C₂-C₁₀-alcynyle, C₃-C₁₂-cycloalkyle ou C₆-C₁₄-aryle ;
R^{9a} représentant hydrogène, C₁-C₁₀-alkyle, C₂-C₁₀-alcényle, C₂-C₁₀-alcynyle, C₃-C₁₂-cycloalkyle ou C₆-C₁₄-aryle ; et
R^{10a}, R^{11a} représentant, indépendamment l'un de l'autre, hydrogène, C₁-C₁₀-alkyle, C₂-C₁₀-alcényle, C₂-C₁₀-alcynyle, C₃-C₁₂-cycloalkyle ou C₆-C₁₄-aryle,
et des mélanges correspondants.

2. Composé cyanuré à base de pérylène de formule I selon la revendication 1 et mélanges correspondants, dans laquelle un ou deux substituants parmi R¹, R², R³, R⁴, R⁵, R⁶, R⁷ ou R⁸ représente (nt) cyano et les autres substituants R¹, R², R³, R⁴, R⁵, R⁶, R⁷ ou R⁸ représentent hydrogène.

3. Composé cyanuré à base de pérylène de formule I selon la revendication 1 ou 2 et mélanges correspondants, dans lequel un des substituants Z et un des substituants Z* sont choisis, indépendamment l'un de l'autre, parmi C₁-C₁₀-alkyle, CO₂R⁹, phényl-C₁-C₁₀-alkyle et phényle, phényle et le fragment phényle de phényl-C₁-C₁₀-alkyle étant non substitués ou portant un ou plusieurs substituants choisis parmi C₁-C₆-alkyle et R⁹ étant défini comme ci-dessus.

4. Composé cyanuré à base de pérylène de formule I selon l'une quelconque des revendications précédentes, choisi parmi les composés des formules (1), (2), (3), (4), (5), (6), (7), (8), (9), (10), (11), (12), (13), (14), (15), (16), (17), (18), (19), (20) 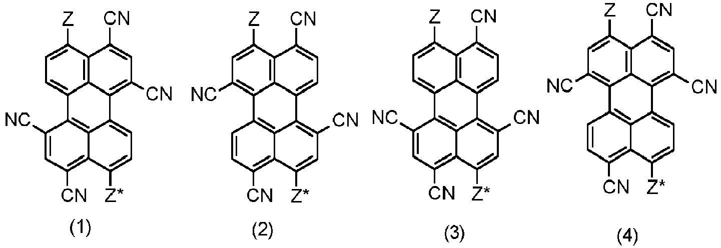 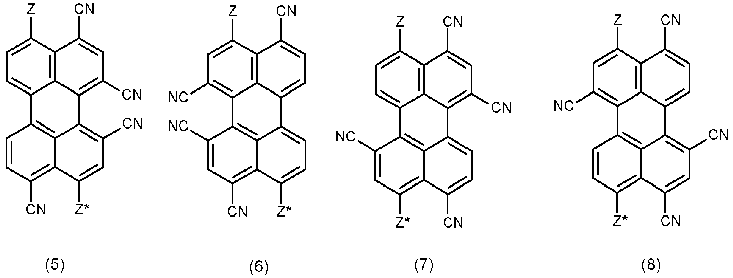 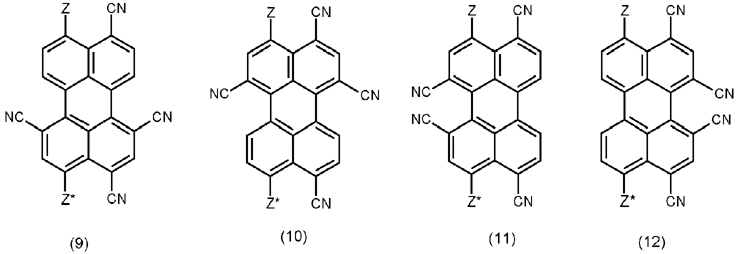 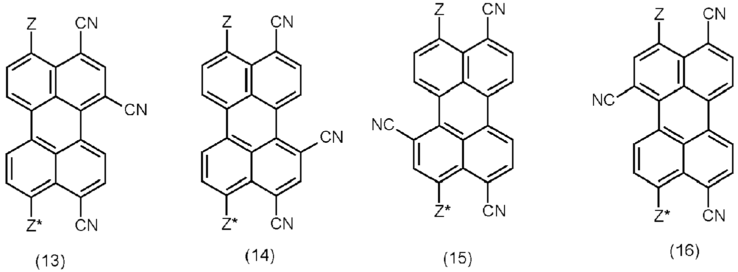 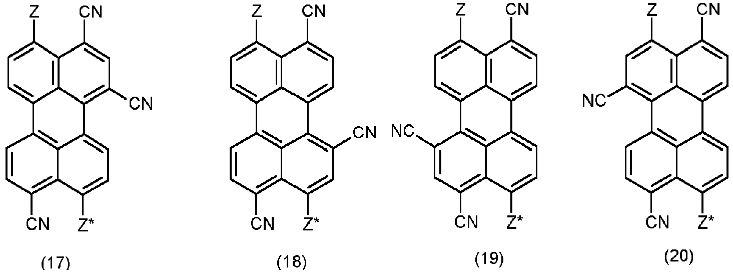 dans lesquelles
Z est choisi parmi C₁-C₆-alkyle, C₁-C₆-alcoxycarbonyle, phényle et phényle qui porte 1, 2 ou 3 groupes C₁-C₄-alkyle ; et
Z* est choisi parmi C₁-C₆-alkyle, C₁-C₆-alcoxycarbonyle, phényle et phényle qui porte 1, 2 ou 3 groupes C₁-C₄-alkyle ;
et des mélanges correspondants.

5. Composition, contenant au moins un composé cyanuré à base de pérylène de formule I selon l'une ou plusieurs des revendications 1 à 4 ou des mélanges correspondants.

6. Composition selon la revendication 5, contenant au moins un composé cyanuré à base de pérylène de formule I-A, 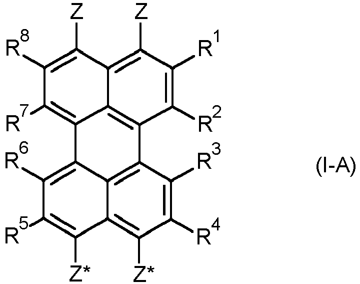 dans laquelle
un des substituants Z représente cyano et l'autre substituant Z représente C₁-C₁₈-alkyle, C₂-C₁₈-alcényle, C₂-C₁₈-alcynyle, C₃-C₁₂-cycloalkyle ou C₆-C₁₄-aryle,
C₁-C₁₈-alkyle, C₂-C₁₈-alcényle, C₂-C₁₈-alcynyle étant non substitués ou portant un ou plusieurs substituants Z^{a} identiques ou différents, Z^{a} présentant les significations susmentionnées,
C₃-C₁₂-cycloalkyle étant non substitué ou portant un ou plusieurs substituants Z^{b} identiques ou différents, Z^{b} présentant les significations susmentionnées ; et
C₆-C₁₄-aryle étant non substitué ou portant un ou plusieurs substituants Z^{Ar} identiques ou différents, Z^{Ar} présentant les significations susmentionnées ;
un des substituants Z* représente cyano et l'autre substituant Z* représente C₁-C₁₈-alkyle, C₂-C₁₈-alcényle, C₂-C₁₈-alcynyle, C₃-C₁₂-cycloalkyle ou C₆-C₁₄-aryle
C₁-C₁₈-alkyle, C₂-C₁₈-alcényle, C₂-C₁₈-alcynyle étant non substitués ou portant un ou plusieurs substituants Z^{a} identiques ou différents, Z^{a} présentant les significations susmentionnées,
C₃-C₁₂-cycloalkyle étant non substitué ou portant un ou plusieurs substituants Z^{b} identiques ou différents, Z^{b} présentant les significations susmentionnées ; et
C₆-C₁₄-aryle étant non substitué ou portant un ou plusieurs substituants Z^{Ar} identiques ou différents, Z^{Ar} présentant les significations susmentionnées ;
R¹, R⁴, R⁵ et R⁸ représentent à chaque fois hydrogène ; deux des substituants R², R³, R⁶ ou R⁷ représentent hydrogène ; et les autres substituants R², R³, R⁶ ou R⁷ représentent cyano ;
ou des mélanges correspondants, pouvant être obtenue selon un procédé dans lequel
a) on halogène du pérylène de formule II 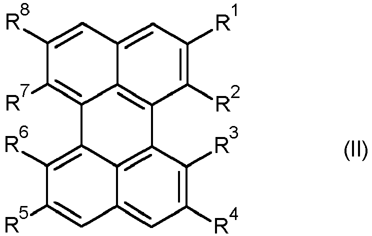 dans laquelle
R¹, R², R³, R⁴, R⁵, R⁶, R⁷ et R⁸ représentent à chaque fois hydrogène ;
avec obtention d'un mélange de 3,9-dihalogénoperylène de formule IIIa et de 3,10-dihalogénopérylène de formule IIIb 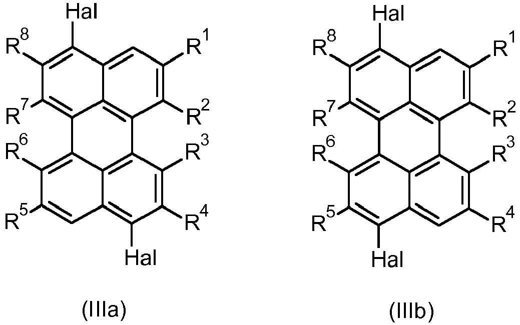 dans lesquelles
R¹, R², R³, R⁴, R⁵, R⁶, R⁷ et R⁸ représentent à chaque fois hydrogène ; et
les radicaux Hal représentent à chaque fois tous chlore ou brome ;
b) on transforme le mélange constitué par des composés des formules IIIa et IIIb, obtenu dans l'étape a), avec un composé organométallique de formule IV
Z-Met (IV)
et le cas échéant avec un composé organométallique de formule V
Z*-Met (V)
dans lesquelles
Z est choisi parmi C₁-C₁₈-alkyle, C₂-C₁₈-alcényle, C₂-C₁₈-alcynyle, C₃-C₁₂-cycloalkyle et C₆-C₁₄-aryle,
C₁-C₁₈-alkyle, C₂-C₁₈-alcényle, C₂-C₁₈-alcynyle étant non substitués ou portant un ou plusieurs substituants Z^{a} identiques ou différents,
C₃-C₁₂-cycloalkyle étant non substitué ou portant un ou plusieurs substituants Z^{b} identiques ou différents et
C₆-C₁₄-aryle étant non substitué ou portant un ou plusieurs substituants Z^{Ar} identiques ou différents ;
Z* est choisi parmi C₁-C₁₈-alkyle, C₂-C₁₈-alcényle, C₂-C₁₈-alcynyle, C₃-C₁₂-cycloalkyle et C₆-C₁₄-aryle,
C₁-C₁₈-alkyle, C₂-C₁₈-alcényle, C₂-C₁₈-alcynyle étant non substitués ou portant un ou plusieurs substituants Z^{a} identiques ou différents,
C₃-C₁₂-cycloalkyle étant non substitué ou portant un ou plusieurs substituants Z^{b} identiques ou différents et
C₆-C₁₄-aryle étant non substitué ou portant un ou plusieurs substituants Z^{Ar} identiques ou différents ;
Met Z* pouvant également présenter la même signification que Z ; représente B(OH)₂, B(OR') (OR"), Zn-Hal ou Sn(R*)₃, où
R' et R" représentent, indépendamment l'un de l'autre, hydrogène, C₁-C₃₀-alkyle, C₅-C₈-cycloalkyle, C₆-C₁₄-aryle ou hétéroaryle ou
R' et R" représentent ensemble C₂-C₄-alkylène, qui porte le cas échéant 1, 2, 3, 4, 5, 6, 7 ou 8 substituants, qui sont choisis parmi C₁-C₄-alkyle, C₅-C₈-cycloalkyle, C₆-C₁₄-aryle et hétéroaryle ;
Hal représente chlore ou brome ; et
R* représente C₁-C₈-alkyle ou phényle ;
avec obtention d'un mélange constitué par des composés des formules VIa et VIb, 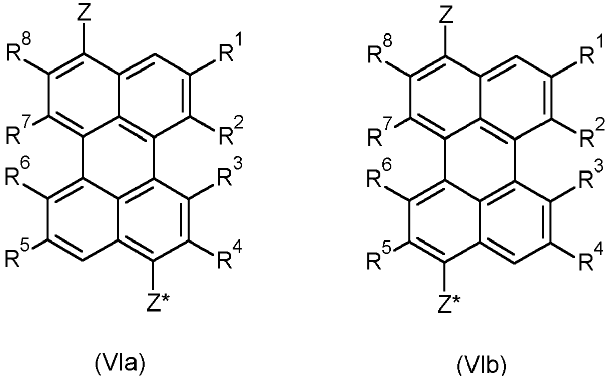 dans lesquelles
R¹, R², R³, R⁴, R⁵, R⁶, R⁷ et R⁸ représentent à chaque fois hydrogène ;
et Z et Z* sont définis comme ci-dessus ;
c) on halogène le mélange constitué par des composés de formules VIa et VIb, obtenu dans l'étape b), avec obtention d'un mélange réactionnel qui contient des composés des formules VIIa et VIIb 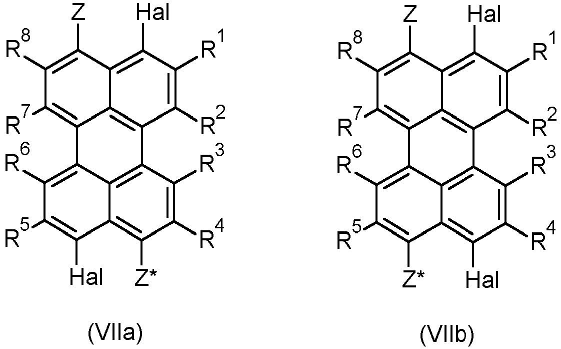 dans lesquelles
Z et Z* sont définis comme ci-dessus,
Hal représente halogène choisi parmi chlore et brome, les substituants Hal représentant soit tous chlore, soit tous brome ;
R¹, R², R³, R⁴, R⁵, R⁶, R⁷ et R⁸ représentent hydrogène ou halogène choisi parmi chlore et brome, les substituants R¹, R², R³, R⁴, R⁵, R⁶, R⁷ et R⁸, qui ne représentent pas hydrogène, représentant soit tous chlore, soit tous brome ;
d) on soumet les composés des formules VIIa et VIIb contenus dans le mélange réactionnel obtenu dans l'étape c) à une substitution d'halogène par cyano, ainsi que le cas échéant partiellement par hydrogène, avec obtention d'au moins un composé de formule I-A ou de mélanges correspondants ; et
e) le cas échéant, on soumet ledit au moins un composé de formule I-A ou les mélanges correspondants, contenu(s) dans le mélange réactionnel obtenu dans l'étape d), à au moins une étape de séparation et/ou de purification.

7. Composition selon la revendication 5, contenant au moins un composé cyanuré à base de pérylène de formule I-B, 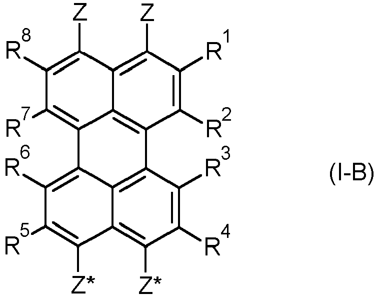 dans laquelle
un des substituants Z représente cyano et l'autre substituant Z représente COOR⁹ ;
un des substituants Z* représente cyano et l'autre substituant Z* représente COOR⁹ ;
R¹, R⁴, R⁵ et R⁸ représentent à chaque fois hydrogène ; un des substituants R², R³, R⁶ ou R⁷ représente cyano et les autres substituants R², R³, R⁶ ou R⁷ représentent hydrogène ; et
R⁹ représente hydrogène, C₁-C₁₀-alkyle, C₂-C₁₀-alcényle, C₂-C₁₀-alcynyle, C₃-C₁₂-cycloalkyle ou C₆-C₁₄-aryle C₁-C₁₀-alkyle, C₂-C₁₀-alcényle, C₂-C₁₀-alcynyle étant non substitués ou portant un ou plusieurs substituants R^{a} identiques ou différents,
C₃-C₁₂-cycloalkyle étant non substitué ou portant un ou plusieurs substituants R^{b} identiques ou différents et
C₆-C₁₄-aryle étant non substitué ou portant un ou plusieurs substituants R^{Ar} identiques ou différents ;
R^{a}, R^{b} et R^{Ar} étant définis comme ci-dessus ;
ou des mélanges correspondants, pouvant être obtenue selon un procédé dans lequel
f) on halogène un mélange constitué par des composés à base de pérylène des formules VIIIa et VIIIb 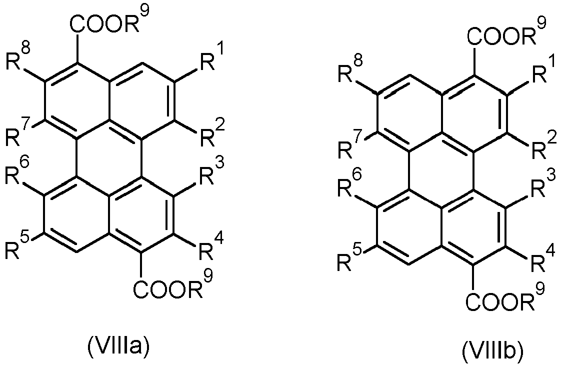 dans lesquelles
R¹, R², R³, R⁴, R⁵, R⁶, R⁷ et R⁸ représentent à chaque fois hydrogène ; et
R⁹ est défini comme ci-dessus
avec obtention d'un mélange réactionnel, qui contient des composés des formules IXa et IXb 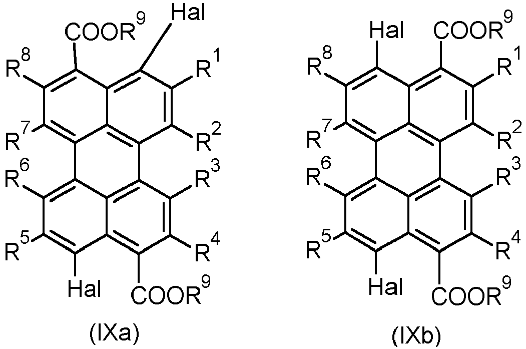 dans lesquelles
R¹, R², R³, R⁴, R⁵, R⁶, R⁷ et R⁸ représentent hydrogène ou halogène choisi parmi chlore et brome, les substituants R¹, R², R³, R⁴, R⁵, R⁶, R⁷ et R⁸, qui ne représentent pas hydrogène, représentant soit tous chlore, soit tous brome ;
Hal représente halogène choisi parmi chlore et brome, les substituants Hal représentant soit tous chlore, soit tous brome ; et
R⁹ est défini comme ci-dessus ;
g) on soumet les composés des formules IXa et IXb contenus dans le mélange réactionnel obtenu dans l'étape f) à une substitution d'halogène par des groupes cyano, ainsi que le cas échéant partiellement par hydrogène, avec obtention d'au moins un composé de formule I-B ou de mélanges correspondants ; et
h) le cas échéant, on soumet ledit au moins un composé de formule I-B ou des mélanges correspondants, contenu(s) dans le mélange réactionnel obtenu dans l'étape g), à au moins une étape de séparation et/ou de purification.

8. Utilisation des composés cyanurés de type pérylène de formule I selon l'une quelconque des revendications 1 à 4 et de mélanges correspondants ou d'une composition selon l'une quelconque des revendications 5 à 7, dans des convertisseurs de couleur, pour des étiquettes optiques, pour le marquage invisible de produits, comme colorants fluorescents, de préférence comme étiquette fluorescente pour des biomolécules, comme pigment, comme colorant fluorescent dans un affichage reposant sur la conversion de fluorescence ; dans une pièce en matériau synthétique collectrice de lumière, qui est le cas échéant combinée à une cellule solaire ; comme colorant pigmentaire dans des affichages électrophorétiques ; comme colorant fluorescent dans une application basée sur la chimiluminescence.

9. Convertisseur de couleur, contenant (i), comme matrice, au moins un polymère, essentiellement constitué par du polystyrène, du polycarbonate, du poly(méthacrylate de méthyle), de la polyvinylpyrrolidone, du polyméthacrylate, du poly(acétate de vinyle), du poly(chlorure de vinyle), du polybutène, du polyéthylèneglycol, du silicone, du polyacrylate, de la résine époxyde, du poly(alcool vinylique), un copolymère d'éthylène-alcool vinylique (EVOH), du polyacrylonitrile, du poly(chlorure de vinylidène) (PVDC), du poly(styrène-acrylonitrile) (SAN), du poly(téréphtalate de butylène) (PBT), du poly(téréphtalate d'éthylène) (PET), du poly(butyrate de vinyle) (PVB), du poly(chlorure de vinyle) (PVC), du polyamide, du polyoxyméthylène, du polyimide, du polyétherimide ou des mélanges correspondants, et (ii), comme colorant fluorescent, au moins un composé cyanuré à base de pérylène de formule I selon l'une quelconque des revendications 1 à 4 ou des mélanges correspondants ou une composition selon l'une quelconque des revendications 5 à 7.

10. Convertisseur de couleur selon la revendication 9, le composant (ii) étant au moins un composé cyanuré à base de pérylène choisi parmi les composés des formules (1) à (20) 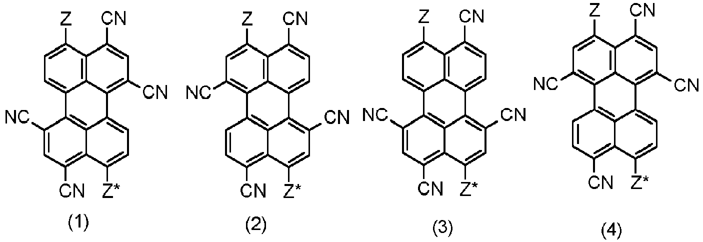 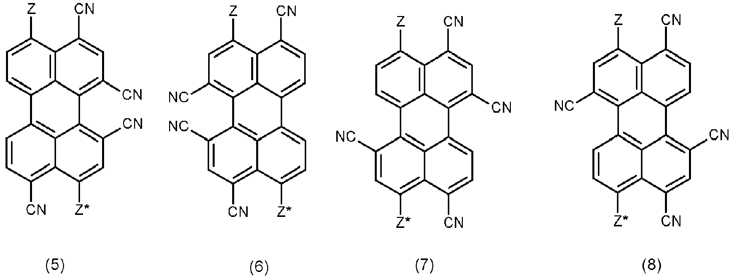 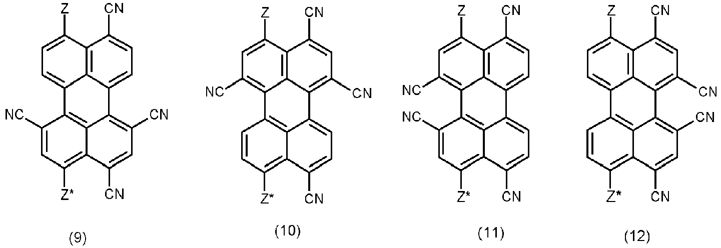 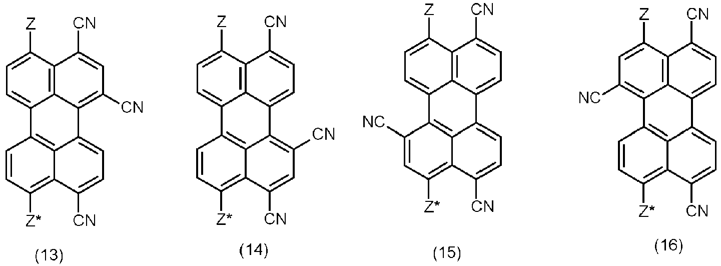 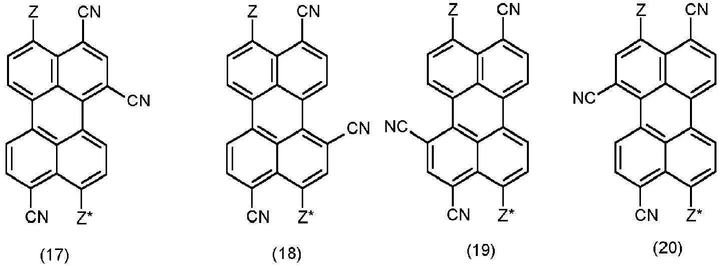 dans lesquelles
Z est choisi parmi C₁-C₆-alkyle, C₁-C₆-alcoxycarbonyle, phényle et phényle qui porte 1, 2 ou 3 groupes C₁-C₄-alkyle ; et
Z* est choisi parmi C₁-C₆-alkyle, C₁-C₆-alcoxycarbonyle, phényle et phényle qui porte 1, 2 ou 3 groupes C₁-C₄-alkyle ;
et des mélanges correspondants,
ou une composition, contenant au moins un composé cyanuré à base de pérylène de formule I choisi parmi les composés (1) à (20) ou des mélanges correspondants.

11. Convertisseur de couleur selon la revendication 9 ou 10, contenant au moins un autre colorant fluorescent organique, choisi parmi les composés ou les mélanges des formules X, XI et XII 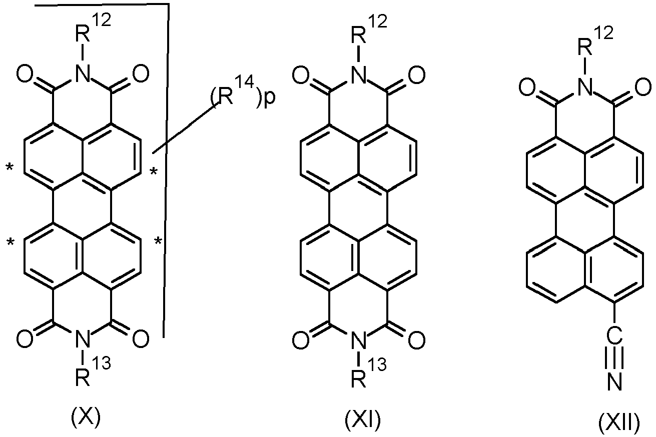 dans lesquelles
p représente 1 à 4,
R¹², R¹³ représentent, indépendamment l'un de l'autre, C₁-C₃₀-alkyle, C₃-C₈-cycloalkyle, C₆-C₁₄-aryle, hétaryle, C₆-C₁₄-aryl-C₁-C₁₀-alkylène, le cycle aromatique dans les trois derniers radicaux mentionnés étant non substitué ou étant monosubstitué ou polysubstitué par C₁-C₁₀-alkyle,
R¹⁴ représente C₁-C₃₀-alcoxy ou C₆-C₁₄-aryloxy, qui est non substitué ou monosubstitué ou polysubstitué par C₁-C₁₀-alkyle, les radicaux R¹⁴ se trouvant en une ou plusieurs des positions **caractérisées par** *.

12. Convertisseur de couleur selon la revendication 11, l'autre colorant fluorescent organique étant choisi parmi le diimide de l'acide N,N'-bis(2,6-diisopropylphényl)-1,6,7,12-tétraphénoxypérylène-3,4;9,10-tétracarboxylique, le diimide de l'acide N,N'-bis(2,6-diisopropylphényl)-1,7-di(2,6-diisopropylphénoxy)pérylène-3,4;9,10-tétracarboxylique, le diimide de l'acide N,N'-bis(2,6-diisopropylphényl)-1,6-di(2,6-diisopropylphénoxy)pérylène-3,4;9,10-tétracarboxylique, le diimide de l'acide N,N'-(bis(2,6-diisopropylphényl)-1,7-di(p-tert-octylphénoxy)-pérylène-3,4 ;9,10-tétracarboxylique, le diimide de l'acide N,N'-(bis(2,6-diisopropylphényl)-1,6-di(p-tert-octylphénoxy)-pérylène-3,4;9,10-tétracarboxylique, le diimide de l'acide N,N'-bis-(2,6-diisopropylphényl)-1,7-diphénoxypérylène-3,4;9,10-tétracarboxylique, le diimide de l'acide N,N'-bis-(2,6-diisopropylphényl)-1,6-diphénoxypérylène-3,4;9,10-tétracarboxylique et des mélanges correspondants.

13. Convertisseur de couleur selon l'une quelconque des revendications 9 à 12, contenant, comme autre colorant fluorescent, au moins un colorant fluorescent inorganique choisi parmi les aluminates, les silicates, les nitrures et les grenats dopés aux terres rares, en particulier le grenat d'yttrium-aluminium dopé au cérium.

14. Utilisation de convertisseurs de couleur tels que définis dans l'une quelconque des revendications 9 à 13 pour la conversion de lumière générée par des DEL.

15. Dispositif d'éclairage, comprenant au moins une DEL et au moins un convertisseur de couleur selon l'une quelconque des revendications 9 à 13.
